# EUROPEAN PATENT SPECIFICATION

(11) **EP 2 994 160 B1**
(45) Date of publication and mention of the grant of the patent: **03.07.2019**
(21) Application number: 14730660.9
(22) Date of filing: 05.05.2014
(51) Int. Cl.: A61K 39/395, A61K 38/47, C07K 16/06, C07K 16/18, G01N 33/68, A61K 39/00

(54) **TREATMENT OF ALZHEIMER'S DISEASE SUBPOPULATIONS WITH POOLED IMMUNOGLOBULIN G**
BEHANDLUNG VON MORBUS-ALZHEIMER-SUBPOPULATIONEN MIT GEPOOLTEM IMMUNGLOBULIN G
TRAITEMENT DE SOUS-POPULATIONS ATTEINTES DE LA MALADIE D'ALZHEIMER AVEC DE L'IMMUNOGLOBULINE G RASSEMBLÉE

(30) Priority: 06.05.2013 US 201361855062 P; 10.06.2013 US 201361833447 P; 10.07.2013 US 201361844732 P; 03.10.2013 US 201361886464 P
(43) Date of publication of application: 16.03.2016
(60) Divisional application: 19175106.4
(73) Proprietor: Baxalta Incorporated, Bannockburn, IL 60015 (US); Baxalta GmbH, 6300 Zug (CH)
(72) Inventor: GELMONT, David, M., Woodland Hills, CA 91364 (US); SINGER, Julia, 1210 Vienna (AT); FRITSCH, Sandor, 1210 Vienna (AT); SCHWARZ, Hans-Peter, 1180 Vienna (AT)
(74) Representative: Hoffmann Eitle
(86) International application number: PCT/US2014/036848
(87) International publication number: WO 2014/182631

(56) References cited:
- WO-A2-2009/052439
- US-A1- 2011 251 479
- RELKIN N R ET AL: "18-Month study of intravenous immunoglobulin for treatment of mild Alzheimer disease", NEUROBIOLOGY OF AGING, TARRYTOWN, NY, US, vol. 30, no. 11, 1 November 2009 (2009-11-01), pages 1728-1736, XP026684154, ISSN: 0197-4580, DOI: 10.1016/J.NEUROBIOLAGING.2007.12.021 [retrieved on 2008-02-21]
- JACOB RABER ET AL: "ApoE genotype accounts for the vast majority of AD risk and AD pathology", NEUROBIOLOGY OF AGING, vol. 25, no. 5, 1 May 2004 (2004-05-01), pages 641-650, XP055133307, ISSN: 0197-4580, DOI: 10.1016/j.neurobiolaging.2003.12.023
- L. M. TAI ET AL: "Levels of Soluble Apolipoprotein E/Amyloid- (A ) Complex Are Reduced and Oligomeric A Increased with APOE4 and Alzheimer Disease in a Transgenic Mouse Model and Human Samples", JOURNAL OF BIOLOGICAL CHEMISTRY, vol. 288, no. 8, 22 February 2013 (2013-02-22), pages 5914-5926, XP055132975, ISSN: 0021-9258, DOI: 10.1074/jbc.M112.442103
- DAVID A LOEFFLER: "Intravenous immunoglobulin and Alzheimer's disease: what now?", JOURNAL OF NEUROINFLAMMATION, vol. 10, no. 1, 1 January 2013 (2013-01-01), page 70, XP055121266, ISSN: 1742-2094, DOI: 10.1111/j.1600-065X.1989.tb00031.x
- TSAKANIKAS D ET AL: "P4-351: Effects of uninterrrupted intravenous immunoglobulin treatment of Alzheimer's disease for nine months", ALZHEIMER'S & DEMENTIA: THE JOURNAL OF THE ALZHEIMER'SASSOCIATION, ELSEVIER, NEW YORK, NY, US, vol. 4, no. 4, 1 July 2008 (2008-07-01), page T776, XP023175323, ISSN: 1552-5260, DOI: 10.1016/J.JALZ.2008.05.2422 [retrieved on 2008-07-01]
- JULIEN DELRIEU ET AL: "'Clinical trials in Alzheimer's disease': immunotherapy approaches", JOURNAL OF NEUROCHEMISTRY, vol. 120, 28 January 2012 (2012-01-28), pages 186-193, XP055132857, ISSN: 0022-3042, DOI: 10.1111/j.1471-4159.2011.07458.x
- MARTIN CITRON: "Alzheimer's disease: strategies for disease modification", NATURE REVIEWS DRUG DISCOVERY, vol. 9, no. 5, 1 May 2010 (2010-05-01), pages 387-398, XP055132854, ISSN: 1474-1776, DOI: 10.1038/nrd2896

## Description

### BACKGROUND OF THE INVENTION

Alzheimer's disease (AD) is a progressive neurodegenerative disorder and the leading cause of dementia in the elderly. Increasing longevity in the past century has contributed to an exponential rise in AD. It is estimated more than 5 million people in the United States (US) currently suffer from AD. The prevalence of AD is forecast to increase 3-fold by 2050 (Herbert et al., Alzheimer Dis. Assoc. Disord., 15:169-173 (2001)). The annual costs of AD treatment to American society approach $100 billion and projected future expenditures threaten to overwhelm the healthcare budget unless more effective means of treating and preventing AD are found. Currently, four acetylcholinesterase inhibitors and an N-methyl-D-aspartic acid (NMDA) receptor antagonist have been approved as treatments for symptomatic AD in the United States. These medications can transiently improve cognitive abilities and slow cognitive decline in AD patients. However, most patients receiving these agents decline below their pretreatment baseline within six to twelve months of initiating therapy. There is a paucity of evidence to suggest that these medications change the course of AD's underlying neuropathology.

Alzheimer's disease is becoming more prevalent in developed nations, where an increase in the population of elder persons has occurred due in part to improved healthcare. While less than 1% of the population under the age of 60 is affected by Alzheimer's, it is estimated that 25% to 33% of persons develop some form of Alzheimer's by the age of 85. As of 2012, 5.4 million Americans were diagnosed with Alzheimer's. As life expectancy continues to increase worldwide, the prevalence of Alzheimer's and other age-related dementia should continue to grow as well.

Alzheimer's disease is typically classified as either "early onset," referring to cases that begin to manifest at between 30 and 60 years of age in affected individuals, and the more common "late onset" Alzheimer's, in which symptoms first become apparent after the age of 60. Although only about 10% of all Alzheimer's cases are familial, early onset Alzheimer's disease has been linked to mutations in the amyloid precursor protein (*app*), presenilin 1 (*psen1*), and presenilin 2 (*psen2*) genes, while late onset Alzheimer's disease has been linked to mutations in the apolipoprotein E (*apoE*) gene (Ertekin-Taner N., Neurol Clin., 25:611-667 (2007)).

Histopathologically, this neurodegenerative disease is characterized by the formation of amyloid plaques, neurofibrillary tangles, amyloid angiopathy, and granolovacuolar degeneration in the cerebral cortex (Mirra et al., Arch Pathol Lab Med., 117:132-144 (1993); Perl DP, Neurol Clin., 18:847-864 (2000)). The characteristic amyloid plaques, used to confirm Alzheimer's disease post-mortem, are formed largely by deposition of a small amyloid-beta (Aβ) peptide derived from the amyloid precursor protein (APP).

While there are a great many potential targets for AD pharmacotherapy, abnormalities in the production, processing and/or brain clearance of the amyloid beta (Aβ) peptide are thought to be among the most important and earliest steps in the AD's pathogenesis (Selkoe DJ., Ann. Intern. Med. 2004; 140:627-638). Aβ is known to spontaneously form soluble aggregates called oligomers and insoluble fibrils that can form deposits in the brain. These aggregates can precipitate a cascade of inflammatory and other reactive brain changes that eventually interfere with synaptic transmission and accelerate the death of neurons in many brain regions including cortical and subcortical networks that subserve cognition and behavior (Selkoe DJ., *supra*). Early treatment of Aβ-related abnormalities could pre-empt downstream elements of AD's pathogenic cascade and thereby alter the course of the disease.

To date, the U.S. Food and Drug Administration (FDA) has approved two types of medications for the management of Alzheimer's disease: cholinesterase inhibitors, including Aricept® (donepezil), Exelon (rivastigmine), Razadyne (galantamine), and Cognex (tacrine); and the NMDA-type glutamate receptor inhibitor memantine (marketed under a number of different brands). Although a cure for Alzheimer's disease has not been identified, these therapies serve to alleviate cognitive symptoms such as memory loss, confusion, and loss of critical thinking abilities in subjects diagnosed with age-related dementia (*e*.*g*., Alzheimer's disease). In all, it is estimated that healthcare spending on Alzheimer's disease and related age-related dementias in 2012 will be $200 billion in the United States alone (Factsheet, Alzheimer's Association, Mar 2012).

In addition to these approved therapies, several studies have suggested that pooled intravenous immunoglobulin (IVIG) is effective in slowing the progression of symptoms in Alzheimer's patients (Dodel RC et al., J Neurol Neurosurg Psychiatry, Oct; 75(10):1472-4 (2004); Magga J. et al., J Neuroinflammation, Dec 7; 7:90 (1997); Relkin NR et al., Neurobiol Aging, 30(11):1728-36 (2008); Puli L. et al., J Neuroinflammation May 29; 9:105 (2012)).

US 2011/251479 concerns the use of MRI monitoring of ventricular enlargement rate as an objective measure for the purpose of assessing disease progression in patients suffering from Alzheimer's disease and for the purpose of determining therapeutic effectiveness of a treatment regimen for Alzheimer's patients.

A recent review of bapineuzumab describes a Phase II study having a total of 234 patients with a clinical diagnosis of probable AD, aged 50-85 with MMSE scores of 16-26. Patients entered three dosing cohorts: 0.5 mg/kg, 1.0 mg/kg, and 2.0 mg/kg. No significant effect of bapineuzumab emerged in patients who received at least one infusion followed by at least one assessment. Although slight clinical effects were observed, these were in ApoE4 non-carriers. 12 of 124 bapineuzumab-treated patients developed vasogenic cerebral edema, which showed increased risk with increased ApoE4 gene copy. (Kerchner GA, Boxer AL., Expert Opin Biol Ther. 2010 Jul; 10(7):1121-30*)*. Another review that describes the bapineuzumab side effects relating to vasogenic cerebral edema, and proposes that future treatment should include lower doses, particularly in ApoE4 carriers. (Cribbs DH., CNS Neurol Disord Drug Targets, 9(2):207-16 (2010)).

Another study describes a follow-up safety and pharmacology analysis of ponezumab after a single 10-minute IV infusion in subjects with mild to moderate AD. The study was a Phase I, single-dose, dose-escalation, open-label, safety, tolerability, and pharmacology study. Subjects received 1 mg/kg, 3 mg/kg, 5 mg/kg, or 10 mg/kg (n= 3, 3, 4, and 5, respectively). Subjects were over 50 years of age, and had MMSE scores of 16 to 26. Cognitive function showed no treatment-related trends (Burstein AH, Zhao Q, Ross J, Styren S, Landen JW, Ma WW, McCush F, Alvey C, Kupiec JW, Bednar MM., Clin Neuropharmacol., 36(1):8-13 (2013)).

The purpose of the study described in this Spearling *et al.* was to determine correlation of bapineuzumab with vasogenic oedema and sulcal effusions (ARIA-E) and microhaemorrhages and haemosiderin deposits (ARIA-H). MRI scans were reviewed from 262 participants in two phase-II studies. 17% of the patients developed ARIA-E Occurrence of ARIA-E increased with bapineuzumab dose and the presence of ApoE4 alleles. (Sperling R, Salloway S, Brooks DJ, Tampieri D, Barakos J, Fox NC, Raskind M, Sabbagh M, Honig LS, Porsteinsson AP, Lieberburg I, Arrighi HM, Morris KA, Lu Y, Liu E, Gregg KM, Brashear HR, Kinney GG, Black R, Grundman M., Lancet Neurol., 11(3):241-9 (2012)).

Farlow *et al.* describes a study that assessed the safety and tolerability of 12 weekly infusions of solanezumab in patients with mild-to-moderate AD. This was a phase 2, randomized, double-blind, placebo-controlled trial with 52 AD patients. Cohorts were 100 mg every 4 weeks, 100 mg weekly, 400 mg every 4 weeks, or 400 mg weekly for 12 weeks. Patients were over 50 years of age with mild-to-moderate probable AD with MMSE scores of 15-26. Patients disclosed ulcer hemorrhages, pancreatitis, chest pain, infection, lumbar puncture, headache, subdural hematoma, syncope, agitation, ovarian cyst, and skin ulcer. There were no significant differences between solanezumab and placebo on an 11 item or 14 item cognitive score. (Farlow M, Arnold SE, van Dyck CH, Aisen PS, Snider BJ, Porsteinsson AP, Friedrich S, Dean RA, Gonzales C, Sethuraman G, DeMattos RB, Mohs R, Paul SM, Siemers ER., Alzheimer's Dement. 8(4):261-71 (2012)).

*See also:* Landen JW, Zhao Q, Cohen S, Borrie M, Woodward M, Billing CB Jr, Bales K, Alvey C, McCush F, Yang J, Kupiec JW, Bednar MM., Clin Neuropharmacol. 2013 Jan-Feb; 36(1):14-23; Uenaka K, Nakano M, Willis BA, Friedrich S, Ferguson-Sells L, Dean RA, Ieiri I, Siemers ER. Clin Neuropharmacol., 35(1):25-9(2012); Ostrowitzki S, Deptula D, Thurfjell L, Barkhof F, Bohrmann B, Brooks DJ, Klunk WE, Ashford E, Yoo K, Xu ZX, Loetscher H, Santarelli L.. Arch Neurol., 69(2):198-207 (2012); Siemers ER, Friedrich S, Dean RA, Gonzales CR, Farlow MR, Paul SM, Demattos RB. Clin Neuropharmacol., 33(2):67-7 (2012); Imbimbo BP, Ottonello S, Frisardi V, Solfrizzi V, Greco A, Seripa D, Pilotto A, Panza F., Expert Rev Clin Immunol., 8(2):135-49 (2012); Carlson C, Estergard W, Oh J, Suhy J, Jack CR Jr, Siemers E, Barakos J., Alzheimer's Dement., 7(4):396-401 (2011); Rinne JO, Brooks DJ, Rossor MN, Fox NC, Bullock R, Klunk WE, Mathis CA, Blennow K, Barakos J, Okello AA, Rodriguez Martinez de Liano S, Liu E, Koller M, Gregg KM, Schenk D, Black R, Grundman M, Lancet Neurol., 9(4):363-72 (2010). Blennow K, Zetterberg H, Rinne JO, Salloway S, Wei J, Black R, Grundman M, Liu E; AAB-001 201/202 Investigators, Arch Neurol., 69(8):1002-10 (2012).

Different ApoE isoforms can mod*ulate Aβ levels. Tai et al.* describes an ELI*SA* assay that measures the soluble apoE/Aβ complex to address the hypothesis *that reduced leve*ls of soluble apoE/Aβ complex correlates with an increase in soluble oligomeric Aβ. In mice and human cortical synaptosome preparations, apoE/Aβ levels were lower in an ApoE4 mouse model than in ApoE2 and ApoE3 mouse *model, suggesting t*hat ApoE isofor*ms* specifically modulate oligomeric Aβ clearance (ApoE2 is protective). In human cortical synaptosomes, apoE/Aβ co*mplex levels were* lower in AD patients, and lower in the cohort having the ApoE4 isoform within the AD group. Further, oligomeric Aβ levels were increased and were greater in the ApoE4 isoform cohort. (Tai et al., J.B.C., 288:5914-5926 (2013))..

Kim *et al.* tested the hypothesis that anti-apoI antibodies can have antiamyloidogenic effects by binding to apoE in Aβ plaques and activating microglia-mediated amyloid clearance. Several anti-apoE antibodies were generated. The article demonstrates that passive immunization against apoE can attenuate Aβ accumulation. Anti-apoE antibodies increased CD45-positive microglia and decreased proinflammatory cytokines. The authors hypothesized that anti-apoE antibodies may recruit microglia to apoE-containing plaques, triggering direct phagocytosis and the attenuation of proinflammatory cytokines, leading to a reduction in amyloid accumulation. (Kim et al. J. Exp Med 209:2149-2156 (2012)).

Accordingly, there is a need in the art for methods of treating Alzheimer's disease in individuals in need thereof, who have been classified as ApoE4 positive and/or having moderate dementia. There is also a need in the art for methods of assigning effective treatment for Alzheimer's disease to individuals in need thereof, based upon the sub-population of Alzheimer's disease in which the individual is classified.

### BRIEF SUMMARY OF INVENTION

The present disclosure provides solutions to these and other problems by providing methods for the treatment of Alzheimer's disease in patients having moderate Alzheimer's disease and/or carrying an ApoE4 allele by administration of pooled immunoglobulin G. Advantageously, it is shown herein that administration of high dose pooled immunoglobulin G *(e.g.,* 400 mg/kg/2 weeks IVIG) slows down the progression of dementia in Alzheimer's subjects with moderate disease and in Alzheimer's subjects carrying an ApoE4 allele.

In one aspect, the disclosure provides a method for treating Alzheimer's disease in a subject in need thereof, the method comprising: administering a therapeutically effective amount of a composition comprising pooled human immunoglobulin G (IgG) to a subject with moderately severe Alzheimer's disease, wherein the amount of pooled human IgG is from 300 mg/kg to 800 mg/kg body weight of the subject per two week period, and wherein the amount is administered in one or more doses during the two week period after initiation of a therapeutic regimen. In some embodiments, the amount of pooled human IgG is from 200 mg/kg to 800 mg/kg body weight of the subject per two week period.

In another aspect, the disclosure provides a method for treating Alzheimer's disease in a subject in need thereof, the method comprising: administering a therapeutically effective amount of a composition comprising pooled human immunoglobulin G (IgG) to a subject with Alzheimer's disease who is carrier of at least one APOE4 allele, wherein the amount of pooled human IgG is from 300 mg/kg to 800 mg/kg body weight of the subject per two week period, and wherein the amount is administered in one or more doses during the two week period after initiation of a therapeutic regimen. In some embodiments, the amount of pooled human IgG is from 200 mg/kg to 800 mg/kg body weight of the subject per two week period.

In another aspect, the disclosure provides a method for treating Alzheimer's disease in a subject in need thereof, the method comprising: administering a therapeutically effective amount of a composition comprising pooled human immunoglobulin G (IgG) to a subject with moderately severe Alzheimer's disease who is carrier of at least one APOE4 allele, wherein the amount of pooled human IgG is from 300 mg/kg to 800 mg/kg body weight of the subject per two week period, and wherein the amount is administered in one or more doses during the two week period after initiation of a therapeutic regimen. In some embodiments, the amount of pooled human IgG is from 200 mg/kg to 800 mg/kg body weight of the subject per two week period.

In one aspect, the disclosure provides a use of a composition comprising pooled human immunoglobulin G (IgG) for the treatment of moderately severe Alzheimer's disease in a subject in need thereof for treatment comprising administration of from 300 mg/kg to 800 mg/kg body weight of the subject per two week period, wherein the amount is administered in one or more doses during the two week period after initiation of a therapeutic regimen. In some embodiments, the amount of pooled human IgG is from 200 mg/kg to 800 mg/kg body weight of the subject per two week period.

In one aspect, the disclosure provides a use of a composition comprising pooled human immunoglobulin G (IgG) for the treatment of Alzheimer's disease in a subject in need thereof who is carrier of at least one APOE4 allele for treatment comprising administration of from 300 mg/kg to 800 mg/kg body weight of the subject per two week period, wherein the amount is administered in one or more doses during the two week period after initiation of a therapeutic regimen. In some embodiments, the amount of pooled human IgG is from 200 mg/kg to 800 mg/kg body weight of the subject per two week period.

In one aspect, the disclosure provides a use of a composition comprising pooled human immunoglobulin G (IgG) for the treatment of moderately severe Alzheimer's disease in a subject in need thereof who is carrier of at least one APOE4 allele for treatment comprising administration of from 300 mg/kg to 800 mg/kg body weight of the subject per two week period, wherein the amount is administered in one or more doses during the two week period after initiation of a therapeutic regimen. In some embodiments, the amount of pooled human IgG is from 200 mg/kg to 800 mg/kg body weight of the subject per two week period.

In one embodiment of the methods and uses described above, the amount of pooled human IgG is 250 mg/kg body weight of the subject per two week period.

In one embodiment of the methods and uses described above, the amount of pooled human IgG is 300 mg/kg body weight of the subject per two week period.

In one embodiment of the methods and uses described above, the amount of pooled human IgG is 350 mg/kg body weight of the subject per two week period.

In one embodiment of the methods and uses described above, the amount of pooled human IgG is 400 mg/kg body weight of the subject per two week period.

In one embodiment of the methods and uses described above, the amount of pooled human IgG is 450 mg/kg body weight of the subject per two week period.

In one embodiment of the methods and uses described above, the amount of pooled human IgG is 500 mg/kg body weight of the subject per two week period.

In one embodiment of the methods and uses described above, the amount of pooled human IgG is 550 mg/kg body weight of the subject per two week period.

In one embodiment of the methods and uses described above, the amount of pooled human IgG is 600 mg/kg body weight of the subject per two week period.

In one embodiment of the methods and uses described above, the amount of pooled human IgG is 650 mg/kg body weight of the subject per two week period.

In one embodiment of the methods and uses described above, the amount of pooled human IgG is 700 mg/kg body weight of the subject per two week period.

In one embodiment of the methods and uses described above, the amount of pooled human IgG is 750 mg/kg body weight of the subject per two week period.

In one embodiment of the methods and uses described above, the amount of pooled human IgG is 800 mg/kg body weight of the subject per two week period.

In one aspect, the disclosure provides a method for treating Alzheimer's disease in a subject in need thereof, the method comprising: administering directly to the CNS a therapeutically effective amount of a composition comprising pooled human immunoglobulin G (IgG) to a subject with moderately severe Alzheimer's disease, wherein the amount of pooled human IgG is from 50 mg/kg to 400 mg/kg body weight of the subject per two week period, and wherein the amount is administered in one or more doses during the two week period after initiation of a therapeutic regimen.

In one aspect, the disclosure provides a method for treating Alzheimer's disease in a subject in need thereof, the method comprising: administering directly to the CNS a therapeutically effective amount of a composition comprising pooled human immunoglobulin G (IgG) to a subject with moderately severe Alzheimer's disease, wherein the amount of pooled human IgG is from 1 mg to 400 mg total dose per two week period, and wherein the amount is administered in one or more doses during the two week period after initiation of a therapeutic regimen.

In one aspect, the disclosure provides a method for treating Alzheimer's disease in a subject in need thereof, the method comprising: administering directly to the CNS a therapeutically effective amount of a composition comprising pooled human immunoglobulin G (IgG) to a subject with Alzheimer's disease who is carrier of at least one APOE4 allele, wherein the amount of pooled human IgG is from 50 mg/kg to 400 mg/kg body weight of the subject per two week period, and wherein the amount is administered in one or more doses during the two week period after initiation of a therapeutic regimen.

In one aspect, the disclosure provides a method for treating Alzheimer's disease in a subject in need thereof, the method comprising: administering directly to the CNS a therapeutically effective amount of a composition comprising pooled human immunoglobulin G (IgG) to a subject with Alzheimer's disease who is carrier of at least one APOE4 allele, wherein the amount of pooled human IgG is from 1 mg to 400 mg total dose per two week period, and wherein the amount is administered in one or more doses during the two week period after initiation of a therapeutic regimen.

In one aspect, the disclosure provides a method for treating Alzheimer's disease in a subject in need thereof, the method comprising: administering directly to the CNS a therapeutically effective amount of a composition comprising pooled human immunoglobulin G (IgG) to a subject with moderately severe Alzheimer's disease who is carrier of at least one APOE4 allele, wherein the amount of pooled human IgG is from 50 mg/kg to 400 mg/kg body weight of the subject per two week period, and wherein the amount is administered in one or more doses during the two week period after initiation of a therapeutic regimen.

In one aspect, the disclosure provides a method for treating Alzheimer's disease in a subject in need thereof, the method comprising: administering directly to the CNS a therapeutically effective amount of a composition comprising pooled human immunoglobulin G (IgG) to a subject with moderately severe Alzheimer's disease who is carrier of at least one APOE4 allele, wherein the amount of pooled human IgG is from 1 mg to 400 mg total dose per two week period, and wherein the amount is administered in one or more doses during the two week period after initiation of a therapeutic regimen.

In one aspect, the disclosure provides a use of a composition comprising pooled human immunoglobulin G (IgG) for the treatment of moderately severe Alzheimer's disease in a subject in need thereof for treatment comprising administration directly to the CNS of from 50 mg/kg to 400 mg/kg body weight of the subject per two week period, wherein the amount is administered in one or more doses during the two week period after initiation of a therapeutic regimen.

In one aspect, the disclosure provides a use of a composition comprising pooled human immunoglobulin G (IgG) for the treatment of moderately severe Alzheimer's disease in a subject in need thereof for treatment comprising administration directly to the CNS of from 1 mg to 400 mg total dose per two week period, wherein the amount is administered in one or more doses during the two week period after initiation of a therapeutic regimen.

In one aspect, the disclosure provides a use of a composition comprising pooled human immunoglobulin G (IgG) for the treatment of Alzheimer's disease in a subject in need thereof who is carrier of at least one APOE4 allele for administration directly to the CNS of from 50 mg/kg to 400 mg/kg body weight of the subject per two week period, wherein the amount is administered in one or more doses during the two week period after initiation of a therapeutic regimen.

In one aspect, the disclosure provides a use of a composition comprising pooled human immunoglobulin G (IgG) for the treatment of Alzheimer's disease in a subject in need thereof who is carrier of at least one APOE4 allele for administration directly to the CNS of from 1 mg to 400 mg total dose per two week period, wherein the amount is administered in one or more doses during the two week period after initiation of a therapeutic regimen.

In one aspect, the disclosure provides a use of a composition comprising pooled human immunoglobulin G (IgG) for the treatment of moderately severe Alzheimer's disease in a subject in need thereof who is carrier of at least one APOE4 allele for treatment administration directly to the CNS of from 50 mg/kg to 400 mg/kg body weight of the subject per two week period, wherein the amount is administered in one or more doses during the two week period after initiation of a therapeutic regimen.

In one aspect, the disclosure provides a use of a composition comprising pooled human immunoglobulin G (IgG) for the treatment of moderately severe Alzheimer's disease in a subject in need thereof who is carrier of at least one APOE4 allele for treatment administration directly to the CNS of from 1 mg to 400 mg total dose per two week period, wherein the amount is administered in one or more doses during the two week period after initiation of a therapeutic regimen.

In one embodiment of the methods and uses described above, the method further comprising the step of diagnosing the subject with moderately severe Alzheimer's disease prior to initiating the therapeutic regimen.

In one embodiment of the methods and uses described above, the APOE4 status of the subject is determined prior to starting the therapeutic regimen.

In one embodiment of the methods and uses described above, the subject is homozygous for the APOE4 allele.

In one embodiment of the methods and uses described above, the subject is heterozygous for the APOE4 allele.

In one embodiment of the methods and uses described above, the subject has an APOE4/APOE3 genotyope.

In one embodiment of the methods and uses described above, the subject has an APOE4/APOE2 genotyope.

In one embodiment of the methods and uses described above, the subject does not have an APOE4 allele.

In one embodiment of the methods and uses described above, the amount of pooled human IgG is administered in a single dose over the two week period.

In one embodiment of the methods and uses described above, the amount of pooled human IgG is administered in multiple doses over the two week period.

In one embodiment of the methods and uses described above, the one or more doses administered during the two week period are the same.

In one embodiment of the methods and uses described above, the one or more doses administered during the two week period are variable.

In one embodiment of the methods and uses described above, the dose is administered at least twice daily.

In one embodiment of the methods and uses described above, the dose is administered every day.

In one embodiment of the methods and uses described above, the dose is administered every other day.

In one embodiment of the methods and uses described above, the dose is administered twice a week.

In one embodiment of the methods and uses described above, the dose is administered every week.

In one embodiment of the methods and uses described above, the amount is administered by intravenous administration, subcutaneous administration, intramuscular administration, or intraperitoneal administration.

In one embodiment of the methods and uses described above, the amount is administered by intranasal administration, intrathecal administration, intracerebral administration, intracerebroventricular administration, epidural administration, or spinal administration.

In one embodiment of the methods and uses described above, the method further comprising administering a therapeutically effective amount of a second composition for treatment of Alzheimer's disease.

In one aspect, the disclosure provides a method of selecting a treatment regimen for a subject with Alzheimer's disease, the method comprising the steps of: (a) diagnosing the severity of the Alzheimer's disease as mildly severe, moderately severe or severe; (b) determining if the subject carries the APOE4 allele; and (c)assigning a treatment regimen comprising administration of pooled human immunoglobulin (IgG) if the subject has moderately severe Alzheimer's disease and is a carrier of an APOE4 allele or assigning a treatment regimen comprising administration of an anti-beta amyloid monoclonal antibody if the subject has mildly severe Alzheimer's disease and is not a carrier of an APOE4 allele.

In one embodiment of the methods described above, the subject is homozygous for the APOE4 allele.

In one embodiment of the methods described above, the subject is heterozygous for the APOE4 allele.

In one embodiment of the methods described above, the subject has an APOE4/APOE3 genotyope.

In one embodiment of the methods described above, the subject has an APOE4/APOE2 genotyope.

In one embodiment, the disclosure provides a method for treating Alzheimer's disease in a subject in need thereof, the method comprising: administering a therapeutically effective amount of a composition comprising pooled human immunoglobulin G (IgG) to a subject with moderate to moderately severe Alzheimer's , wherein moderate to moderately severe Alzheimer's patients have a Mini-Mental Status (MMSE) examination score of from 14 to 20, 14 to 21, 14 to 22, 14 to 23; 15 to 20, 15 to 21, 15 to 22, 15 to 23; 16 to 20, 16 to 21, 16 to 22, or 16 to 23, inclusive, wherein the amount of pooled human IgG is from 300 mg/kg to 800 mg/kg body weight of the subject per two week period, and wherein the amount is administered in one or more doses during the two week period after initiation of a therapeutic regimen. In some embodiments, the amount of pooled human IgG is from 200 mg/kg to 800 mg/kg body weight of the subject per two week period.

In other embodiments, the subject is a carrier of at least one APOE4 allele and has a Mini-Mental Status Examination (MMSE) score of from 14 to 20, 14 to 21, 14 to 22, 14 to 23; 15 to 20, 15 to 21, 15 to 22, 15 to 23; 16 to 20, 16 to 21, 16 to 22, or 16 to 23, inclusive.

### BRIEF DESCRIPTION OF DRAWINGS

**Figure 1** illustrates concentration-response curves for anti-ApoE4 ELISAs performed using plate-immobilized recombinant ApoE4 to measure anti-ApoE4 binding in pooled human plasma (1R01B00) and IgG purified from pooled human blood plasma (LE12K246). ELISA readings were blank-corrected by subtracting human serum albumin binding to coated wells from measured intensities of plasma pool and immunoglobulin G binding.
**Figure 2** is a schematic diagram depicting the flow of the study described in Example 2.
**Figure 3A-3E** illustrates a schedule of assessments made during the time period between screening of the subjects and month 9 of the trial.
**Figure 4** illustrates modified mini-mental state examination total score least square mean changes from baseline and 95 CIs estimated within the first mixed model. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 5** illustrates modified mini-mental state examination total score least square mean changes from baseline and 95 CIs estimated within the first mixed model in APOE-e4 carrier subjects. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 6** illustrates modified mini-mental state examination total score least square mean changes from baseline and 95 CIs estimated within the first mixed model in APOE-e4 non-carrier subjects. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 7** illustrates modified mini-mental state examination total score least square mean changes from baseline and 95 CIs estimated within the first mixed model in subjects with mild Alzheimer's disease. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 8A** illustrates modified mini-mental state examination total score least square mean changes from baseline and 95 CIs estimated within the first mixed model in subjects with moderate Alzheimer's disease. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 8B** illustrates modified mini-mental state examination total score least square mean changes from baseline and 95 CIs estimated within the first mixed model in subjects with moderate Alzheimer's disease (MMSE ≤ 20) who are carriers of an ApoE4 allele. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 8C** illustrates ADAS-Cog total score mean changes from baseline and 95 CIs in subjects with moderate Alzheimer's disease. p1 = p-value corresponding to the comparison between treatment group least square means IVIG (10%), 400 mg/kg and placebo at 18 months. p2 = p-value corresponding to the comparison between treatment group least square means IVIG (10%), 200 mg/kg and placebo at 18 months.
**Figure 9** provides change from baseline statistics for ADAS-Cog, ADCS-ADL, and 3MS analyses.
**Figure 10** shows [18F]-2-fluorodeoxyglucose (18F-FDG) positron emission tomography (PET) brain images from subjects treated with placebo (10A) and high dose IVIG (10B).
**Figure 11** provides a summary of imaging biomarker status for all cohorts and patient sub-populations.
**Figure 12** shows images of typical ventricular atrophy in the brains of Alzheimer's subjects treated with placebo (12A) and high dose IVIG (12B).
**Figure 13** provides a summary of mean change from baseline of Aβ42 and Aβ40 peptides levels in plasma for all cohorts and patient sub-populations.
**Figure 14** provides a summary of mean change from baseline of anti-Aβ42 and anti-Aβ40 antibody levels in plasma for all cohorts and patient sub-populations.
**Figure 15** provides a summary of Aβ42 peptide levels in plasma for all treatment cohorts.
**Figure 16** provides a summary of Aβ40 peptide levels in plasma for all treatment cohorts.
**Figure 17** provides a summary of mean change from baseline of Aβ42 and Aβ40 peptides levels in CSF for all cohorts and patient sub-populations.
**Figure 18** provides a summary of Aβ42 peptide levels in CSF for all treatment cohorts.
**Figure 19** provides a summary of total IgG levels in CSF for all treatment cohorts.
**Figure 20** provides a summary of anti-Aβ fibril antibody levels in CSF for all treatment cohorts.
**Figure 21** provides a summary of anti-Aβ oligomer antibody levels in CSF for all treatment cohorts
**Figure 22** provides a summary of anti-Aβ monomer antibody levels in CSF for all treatment cohorts.
**Figure 23** provides a summary of mean change from baseline of total IgG levels in CSF for all cohorts and patient sub-populations.
**Figure 24** provides a summary of mean change from baseline of anti-Aβ fibril, anti-Aβ oligomer, and anti-Aβ monomer antibody levels in CSF for all cohorts and patient sub-populations.
**Figure 25** provides a summary of Tau protein levels in CSF for all treatment cohorts.
**Figure 26** provides a summary of mean change from baseline of Tau and phosphorylated-Tau levels in CSF for all cohorts and patient sub-populations.
**Figure 27** provides a summary of phosphorylated-Tau protein levels in CSF for all treatment cohorts.
**Figure 28** provides a summary of the number of subjects with a decrease in hemoglobin level after treatment for all treatment cohorts.
**Figure 29** provides a summary of the number of serious side effects for all IVIG and placebo cohorts.
**Figure 30** provides a summary of the number of non-serious side effects for all IVIG and placebo cohorts.
**Figure 31** provides a summary of adverse side effects for all treatment cohorts.
**Figure 32** illustrates mean and 95% confidence intervals for differences between changes from baseline for subjects receiving high dose IVIG and placebo at 18 months for ADAS-Cog evaluation.
**Figure 33** illustrates mean and 95% confidence intervals for differences between changes from baseline for subjects receiving high dose IVIG and placebo at 18 months for 3MS evaluation.
**Figure 34** illustrates mean and 95% confidence intervals for differences between changes from baseline for subjects receiving high dose IVIG and placebo at 18 months for CGIC evaluation.
**Figure 35** illustrates mean and 95% confidence intervals for differences between changes from baseline for subjects receiving high dose IVIG and placebo at 18 months for clock drawing.
**Figure 36** illustrates mean and 95% confidence intervals for differences between changes from baseline for subjects receiving high dose IVIG and placebo at 18 months for Trail B evaluation.
**Figure 37** illustrates mean and 95% confidence intervals for differences between changes from baseline of ventricular volume for subjects receiving high dose IVIG, as determined by volumetric MRI.
**Figure 38** provides a summary of outcome correlation analysis performed by imaging biomarker analysis and primary endpoint analysis.
**Figure 39** provides a summary of ApoE4 genotype and allele distribution for the IVIG treatment study.
**Figure 40A** illustrates ADAS-Cog examination mean scores and 95 CIs estimated within the first mixed model in subjects with moderate (MMSE ≤ 22) or mild (MMSE ≥ 23) Alzheimer's disease at 0 months (baseline), 9 months, and 18 months of treatment with 400 mg/kg/2 week, 200 mg/kg/2 week, and placebo.
**Figure 40B** illustrates modified mini-mental state examination mean scores and 95 CIs estimated within the first mixed model in subjects with moderate (MMSE ≤ 22) or mild (MMSE ≥ 23) Alzheimer's disease at 0 months (baseline), 9 months, and 18 months of treatment with 400 mg/kg/2 week, 200 mg/kg/2 week, and placebo.
**Figure 41** provides a summary of total IgG levels in blood serum for all treatment cohorts.
**Figure 42** illustrates change from baseline in ADAS-Cog score, as mean difference from placebo, for individuals in the high dose (0.4 g/kg) and low dose (0.2 g/kg) IVIG treatment cohorts classified with florbetapir scores of equal to or greater than 1.2.
**Figure 43** illustrates change from baseline in modified MMSE score, as mean difference from placebo, for individuals in the high dose (0.4 g/kg) and low dose (0.2 g/kg) IVIG treatment cohorts classified with florbetapir scores of equal to or greater than 1.2.
**Figure 44** illustrates change from baseline in CSF Aβ42 polypeptide levels, as mean difference from placebo, for individuals in the high dose (0.4 g/kg) and low dose (0.2 g/kg) IVIG treatment cohorts classified with florbetapir scores of equal to or greater than 1.2.
**Figure 45** illustrates change from baseline in ADAS-Cog score, as mean difference from placebo, for individuals in the high dose (0.4 g/kg) and low dose (0.2 g/kg) IVIG treatment cohorts classified without amyloid plaques.

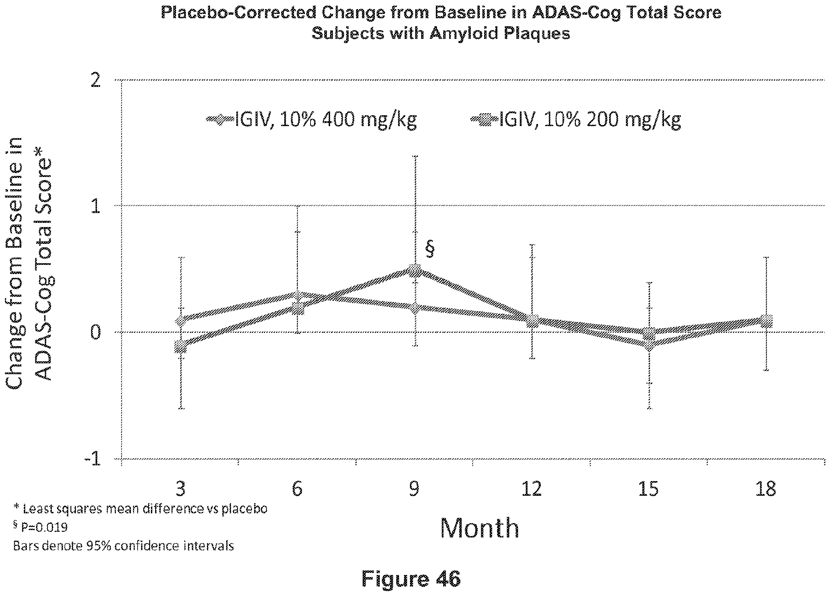

**Figure 46** illustrates change from baseline in ADAS-Cog score, as mean difference from placebo, for individuals in the high dose (0.4 g/kg) and low dose (0.2 g/kg) IVIG treatment cohorts classified with amyloid plaques.
**Figure 47** illustrates change from baseline in ADAS-CGIC score, as mean difference from placebo, for individuals in the high dose (0.4 g/kg) and low dose (0.2 g/kg) IVIG treatment cohorts classified without amyloid plaques.

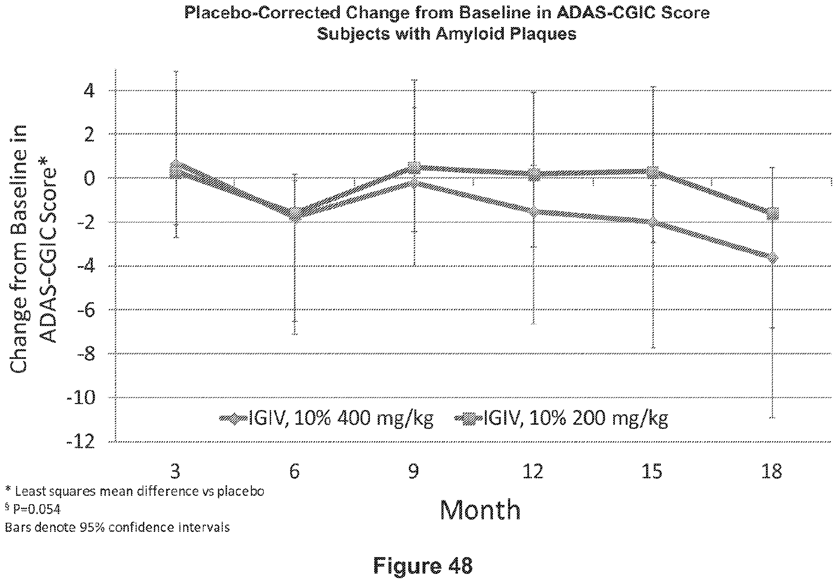

**Figure 48** illustrates change from baseline in ADAS-CGIC score, as mean difference from placebo, for individuals in the high dose (0.4 g/kg) and low dose (0.2 g/kg) IVIG treatment cohorts classified with amyloid plaques.
**Figure 49** illustrates change from baseline in modified MMSE score, as mean difference from placebo, for individuals in the high dose (0.4 g/kg) and low dose (0.2 g/kg) IVIG treatment cohorts classified without amyloid plaques.

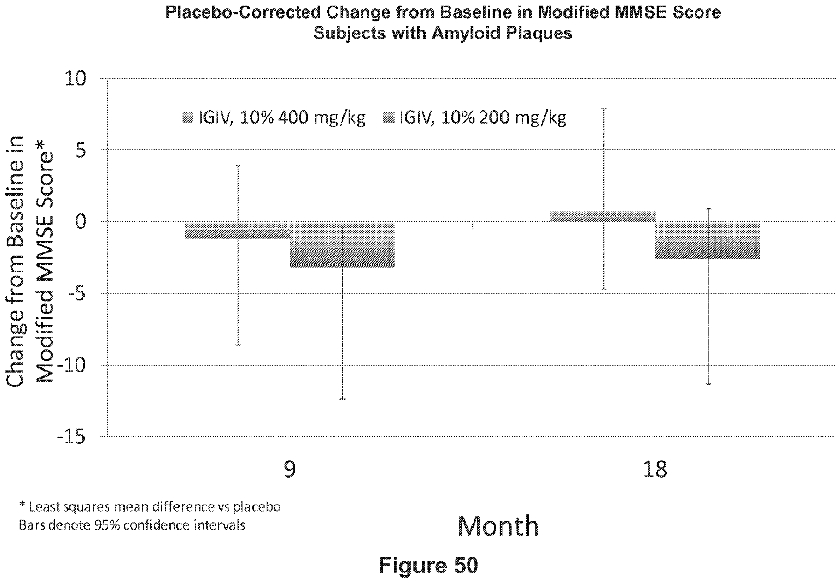

**Figure 50** illustrates change from baseline in modified MMSE score, as mean difference from placebo, for individuals in the high dose (0.4 g/kg) and low dose (0.2 g/kg) IVIG treatment cohorts classified with amyloid plaques.
**Figure 51** illustrates change from baseline in volumetric MRI, as mean difference from placebo, for individuals in the high dose (0.4 g/kg) and low dose (0.2 g/kg) IVIG treatment cohorts classified without amyloid plaques.

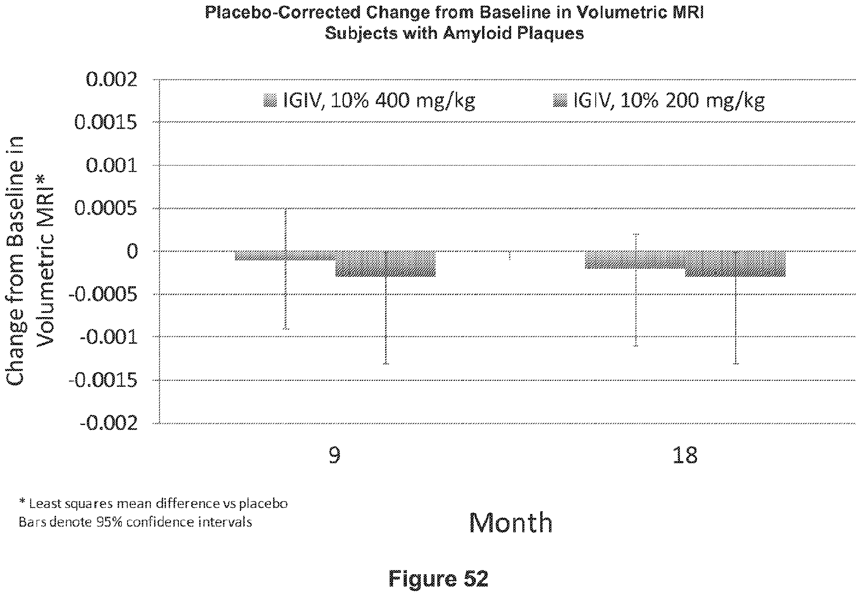

**Figure 52** illustrates change from baseline in volumetric MRI, as mean difference from placebo, for individuals in the high dose (0.4 g/kg) and low dose (0.2 g/kg) IVIG treatment cohorts classified with amyloid plaques.
**Figure 53** illustrates change from baseline in normalized composite SUVR, as mean difference from placebo, for individuals in the high dose (0.4 g/kg) and low dose (0.2 g/kg) IVIG treatment cohorts classified without amyloid plaques.

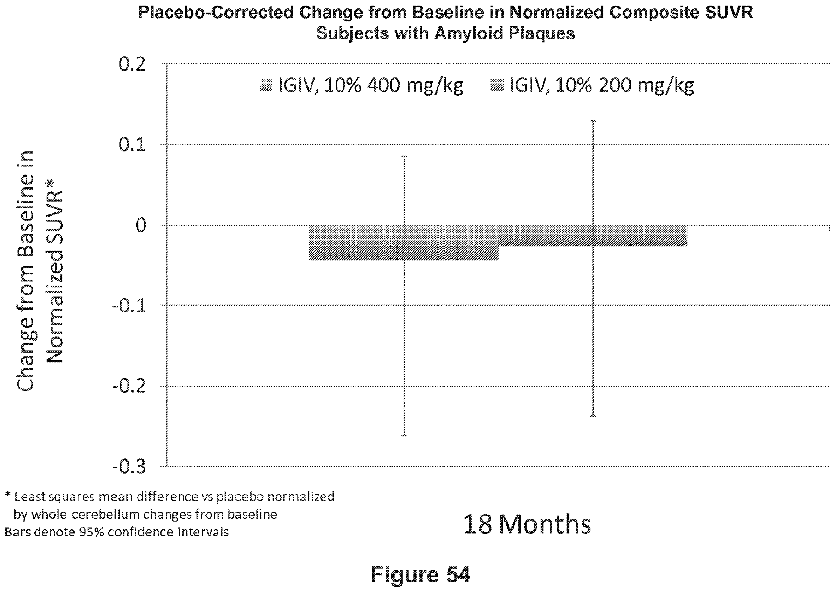

**Figure 54** illustrates change from baseline in normalized composite SUVR, as mean difference from placebo, for individuals in the high dose (0.4 g/kg) and low dose (0.2 g/kg) IVIG treatment cohorts classified with amyloid plaques.
**Figure 55** illustrates change from baseline in plasma Aβ40 polypeptide levels, as mean difference from placebo, for individuals in the high dose (0.4 g/kg) and low dose (0.2 g/kg) IVIG treatment cohorts classified without amyloid plaques.

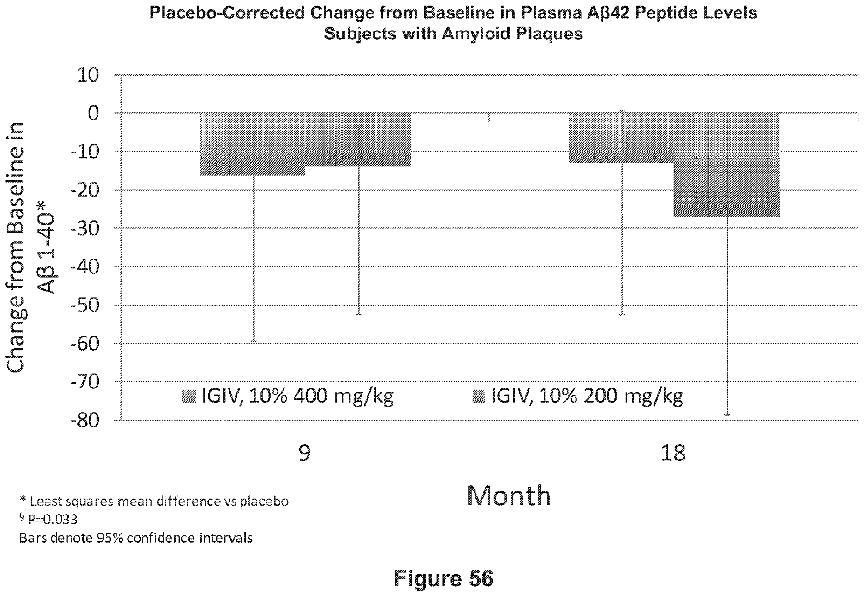

**Figure 56** illustrates change from baseline in plasma Aβ40 polypeptide levels, as mean difference from placebo, for individuals in the high dose (0.4 g/kg) and low dose (0.2 g/kg) IVIG treatment cohorts classified with amyloid plaques.

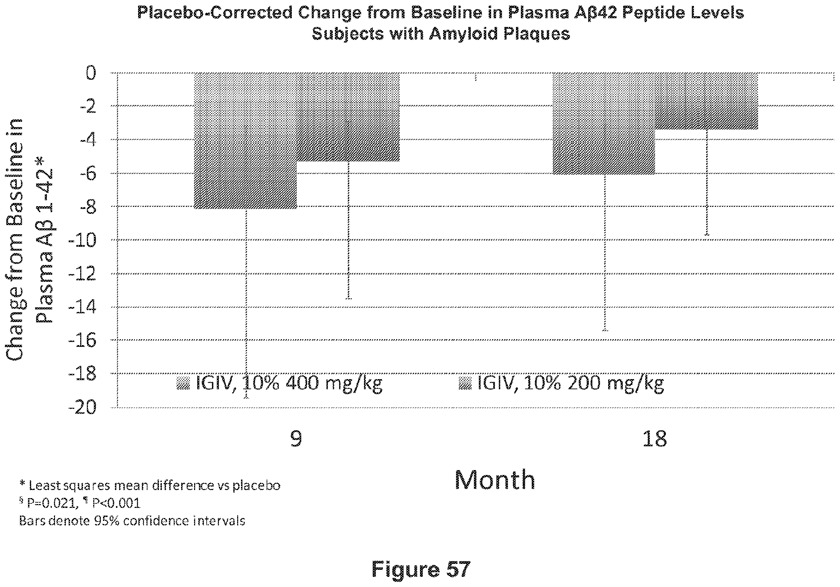

**Figure 57** illustrates change from baseline in plasma Aβ42 polypeptide levels, as mean difference from placebo, for individuals in the high dose (0.4 g/kg) and low dose (0.2 g/kg) IVIG treatment cohorts classified with amyloid plaques.

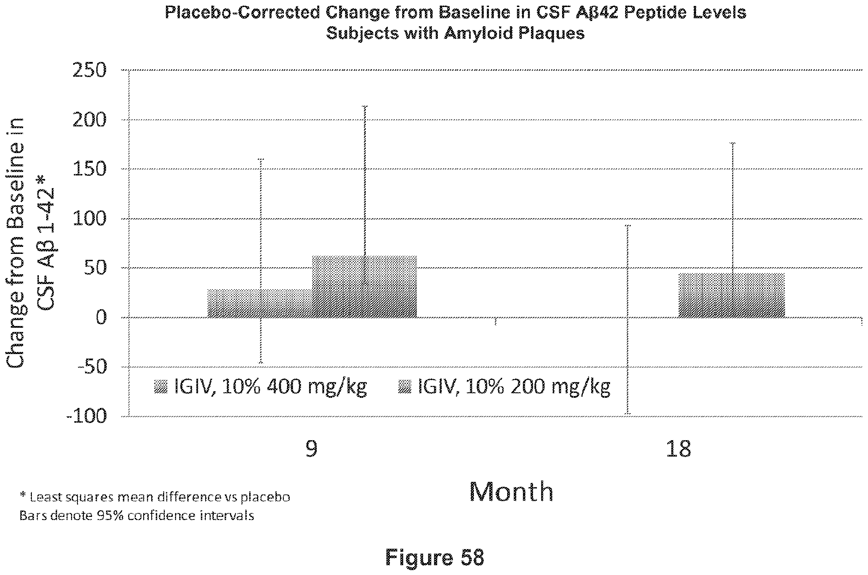

**Figure 58** illustrates change from baseline in CSF Aβ42 polypeptide levels, as mean difference from placebo, for individuals in the high dose (0.4 g/kg) and low dose (0.2 g/kg) IVIG treatment cohorts classified with amyloid plaques.

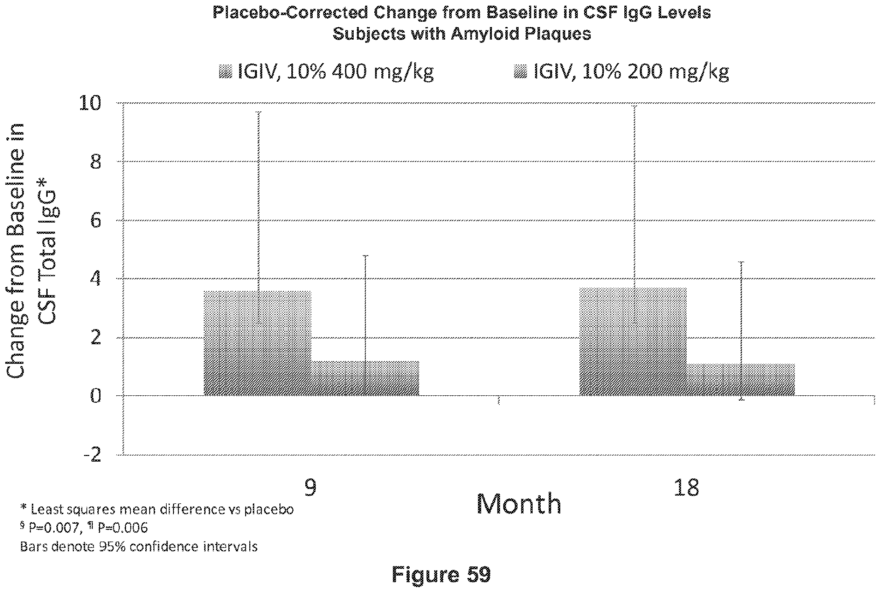

**Figure 59** illustrates change from baseline in CSF total IgG levels, as mean difference from placebo, for individuals in the high dose (0.4 g/kg) and low dose (0.2 g/kg) IVIG treatment cohorts classified with amyloid plaques.

### DETAILED DESCRIPTION OF INVENTION

### Introduction

The present disclosure describes the results of a study performed to evaluate the novel use of pooled immunoglobulin G, which is approved in the United States to treat various immunodeficiency and autoimmune disorders. IVIG is a biologic agent with anti-inflammatory and immuno-modulating properties containing human immunoglobulin G (IgG) antibodies derived from the blood plasma of healthy donors. Specifically, IVIG contains antibodies that bind to oligomeric and fibrillar beta amyloid, thus supporting the use of IVIG as an agent for passive immunotherapy of AD. Passive immunization does not require the recipients to produce antibodies themselves and can thereby circumvent the problem of inadequate antibody generation by older individuals. Unlike active vaccination, T-cell activation is not required to realize the full therapeutic benefits of passive immunization; this may therefore reduce but not entirely eliminate the possibility of reactive inflammatory reactions. Passive immunization could therefore provide a safe and effective alternative to active vaccination for the treatment of elderly AD patients.

Advantageously, it is shown herein that the administration of pooled immunoglobulin G to certain sub-populations of Alzheimer's patients (*e*.*g*., ApoE4 positive and/or patients with moderately severe Alzheimer's disease) results in a significant reduction in the progression of symptoms of dementia.

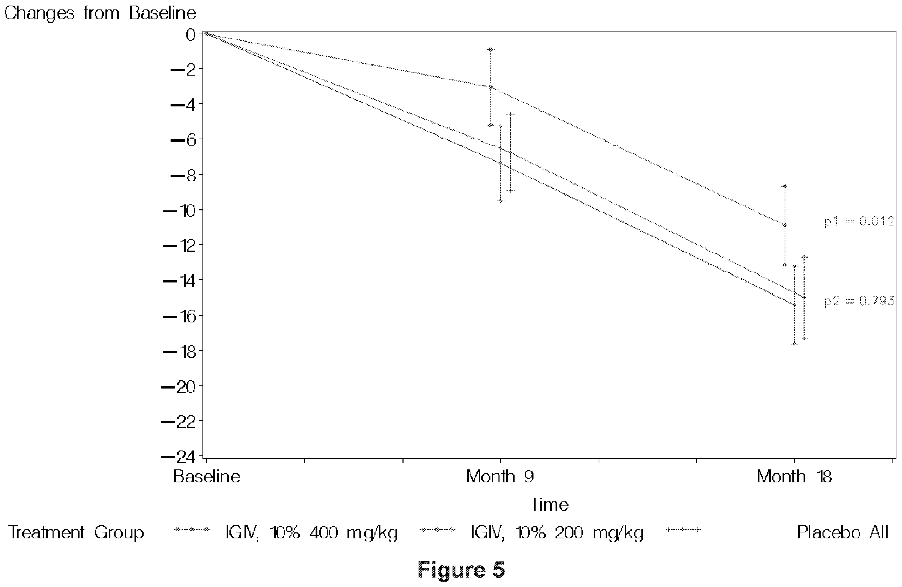

For example, as shown in Figure 5, administration of 400 mg IgG per kg body weight of the individual every two weeks (mg/kg/2 weeks) to Alzheimer's patients carrying at least one ApoE4 allele resulted in a statistically significant reduction in the progression of dementia, as compared to patients administered placebo (p1 = 0.012). However, administration of only 200 mg/kg/2 week IgG to Alzheimer's patients carrying at least one ApoE4 allele did not result in a statistically significant reduction in the progression of dementia, as compared to patients administered placebo (p1 = 0.793).

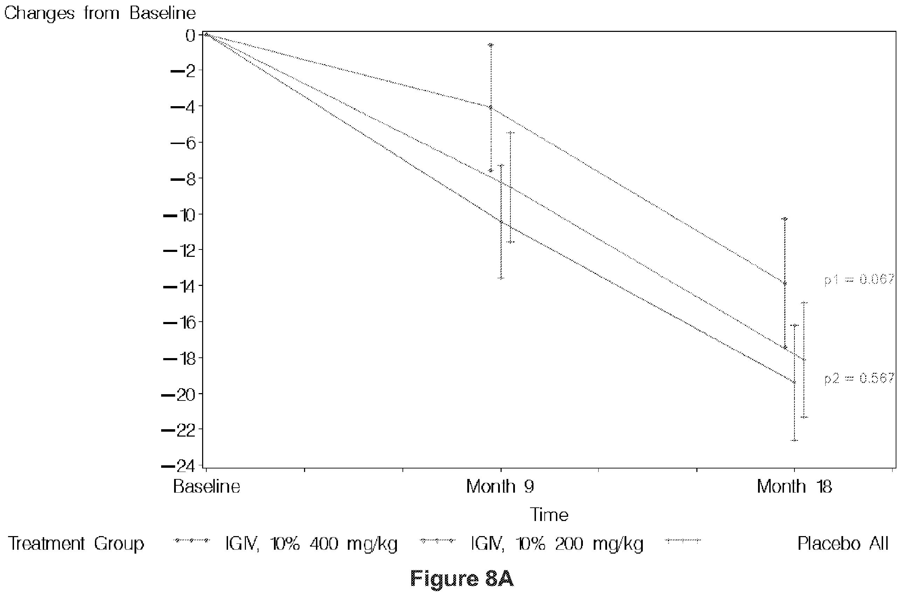

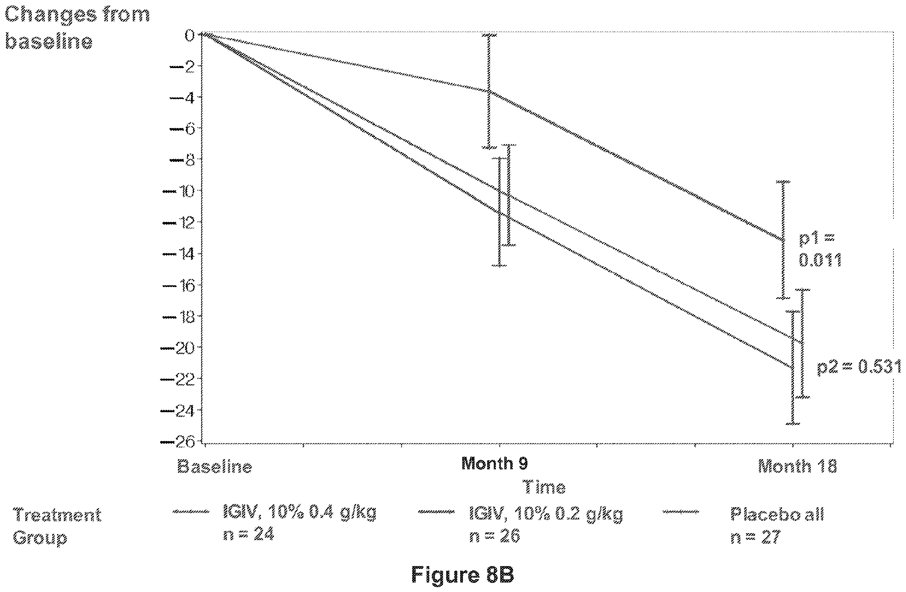

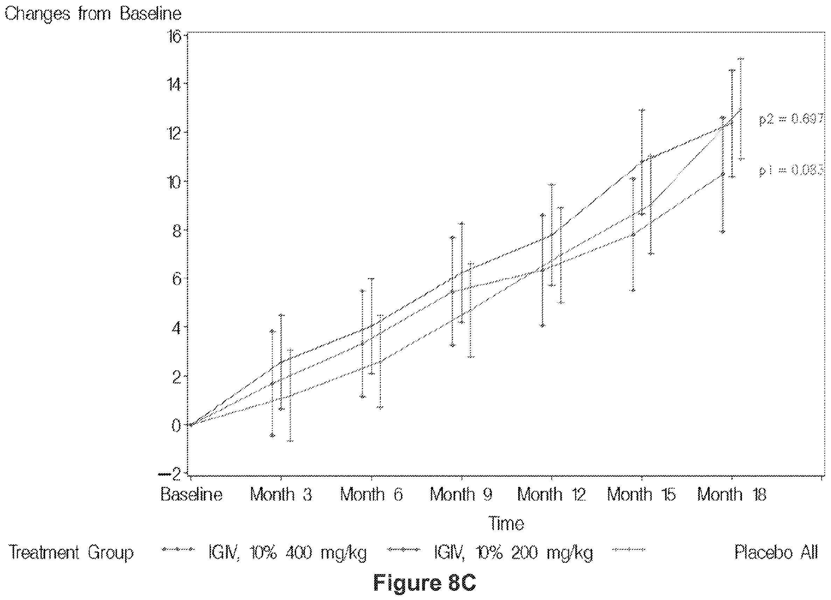

Likewise, as shown in Figure 8, administration of 400 mg IgG per kg body weight of the individual every two weeks (mg/kg/2 weeks) to Alzheimer's patients having moderate disease (defined as having an initial MMSE score between 16 and 20) resulted in a clear reduction in the progression of dementia, as compared to patients administered placebo, as evaluated by 3MS (p1 = 0.067; Figure 8A) and ADAS-Cog (p1 = 0.083; Figure 8C) cognitive assessment. However, administration of only 200 mg/kg/2 week IgG to Alzheimer's patients carrying at least one ApoE4 allele did not result in a clear reduction in the progression of dementia, as compared to patients administered placebo, as evaluated by 3MS (p2 = 0.567; Figure 8A) and ADAS-Cog (p2 = 0.697; Figure 8C) cognitive assessment.

The positive results seen for IVIG treatment of patients with moderate Alzheimer's disease becomes more pronounced when subjects having an initial MMSE score of 22 and/or 21 are included in the moderate Alzheimer's disease cohort. As reported in Table 1, administration of 400 mg/kg/2 weeks IVIG significantly slowed the progression of dementia in patients diagnosed with moderate Alzheimer's disease, as evaluated by ADAS-Cog (p = 0.046, MMSE ≤ 21; p = 0.006, MMSE ≤ 22) and 3MS (p = 0.09, MMSE ≤ 21; p = 0.029, MMSE ≤ 22) cognitive assessment.

Furthermore, analysis of the data reveals that by excluding subjects initially diagnosed with an MMSE score above 22 who are ApoE4 negative, treatment with high dose IVIG significantly reduced the progression of dementia in the subpopulation. As reported in **Table** 2 and **Table 3,** ApoE4 positive and/or patients with moderate Alzheimer's disease significantly benefited from administration of 400 mg/kg/2 week IVIG, as assessed by ADAS-Cog (p = 0.026 vs. placebo) and 3MS (p = 0.032 vs. placebo) cognitive assessment.

The results described above, which taken together suggest that administration of high doses of IgG (*e*.*g*., 300 mg/kg/2 weeks IVIG or more) to Alzheimer's patient sub-populations carrying an ApoE4 allele, and/or having moderate disease, is effective in slowing down the progression of symptoms of dementia.

These data are particularly surprising in light of results reported for anti-β-amyloid and anti-ApoE4 monoclonal therapy. These studies, summarized above, report that monoclonal therapy is more effective in non-ApoE4 carriers and in patients with mildly severe Alzheimer's disease. Moreover, several studies have reported negative outcomes (e.g., vasogenic edema and sulcal effusions) in ApoE4 carriers.

For example, at the October 2012 meeting of the American Neurological Association (ANA), it was reported that treatment of Bapineuzumab (anti-β-amyloid monoclonal antibody) over 78 weeks resulted in no change in ADAS-Cog or Disability Assessment for dementia (DAD) score in ApoE4 subjects, and provided no significant effect on the rate of change in MRI brain volume (BBSI) in subjects with moderate Alzheimer's disease. At the same October 2012 ANA meeting, it was reported that treatment of Solanezumab (anti-β-amyloid monoclonal antibody) provided no significant reduction in ADAS-Cog score was reported for subjects with moderate Alzheimer's disease.

It has been proposed that pooled immunoglobulin G *(e.g.,* IVIG) contains natural antibodies against β-amyloid. Relkin et al. 2009 (Neurobiol. Aging 30(11): 1728-36). In this study, pooled human IgG was administered intravenously (IVIG therapy) to eight subjects diagnosed with mild Alzheimer's disease (AD). The patients received IVIG therapy for 6 months, discontinued treatment, and then resumed treatment for 9 more months. It was found that β-amyloid antibodies in the serum from AD patients increased in proportion to IVIG dose and plasma levels of β-amyloid increased transiently after each infusion. Moreover, it is shown herein that a commercial preparation of pooled immunoglobulin G also contains natural antibodies against ApoE4 protein. Without being bound by theory, the combination of natural antibodies to various Alzheimer's-related proteins found in immunoglobulin G prepared from pooled plasma may contribute to the enhanced efficacy of pooled IgG as compared to anti-β-amyloid and anti-ApoE monoclonal antibody therapy in some cohorts.

### Definitions

As used herein, the terms "pooled human immunoglobulin G" and "pooled human IgG" refer to a composition containing polyvalent immunoglobulin G (IgG) purified from the blood/plasma of multiple donors, e.g., more than a hundred or more than a thousand blood donors. Typically, the composition will be at least 80% IgG (w/w, e.g., weight IgG per weight total protein), preferably at least 85%, 86%, 87%, 88%, 89%, 90%, 91%, 92%, 93%, 94%, 95%, 96%, 97%, 98%, or 99% IgG (w/w). In certain embodiments, the pooled human IgG composition contains intact IgG immunoglobulins. In other embodiments, the pooled human IgG composition contains IgG fragments, for example those prepared by treatment of intact antibodies with trypsin. In certain embodiments, the pooled human IgG compositions used in the treatments disclosed herein contain natural or synthetic modifications, e.g., post-translational modifications and/or chemical modifications. In one embodiment, the pooled human immunoglobulin G composition is formulated for intravenous administration (e.g., an IVIG preparation).

As used herein, the terms "ApoE4 positive" and "ApoE4 carrier" are used interchangeably and refer to a subject or patient population having at least one ApoE4 polymorphic allele. As used herein, an ApoE4 allele refers to an *apoE* allele *(e.g.,* gene coding for NCBI Reference Sequence: NM_000041.2) encoding a protein having arginine residues at positions 112 and 158 of the mature ApoE protein and positions 130 and 176 of the ApoE precursor polypeptide (NCBI Reference Sequence: NP_000032.1).

As used herein, the terms "moderate Alzheimer's disease" and "moderately severe Alzheimer's disease" are used interchangeably and refer to the state of Alzheimer's disease in a subject or patient population with a Mini-Mental Status examination (MMSE) score of from 14 to 20, inclusive. Preferred sub-populations of moderate and/or moderately severe Alzheimer's patients have a Mini-Mental Status (MMSE) examination score of from 14 to 20, 14 to 21, 14 to 22, 14 to 23; 15 to 20, 15 to 21, 15 to 22, 15 to 23; 16 to 20, 16 to 21, 16 to 22, or 16 to 23, inclusive.

In the context of the present disclosure, MMSE is employed as an exemplary test that can be used to identify an individual having moderate or moderately severe Alzheimer's disease who is likely to respond favorably to treatment with pooled human immunoglobulin G. The skilled artisan will recognize that a test other than MMSE may be used to classify a subject with moderate Alzheimer's disease as a candidate for treatment with pooled human immunoglobulin G (*e*.*g*., IVIG treatment), in connection with the methods described herein. For example, a subject or patient population is also considered to have moderate or moderately severe Alzheimer's disease if they have been assessed by a different test (*e*.*g*., via Modified Mini-Mental State (3MS) test, Cognitive subscale of the Alzheimer's Disease Assessment Scale (ADAS-Cog) assessment, ADCS-Clinical Global Impression of Change (ADCS-CGIC) assessment, or other known assessment of Alzheimer's disease) to have a score equivalent to an MMSE score corresponding to moderate Alzheimer's disease. The skilled artisan will understand how to correspond the results of an alternative test to characterize a subject as having moderate Alzheimer's disease, as defined above using the MMSE cognitive assessment.

As used herein, the term "two week period" refers to an interval of from about 10 to about 18 days within a pooled human IgG dosing cycle. In one embodiment, a two week period refers to a dosing interval of 14 days. In another embodiment, a two week period refers to a dosing interval of twice monthly. In another embodiment, a two week period refers to a dosing interval of about 26 times per year. In some embodiments, a two-week period refers to a dosing interval of 10 days, 11 days, 12 days, 13 days, 14 days, 15 days, 16 days, 17 days, 18 days, 24 times a year, 25 times a year, 26 times a year, 27 times a year, or 28 times a year. As understood by one of skill in the art, the two week period includes reasonable boundaries based on patient compliance.

In the context of the present disclosure, a dosage of pooled human IgG administered per two week period refers to the total amount of pooled human IgG administered during the two week period, whether it is administered in a single dose or multiple doses during the two week period. In one embodiment, the entire dosage is administered in a single dose once during the two week period. In another embodiment, the dosage is administered in two or more smaller doses during the two week period. For example, a 400 mg/kg/2 week dose encompasses a single dosage of 400 mg/kg administered once during the two week period, a dosage of 200 mg/kg administered twice during the two week period, a dosage of 100 mg/kg administered four times during the two week period, and other dosing regimens in which multiple doses totaling 400 mg/kg are administered during the two week period.

In some embodiments, the amount of pooled human IgG administered per two week period refers to an average of pooled human IgG administered per two week period over the duration of the treatment. In this fashion, in some embodiments, the pooled human IgG dose administered in consecutive two week periods varies. For example, in one embodiment, a subject administered alternating 200 mg/kg/2 week and 600 mg/kg/2 week pooled human IgG doses is said to have received 400 mg/kg/2 week period pooled human IgG. In some embodiments, the subject is administered a repeated dosage spread over period longer than 2 weeks, which averages out to a standard dose per two week period. In one embodiment, the subject is administered a dosage spread over a three week period. In another embodiment, the subject is administered a dosage spread over a period of a month. In other embodiments, the subject is administered a dosage spread over a 10 day, 11 day, 12 day, 13 day, 14 day, 15 day, 16 day, 17 day, 18 day, 19 day, 20 day, 21 day, 22 day, 23 day, 24 day, 25 day, 26 day, 27 day, 28 day, 29 day, 30 day, 31 day, 3 week, 4 week, 5 week, 6 week, 7 week, 8 week, 9 week, 10 week, 11 week, 12 week, 1 month, 2 month, or longer period. For example, in one embodiment, a subject administered repeating weekly doses of 100 mg/kg, 200 mg/kg, and 300 mg/kg pooled human IgG (*e*.*g*., 600 mg/kg/3 week period) is said to have received 400 mg/kg/ 2 week period.

As used herein, the terms "intranasal administration" and "nasal administration" refer to administration of a therapeutic composition to the nasal cavity of a subject such that a therapeutic agent in the composition is delivered directly to one or more epithelium located in the nose. In certain embodiments, intranasal administration is achieved using a liquid preparation (*e*.*g*., an aqueous preparation), an aerosolized preparation, or a dry powder preparation, each of which can be administered via an externally propelled or self-propelled (*e*.*g*., via inhalation) non-invasive nasal delivery device, or via a gel, cream, ointment, lotion, or paste directly applied to one or more nasal epithelium (*e*.*g*., olfactory epithelium or nasal respiratory epithelium).

The term "treatment" or "therapy" generally means obtaining a desired physiologic effect. The effect may be prophylactic in terms of completely or partially preventing a disease or condition or symptom thereof and/or may be therapeutic in terms of a partial or complete cure for an injury, disease or condition and/or amelioration of an adverse effect attributable to the injury, disease or condition and includes arresting the development or causing regression of a disease or condition. Treatment can also refer to any delay in onset, amelioration of symptoms, improvement in patient survival, increase in survival time or rate, improvement in cognitive function, *etc.* The effect of treatment can be compared to an individual or pool of individuals not receiving the treatment.

As used herein, a "therapeutically effective amount or dose" or "sufficient/effective amount or dose," refers to a dose that produces effects for which it is administered. The exact dose will depend on the purpose of the treatment, and will be ascertainable by one skilled in the art using known techniques (see, e.g., Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

In some embodiments, the progression or severity of Alzheimer's disease is measured by a cognitive assessment (*e*.*g*., Mini-Mental Status examination (MMSE), Cognitive subscale of the Alzheimer's Disease Assessment Scale (ADAS-Cog), Modified Mini-Mental State (3MS) examination, verbal fluency test, or adjunct neuropsychological test), a clinical, behavioral, and functional assessment (*e*.*g*., Alzheimer's Disease Cooperative Study (ADCS)-Activities of Daily Living (ADL), ADCS-Clinical Global Impression of Change (ADCS-CGIC), or Neuropsychiatric Inventory (NPI) assessment), a quality of life assessment (*e*.*g*., Logsdon Quality of Life in Alzheimer's Disease (QOL-AD) or Caregiver Burden Questionnaire), and/or a healthcare resource utilization assessment (*e*.*g*., an ADCS-Resource Use Inventory (ADCS-RUI) assessment).

As used here, the terms "dose" and "dosage" are used interchangeably and refer to the amount of active ingredient given to an individual at each administration. The dose will vary depending on a number of factors, including frequency of administration; size and tolerance of the individual; severity of the condition; risk of side effects; and the route of administration. One of skill in the art will recognize that the dose can be modified depending on the above factors or based on therapeutic progress. The term "dosage form" refers to the particular format of the pharmaceutical, and depends on the route of administration. For example, a dosage form can be a liquid or dry powder, formulated for intranasal administration.

As used herein, the term "dry powder composition" refers to a lyophilized or spray dried form of a therapeutic pooled human IgG formulation. In one embodiment, a dry powder composition contains less than 10%, 9%, 8%, 7%, 6%, 5%, 4%, 3%, 2%, 1%, or less residual water content.

A "control" is used herein, refers to a reference, usually a known reference, for comparison to an experimental group. One of skill in the art will understand which controls are valuable in a given situation and be able to analyze data based on comparisons to control values. Controls are also valuable for determining the significance of data. For example, if values for a given parameter vary widely in controls, variation in test samples will not be considered as significant.

As used herein, the term "about" denotes an approximate range of plus or minus 10% from a specified value. For instance, the language "about 20%" encompasses a range of 18-22%. As used herein, about also includes the exact amount. Hence "about 20%" means "about 20%" and also "20%."

Before the present disclosure is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a," "an," and "the" include plural referents unless the context clearly dictates otherwise. It is further noted that the claims may be drafted to exclude any optional element. As such, this statement is intended to serve as antecedent basis for use of such exclusive terminology as "solely," "only," and the like in connection with the recitation of claim elements, or use of a "negative" limitation.

### Manufacture of Pooled Immunoglobulin G

Immune globulin products from human plasma were first used in 1952 to treat immune deficiency. Initially, intramuscular or subcutaneous administration of immunoglobulin isotype G (IgG) isolated from plasma was the methods of choice. However, IgG products that could be administered intravenously, referred to as intravenous immunoglobulin (IVIG), were later developed to allow for the administration of larger amounts of IgG necessary for effective treatment of various diseases. Usually, IVIG contains the pooled immunoglobulin G (IgG) immunoglobulins from the plasma of multiple donors, *e*.*g*., more than a hundred or more than a thousand blood donors. These purified IgG products are primarily used in treating three main categories of medical conditions: (1) immune deficiencies: X-linked agammaglobulinemia, hypogammaglobulinemia (primary immune deficiencies), and acquired compromised immunity conditions (secondary immune deficiencies), featuring low antibody levels; (2) inflammatory and autoimmune diseases; and (3) acute infections.

Pooled human immunoglobulin G (IgG) is manufactured according to different processes depending upon the specific manufacturer. However, the origin of most manufacturing processes is found in the fourth installment of a series of seminal papers published on the preparation and properties of serum and plasma proteins, Cohn et al. (J. Am. Chem. Soc., 1946, 68(3): 459-475). This paper first described a method for the alcohol fractionation of plasma proteins (method 6), which allows for the isolation of a fraction enriched in IgG from human plasma.

The Cohn procedures were initially developed to obtain albumin at relatively high (95%) purity by fractional precipitation with alcohol. However, it was realized by Oncley et al. (J. Am. Chem. Soc., 71(2): 541-550 (1949)), Deutsch et al. (J. Biol. Chem., 164, 109-118 (1946)), and Kistler and Nitschmann (Vox Sang., 7, 414-424 (1962)), that particular protein precipitates (Fraction II and Fraction II+III) from the Cohn method could be used as a starting material for the purification of highly enriched immunoglobulin compositions. In order to achieve the higher purity and safety required for the intravenous administration of IgG compositions, several purification and polishing steps (*e*.*g*. solid phase adsorption, various chromatographic techniques, cross-flow-filtration, solvent and/or detergent treatment, heat treatment, and nanofiltration) have been added to IgG manufacturing processes after the alcohol fractionation steps.

Current IgG manufactures typically rely on either a Cohn method 6 Fraction II+III precipitate or a Kistler-Nitschmann precipitate A as the starting material for downstream processing. Both fractions are formed by a two step process in which proteins such as fibrinogen and Factor XIII are removed by an initial precipitation step (Fraction I precipitation) performed at high pH (7.2) with low ethanol concentration (8-10% v/v), followed by a second precipitation step in which IgG is precipitated from the Fraction I supernatant at pH 6.8 with 20-25% (v/v) ethanol (Fraction II+III) or at pH 5.85 with 19% ethanol (v/v) ethanol (precipitate A), while albumin and a significant portion of A1PI remain in the supernatant.

These methods, while laying the foundation for an entire industry of plasma derived blood proteins, were unable to provide IgG preparations having sufficiently high purity for the chronic treatment of several immune-related diseases, including Kawasaki syndrome, immune thrombocytopenic purpura, and primary immune deficiencies, without an undue occurrence of serious side effects. As such, additional methodologies employing various techniques, such as ion exchange chromatography, were developed to provide higher purity IgG formulations. Hoppe et al. (Munch Med Wochenschr, (34):1749-1752 (1967)), Falksveden (Swedish Patent No. 348942), and Falksveden and Lundblad (Methods of Plasma Protein Fractionation 1980) were among the first to employ ion exchange chromatography for this purpose.

### Administration

In one aspect, the present invention provides a method for treating Alzheimer's disease in a subject in need thereof by administering a therapeutically effective amount of a composition comprising pooled human immunoglobulin G (IgG) to a subject with moderately severe Alzheimer's disease and/or carrying an ApoE4 allele.

In one embodiment, the method includes administering a therapeutically effective amount of a composition comprising pooled human immunoglobulin G (IgG) to a subject with moderately severe Alzheimer's disease and/or carrying an ApoE4 allele, wherein the amount of pooled human IgG is from 300 mg/kg to 800 mg/kg body weight of the subject per two week period, and wherein the amount is administered in one or more doses during the two week period after initiation of a therapeutic regimen. In some embodiments, the amount of pooled human IgG is from 200 mg/kg to 800 mg/kg body weight of the subject per two week period.

In some embodiments, the pooled human immunoglobulin G (IgG) is administered to the subject via a systemic route. Non-limiting examples of systemic administration include intravenous administration, subcutaneous administration, intramuscular administration, or intraperitoneal administration.

In some embodiments, when administered systemically, the amount of pooled human IgG is from 300 mg/kg to 800 mg/kg body weight of the subject per two week period (mg/kg/2 week IgG). In one embodiment, the subject is systemically administered from 400 mg/kg to 800 mg/kg/2 week IgG. In one embodiment, the subject is systemically administered from 300 mg/kg to 700 mg/kg/2 week IgG. In one embodiment, the subject is systemically administered from 400 mg/kg to 700 mg/kg/2 week IgG. In one embodiment, the subject is systemically administered from 300 mg/kg to 600 mg/kg/2 week IgG. In one embodiment, the subject is systemically administered from 400 mg/kg to 600 mg/kg/2 week IgG. In one embodiment, the subject is systemically administered from 300 mg/kg to 500 mg/kg/2 week IgG. In one embodiment, the subject is systemically administered from 400 mg/kg to 500 mg/kg/2 week IgG. In one embodiment, the subject is systemically administered about 300, 350, 400, 450, 500, 550, 600, 650, 700, 750, or 800 mg/kg/2 week IgG.

In some embodiments, when administered systemically, the amount of pooled human IgG is from 200 mg/kg to 300 mg/kg body weight of the subject per two week period (mg/kg/2 week IgG). In one embodiment, the subject is systemically administered about 200 mg/kg/2 week. In one embodiment, the subject is systemically administered about 250 mg/kg/2 week.

In one embodiment, the method includes administering a therapeutically effective amount of a composition comprising pooled human immunoglobulin G (IgG) directly to the CNS of a subject with moderately severe Alzheimer's disease and/or carrying an ApoE4 allele, wherein the amount of pooled human IgG is from 400 mg/kg to 800 mg/kg body weight of the subject per two week period, and wherein the amount is administered in one or more doses during the two week period after initiation of a therapeutic regimen. Non-limiting examples of administration directly to the CNS include intranasal administration, intrathecal administration, intracerebral administration, intracerebroventricular administration, epidural administration, or spinal administration.

In one embodiment, the method includes administering a therapeutically effective amount of a composition comprising pooled human immunoglobulin G (IgG) directly to the CNS of a subject with moderately severe Alzheimer's disease and/or carrying an ApoE4 allele, wherein the amount of pooled human IgG is from 1 mg to 400 mg total dose per two week period, and wherein the amount is administered in one or more doses during the two week period after initiation of a therapeutic regimen. Non-limiting examples of administration directly to the CNS include intranasal administration, intrathecal administration, intracerebral administration, intracerebroventricular administration, epidural administration, or spinal administration.

Generally, when administered subcutaneously, the dosage of pooled human immunoglobulin G (IgG) is increased to account for lower bioavailability. In one embodiment, when administered subcutaneously, the dosage of pooled human IgG is increased by from 25% to 50%, as compared to a standard dosage used for intravenous administration. In one embodiment, when administered subcutaneously, the dosage of pooled human IgG is increased by from 30% to 45%, as compared to a standard dosage used for intravenous administration. In a specific embodiment, when administered subcutaneously, the dosage of pooled human IgG is increased by about 37%, as compared to a standard dosage used for intravenous administration.

In one embodiment, when administered subcutaneously, the amount of pooled human IgG is from 375 mg/kg to 1,000 mg/kg body weight of the subject per two week period (mg/kg/2 week IgG). In one embodiment, the subject is subcutaneously administered from 500 mg/kg to 1,000 mg/kg/2 week IgG. In one embodiment, the subject is subcutaneously administered from 375 mg/kg to 875 mg/kg/2 week IgG. In one embodiment, the subject is subcutaneously administered from 500 mg/kg to 875 mg/kg/2 week IgG. In one embodiment, the subject is subcutaneously administered from 375 mg/kg to 750 mg/kg/2 week IgG. In one embodiment, the subject is subcutaneously administered from 500 mg/kg to 750 mg/kg/2 week IgG. In one embodiment, the subject is subcutaneously administered from 375 mg/kg to 625 mg/kg/2 week IgG. In one embodiment, the subject is subcutaneously administered from 500 mg/kg to 625 mg/kg/2 week IgG. In one embodiment, the subject is subcutaneously administered about 375, 400, 450, 500, 550, 600, 650, 700, 750, 800, 850, 900, 950, or 1,000 mg/kg/2 week IgG.

In one embodiment, when administered subcutaneously, the amount of pooled human IgG is from 400 mg/kg to 1,100 mg/kg body weight of the subject per two week period (mg/kg/2 week IgG). In one embodiment, the subject is subcutaneously administered from 550 mg/kg to 1,100 mg/kg/2 week IgG. In one embodiment, the subject is subcutaneously administered from 400 mg/kg to 950 mg/kg/2 week IgG. In one embodiment, the subject is subcutaneously administered from 550 mg/kg to 950 mg/kg/2 week IgG. In one embodiment, the subject is subcutaneously administered from 400 mg/kg to 825 mg/kg/2 week IgG. In one embodiment, the subject is subcutaneously administered from 550 mg/kg to 825 mg/kg/2 week IgG. In one embodiment, the subject is subcutaneously administered from 400 mg/kg to 675 mg/kg/2 week IgG. In one embodiment, the subject is subcutaneously administered from 550 mg/kg to 675 mg/kg/2 week IgG. In one embodiment, the subject is subcutaneously administered about 400, 450, 500, 550, 600, 650, 700, 750, 800, 850, 900, 950, 1,000, 1,050, or 1,100 mg/kg/2 week IgG.

In one embodiment, when administered subcutaneously, the amount of pooled human IgG is from 450 mg/kg to 1,200 mg/kg body weight of the subject per two week period (mg/kg/2 week IgG). In one embodiment, the subject is subcutaneously administered from 600 mg/kg to 1,200 mg/kg/2 week IgG. In one embodiment, the subject is subcutaneously administered from 450 mg/kg to 1,050 mg/kg/2 week IgG. In one embodiment, the subject is subcutaneously administered from 600 mg/kg to 1,050 mg/kg/2 week IgG. In one embodiment, the subject is subcutaneously administered from 450 mg/kg to 900 mg/kg/2 week IgG. In one embodiment, the subject is subcutaneously administered from 600 mg/kg to 900 mg/kg/2 week IgG. In one embodiment, the subject is subcutaneously administered from 450 mg/kg to 750 mg/kg/2 week IgG. In one embodiment, the subject is subcutaneously administered from 600 mg/kg to 750 mg/kg/2 week IgG. In one embodiment, the subject is subcutaneously administered about 450, 500, 550, 600, 650, 700, 750, 800, 850, 900, 950, 1,000, 1,050, 1,100, 1,150, or 1,200 mg/kg/2 week IgG.

In one embodiment, the bioavailability of subcutaneously administered pooled human IgG can be increased by co-administration of a permeation agent, for example, a hyaluronidase such as PH20 (*see*, PCT Application Publication Numbers WO 2011/034604 and WO 2009/117085. One of skill in the art will readily be able to determine an appropriate dosage of permeation agent (*e*.*g*., a hyaluronidase) to be co-administered with the pooled human IgG.

Thus, in one embodiment, the pooled human IgG is co-formulated with the permeation agent (*e*.*g*., a hyaluronidase). In another embodiment, the pooled human IgG and permeation agent (*e*.*g*., a hyaluronidase) are formulated separately and mixed prior to subcutaneous administration. In another embodiment, the pooled human IgG and permeation agent (*e*.*g*., a hyaluronidase) are formulated and administered separately (*e*.*g*., the permeation agent is administered directly before or after administration of the pooled human IgG).

In one embodiment, when subcutaneously co-administered with a permeation agent (*e*.*g*., a hyaluronidase), the amount of pooled human IgG is from 300 mg/kg to 800 mg/kg body weight of the subject per two week period (mg/kg/2 week IgG). In one embodiment, the subject is subcutaneously co-administered a permeation agent (*e*.*g*., a hyaluronidase) and from 400 mg/kg to 800 mg/kg/2 week IgG. In one embodiment, the subject is subcutaneously co-administered a permeation agent (*e*.*g*., a hyaluronidase) and from 300 mg/kg to 700 mg/kg/2 week IgG. In one embodiment, the subject is subcutaneously co-administered a permeation agent *(e.g.,* a hyaluronidase) and from 400 mg/kg to 700 mg/kg/2 week IgG. In one embodiment, the subject is subcutaneously co-administered a permeation agent *(e.g.,* a hyaluronidase) and from 300 mg/kg to 600 mg/kg/2 week IgG. In one embodiment, the subject is subcutaneously co-administered a permeation agent *(e.g.,* a hyaluronidase) and from 400 mg/kg to 600 mg/kg/2 week IgG. In one embodiment, the subject is subcutaneously co-administered a permeation agent *(e.g.,* a hyaluronidase) and from 300 mg/kg to 500 mg/kg/2 week IgG. In one embodiment, the subject is subcutaneously co-administered a permeation agent (*e*.*g*., a hyaluronidase) and from 400 mg/kg to 500 mg/kg/2 week IgG. In one embodiment, the subject is subcutaneously co-administered a permeation agent (*e*.*g*., a hyaluronidase) and about 300, 350, 450, 500, 550, 600, 650, 700, 750, or 800 mg/kg/2 week IgG.

In one embodiment, when subcutaneously co-administered with a permeation agent (*e*.*g*., a hyaluronidase), the amount of pooled human IgG is from 200 mg/kg to 300 mg/kg body weight of the subject per two week period (mg/kg/2 week IgG). In one embodiment, the subject is subcutaneously co-administered a permeation agent (*e*.*g*., a hyaluronidase) and about 200 or 250 mg/kg/2 week IgG.

Generally, when administered directly to the central nervous system, the dosage of pooled human immunoglobulin G (IgG) can be reduced by a factor of from about 2 to 20, preferably by a factor of from about 4 to about 10 (*e*.*g*., about 6-fold). In some embodiments, when administered directly to the CNS, the amount of pooled human IgG is from 50 mg/kg to 400 mg/kg body weight of the subject per two week period (mg/kg/2 week IgG). In one embodiment, the subject is administered from 50 mg/kg to 350 mg/kg/2 week IgG directly to the CNS. In one embodiment, the subject is administered from 50 mg/kg to 300 mg/kg/2 week IgG directly to the CNS. In one embodiment, the subject is administered from 50 mg/kg to 250 mg/kg/2 week IgG directly to the CNS. In one embodiment, the subject is administered from 50 mg/kg to 200 mg/kg/2 week IgG directly to the CNS. In one embodiment, the subject is administered from 50 mg/kg to 150 mg/kg/2 week IgG directly to the CNS. In one embodiment, the subject is administered from 50 mg/kg to 100 mg/kg/2 week IgG directly to the CNS. In some embodiments, the subject is administered about 50, 75, 100, 125, 150, 175, 200, 225, 250, 275, 300, 325, 350, 375, or 400 mg/kg/2 week IgG directly to the CNS.

In some embodiments, when administered directly to the CNS, the amount of pooled human IgG is from 1 mg to 400 mg total dose per two week period. In one embodiment, the subject is administered from 1 mg to 350 mg total dose IgG directly to the CNS. In one embodiment, the subject is administered from 1 mg to 300 mg total dose IgG directly to the CNS. In one embodiment, the subject is administered from 1 mg to 250 mg total dose IgG directly to the CNS. In one embodiment, the subject is administered from 1 mg to 200 mg total dose IgG directly to the CNS. In one embodiment, the subject is administered from 1 mg to 150 mg total dose IgG directly to the CNS. In one embodiment, the subject is administered from 1 mg to 100 mg total dose IgG directly to the CNS. In one embodiment, the subject is administered from 1 mg to 50 mg total dose IgG directly to the CNS. In some embodiments, the subject is administered about 50 mg, 75 mg, 100 mg, 125 mg, 150 mg, 175 mg, 200 mg, 225 mg, 250 mg, 275 mg, 300 mg, 325 mg, 350 mg, 375 mg, or 400 mg IgG total dose per 2 week period directly to the CNS.

In some embodiments, where multiple dosages are administered to the subject over the two week period, each individual dose will be the same. In these embodiments, the individual dosages will be inversely proportional to the number of administrations. For example, to administer a total amount of 400 mg/kg IgG in two administrations over the two week period, two-200 mg/kg dosages are used. Whereas to deliver the same 400 mg/kg IgG in four administrations over the two week period, four-100 mg/kg dosages are used.

In some embodiments, where multiple dosages are administered to the subject over the two week period, each individual dose will vary. In one embodiment, a first high dose is administered at the start of the two week period and one or more smaller dosages are subsequently administered. For example, to administer a total amount of 400 mg/kg over the two week period, an initial dose of 200 mg/kg is administered at the start of the period and ten-20 mg/kg doses are administered subsequently.

By administering multiple dosages over the two week period, certain pharmacokinetic parameters can be controlled over the duration of the two-week period. For example, in one embodiment, a physician stabilizes the AUC (area under the curve) of pooled human IgG in the patient by administering, or prescribing administration, of one or more maintenance dosages over the two-week period. Likewise, in some embodiments, the bioavailability, Cₘₐₓ (peak concentration), Tₘₐₓ (time to achieve Cₘₐₓ), Cₘᵢₙ (lowest or trough concentration), and/or peak-trough fluctuation of IgG is controlled by administering multiple doses and/or varying the dose over the two week period.

In a particular embodiment, pooled human IgG may be administered in combination with another treatment for an age-related dementia, *e*.*g*., Alzheimer's disease. In certain embodiments, the treatment for an age-related dementia co-administered with pooled human IgG is administration of a cholinesterase inhibitor (*e*.*g*., ARICEPT (donepezil), EXELON (rivastigmine), RAZADYNE (galantamine), or COGNEX (tacrine), or an inhibitor of the NMDA-type glutamate receptor (*e*.*g*., memantine).

In further embodiments the second therapy is levodopa (L-DOPA). The second therapy can also be a dopamine agonist. Non-limiting examples of dopamine agonists include bromocriptine, pergolide, pramipexole, ropinirole, piribedil, cabergoline, apomorphine and lisuride. The second therapy can be a MAO-B inhibitor. Non-limiting examples of MAO-B inhibitors are selegiline and rasgiline. Addition second therapies can include amantaine, anticholinergic compositions, clozapine, modafinil, and non-steroidal anti-inflammatory drugs.

In further embodiments the second therapy is CAMPATH (alemtuzumab), ZENAPX (daclizumab), rituximab, dirucotide, BHT-3009, cladribine, dimethyl fumarate, estriol, laquinimod, pegylated interferon-β-1a, minocycline, statins, temsirolimus, teriflunomide, and low dose naltexone.

In one embodiment, the second therapeutic agent is co-formulated with the pooled human IgG (*e*.*g*., in the same composition). In another embodiment, the second therapeutic agent is administered in a different formulation from the pooled human IgG (*e*.*g*., in a second composition). In one embodiment, the second composition containing the second therapeutic is administered at the same time as the pooled human IgG composition (*e*.*g*., immediately proceeding, immediately following, or in a mixture). In another embodiment, the second composition containing the second therapeutic is administered at a different time, and/or via a different therapeutic regimen, as the pooled human IgG composition.

In certain embodiments the second therapy is psychotherapy. Non-limiting examples of psychotherapy are psychosocial intervention, behavioral intervention, reminiscence therapy, validation therapy, supportive psychotherapy, sensory integration, simulated presence therapy, cognitive retraining, and stimulation-oriented therapies such as art, music, pet, exercise, and recreational activities.

Furthermore, two or more second therapies can be combined with therapeutic IgG. For example, therapeutic pooled IgG can be combined with memantine and donepezil.

### Intranasal Administration

Intranasal administration of therapeutics has become an increasingly explored method for delivering therapeutic agents to the brain because it circumvents the blood-brain barrier (BBB) and is a localized, non-invasive method for delivery. Furthermore, intranasal administration offers the advantages, over more traditional methods of delivery (*e*.*g*., intravenous, subcutaneous, oral transmucosal, oral or rectal administration), of being simple to administer, providing rapid onset of action, and avoiding first-pass metabolism. Unfortunately, intranasal administration has only been shown effective for the transport of small molecules, and to a certain extent smaller Fc fusion proteins, to the brain. The delivery of larger molecules, such as intact antibodies, has not yet been demonstrated. The difficulty in transporting larger proteins is believed to be due to the limited permeability of tight junctions present in the olfactory epithelia, which likely excludes globular molecules having a hydrodynamic radius of more than 3.6 Å (Stevenson BR, et al., Mol Cell Biochem., 83(2):129-45(1988)).

U.S. Patent No. 5,624,898 to Frey describes compositions and methods for transporting neurologic agents, which promote nerve cell growth and survival or augment the activity of functioning cells, to the brain by means of the olfactory neural pathway. The neurological agents of the '898 patent are transported to the brain by means of the nervous system, rather than the circulatory system, so that potentially therapeutic agents that are unable to cross the blood-brain barrier may be delivered to damaged neurons in the brain. The compositions described in the '898 patent include a neurologic agent in combination with a pharmaceutical carrier and/or additive which promote the transfer of the agent within the olfactory system. The '898 patent does not teach intranasal administration of pooled human immunoglobulins.

PCT publications WO 2006/091332 and WO 2009/058957, both by Bentz et al., describe methods for the delivery of therapeutic polypeptides to the brain by fusing the polypeptide to an antibody or antibody fragment and administering the resulting fusion protein intranasally. Although the examples of the '332 and '957 PCT publications suggest that Fc-fusion "mimetibodies" may be administered intranasally, neither publication demonstrates delivery of larger, intact antibodies. In fact, the '957 PCT publication, published three years after the '332 PCT publication, states that "[i]n published delivery studies to date, intranasal delivery efficiency to the CNS has been very low and the delivery of large globular macromolecules, such as antibodies and their fragments, has not been demonstrated." The '957 PCT publication purports to solve this problem through the use of a permeability enhancer (e.g., membrane fluidizers, tight junction modulators, and medium chain length fatty acids and salts and esters thereof, as described below), which enhances intranasal administration to the central nervous system. Neither PCT publication teaches intranasal administration of pooled human immunoglobulins.

PCT publication WO 2003/028668 by Barstow et al., describes the treatment of immune-mediated diseases by alimentary administration (*i*.*e*., administration to the digestive tract) of pooled immunoglobulins. Although the '668 PCT publication defines alimentary administration of pooled immunoglobulins as including nasal administration, it is in the context of delivering the composition to the digestive tract. The '668 PCT publication does not teach the delivery of pooled human immunoglobulins to the brain via intranasal administration.

PCT publication WO 2001/60420 by Adjei et al., describes aerosol formulations of therapeutic polypeptides, including immunoglobulins, for pulmonary delivery. These aerosolizable compositions are formulated such that after oral or nasal inhalation, the therapeutic agent is effectively delivered to the patient's lungs. The '420 PCT publication does not teach the delivery of therapeutic agents to the brain via intranasal administration.

Intranasal (IN) administration is an advantageous mode of delivering a drug to the brain because it is non-invasive and there is a direct connection between the olfactory system and the brain. Intranasal administration of IgG (INIG) to treat neurological diseases is particularly advantageous because the direct connection between the olfactory system and the brain obviates delivery concerns associated with the blood-brain barrier (BBB) and minimizes systemic exposure to the drug, thereby minimizing side effects of the drug. Furthermore, IN delivery allows compositions such as powders, granules, solutions, ointments, and creams, thereby obviating the need for intravenous and intramuscular administration. For example, when a drug is administered intranasally, it is transported through the nasal mucosa and along the olfactory neural pathway. The drug can be administered alone or can be combined with a carrier molecule(s) to promote transport through the nasal mucosa and along the olfactory neural pathway. The drug can also be administered in combination with an absorption enhancer. Absorption enhancers promote the absorption of the drug through the nasal mucosa and along the olfactory neural pathway. Furthermore, additional molecules can be added to facilitate drug transport across the olfactory neural pathway.

IN administration can also be used to deliver therapeutic drugs to the brain via the trigeminal pathway. Specifically, IN administration can be used to deliver IgG via the trigeminal pathway. The olfactory and trigeminal nerves receive high concentrations of a drug with IN administration because the absorbent respiratory and olfactory pseudoepithelium are innervated by the trigeminal nerve. These nerves can then transport the drug into the brain and other connected structures. For example, the trigeminal nerve branches directly or indirectly reach the maxillary sinus, brainstem, hindbrain, cribriform plate, forebrain (e.g., cortex and diencephalon), orofacial structures (e.g., teeth, masseter muscle, and the temporomandibular joint), midbrain, cerebellum, cervical spinal cord, thoracic spinal cord, and lumbar spinal cord. Accordingly, INIG can be carried across the trigeminal pathway to reach and treat neurological diseases.

Many types of intranasal delivery devices can be used to practice the methods provided herein. In some embodiments, the delivery device is an intranasal device for the administration of liquids. Non-limiting examples of devices useful for the administration of liquid compositions (*e*.*g*., liquid pooled IgG compositions) include vapor devices (*e*.*g*., vapor inhalers), drop devices (*e*.*g*., catheters, single-dose droppers, multi-dose droppers, and unit-dose pipettes), mechanical spray pump devices (*e*.*g*., squeeze bottles, multi-dose metered-dose spray pumps, and single/duo-dose spray pumps), bi-directional spray pumps (*e*.*g*., breath-actuated nasal delivery devices), gas-driven spray systems/atomizers (*e*.*g*., single- or multi-dose HFA or nitrogen propellant-driven metered-dose inhalers, including traditional and circumferential velocity inhalers), and electrically powered nebulizers/atomizers (*e*.*g*., pulsation membrane nebulizers, vibrating mechanical nebulizers, and hand-held mechanical nebulizers). In some embodiments, the delivery device is an intranasal device for the administration of powders or gels. Non-limiting examples of devices useful for the administration of powder compositions (e.g., lyophilized or otherwise dried pooled IgG compositions) include mechanical powder sprayers (e.g., hand-actuated capsule-based powder spray devices and hand-actuated powder spray devices, hand actuated gel delivery devices), breath-actuated inhalers (e.g., single- or multi-dose nasal inhalers and capsule-based single- or multi-dose nasal inhalers), and insufflators (e.g., breath-actuated nasal delivery devices).In some embodiments, the pooled human immunoglobulins are preferentially administered to the olfactory area, located in the upper third of the nasal cavity, and particularly to the olfactory epithelium. Fibers of the olfactory nerve are unmyelinated axons of olfactory receptor cells, which are located in the superior one-third of the nasal cavity. The olfactory receptor cells are bipolar neurons with swellings covered by hair-like cilia that project into the nasal cavity. At the other end, axons from these cells collect into aggregates and enter the cranial cavity at the roof of the nose. Surrounded by a thin tube of pia, the olfactory nerves cross the subarachnoid space containing CSF and enter the inferior aspects of the olfactory bulbs. Once the pooled human immunoglobulin is dispensed into the nasal cavity, the immunoglobulin can undergo transport through the nasal mucosa and into the olfactory bulb and interconnected areas of the brain, such as the hippocampal formation, amygdaloid nuclei, nucleus basalis of Meynert, locus ceruleus, the brain stem, and the like (*e*.*g*., Johnson et al., Molecular Pharmaceutics (2010) 7(3):884-893).

In certain embodiments, pooled human immunoglobulin is administered to tissue innervated by the trigeminal nerve. The trigeminal nerve innervates tissues of a mammal's (*e*.*g*., human) head including skin of the face and scalp, oral tissues, and tissues surrounding the eye. The trigeminal nerve has three major branches, the ophthalmic nerve, the maxillary nerve, and the mandibular nerve. In some embodiments, the methods provided herein include targeted administration of pooled human immunoglobulin to one or more of these trigeminal branches, *i*.*e*. the trigeminal pathway. In some embodiments, the methods provided herein include targeted administration of pooled human immunoglobulin to the maxillary sinus, thereby reaching the brainstem, hindbrain, cribriform plate, forebrain (*e*.*g*., cortex and diencephalon), midbrain, cerebellum, cervical spinal cord, thoracic spinal cord, and lumbar spinal cord through the trigeminal pathway. In certain embodiments, methods provided herein include targeted administration of pooled human immunoglobulin for treatment of a disorder of the CNS (*e*.*g*., Alzheimer's disease).

In some embodiments, the pooled human immunoglobulin is administered to nasal tissues innervated by the trigeminal nerve, for example, to nasal tissues including the sinuses, the inferior two-thirds of the nasal cavity and the nasal septum. In certain embodiments, the pooled human immunoglobulin is targeted to the inferior two-thirds of the nasal cavity and/or the nasal septum.

In some embodiments, the pooled human immunoglobulin is administered to one or both maxillary sinus of the individual. Methods and devices for administration to the maxillary sinus are known in the art, for example, *see* United States Patent Application Publication Number 2011/0151393.

The maxillary sinus is in fluid communication with the patient's nasal cavity and comprises right and left maxillary sinuses. Each maxillary sinus communicates with the corresponding nasal passage via the orifice of the maxillary sinus. The maximum volume of the maxillary sinus in adults is approximately 4 to 15 ml, though individual sinuses may comprise volumes outside of this range.

The pathway from the nasal passages to the corresponding orifice of maxillary sinus, and ultimately to the corresponding maxillary sinus, allows for a device to be inserted into the nasal passage to the orifice of the maxillary sinus, whereupon at least one effective amount or dose of pooled human immunoglobulins may be administered and delivered into the maxillary sinus. The pathway to the maxillary sinus is tortuous and requires: traversing the nostril, moving through the region between the lower and middle concha, navigating over and into the semilunar hiatus, traveling superiorly into the maxillary sinus opening, resisting the ciliated action of the ostium/tube passing into the maxillary sinus and ultimately moving into the sinus itself.

Since the trigeminal nerve passes through the maxillary sinus, the pooled human immunoglobulins in the maxillary sinus after delivery therein will be moved along the trigeminal nerve to structures innervated by the trigeminal nerve. In this fashion, pooled human IgG administered to one or both of the maxillary sinuses is delivered to the brain via the trigeminal nerve.

In one embodiment the non-invasive intranasal delivery device delivers a liquid drop of a pooled human IgG composition to the nasal cavity of a subject. In a particular embodiment, the non-invasive intranasal delivery device delivers a liquid drop of pooled human IgG directly to a nasal epithelium of the subject. In a more specific embodiment, the non-invasive intranasal delivery device delivers a liquid drop of pooled human IgG directly to the olfactory epithelium of the subject. In one embodiment, the liquid drop is administered by tilting the head of the subject back and administering the drop into a nare of the subject. In another embodiment, the liquid drop is administered by inserting the tip of a non-invasive intranasal delivery device into a nare of the subject and squirting or spraying the drop into the nasal cavity of the subject.

In another embodiment, the non-invasive intranasal delivery device delivers a liquid or a powder aerosol of a pooled human IgG composition to the nasal cavity of a subject. In a particular embodiment, the non-invasive intranasal delivery device delivers a liquid or a powder aerosol of pooled human IgG directly to a nasal epithelium of the subject. In a more specific embodiment, the non-invasive intranasal delivery device delivers a liquid or a powder aerosol of pooled human IgG directly to the olfactory epithelium of the subject.

In another embodiment, the non-invasive intranasal delivery device delivers a dry powder composition of pooled human IgG composition to the nasal cavity of a subject. In a particular embodiment, the non-invasive intranasal delivery device delivers a dry powder composition of pooled human IgG directly to a nasal epithelium of the subject. In a more specific embodiment, the non-invasive intranasal delivery device delivers a dry powder composition of pooled human IgG directly to the olfactory epithelium of the subject.

In one embodiment, the intranasal device is a single-use, disposable device. In another embodiment, the intranasal device is a multi- or repeat-use device. In certain embodiments, the single-use or multi-use device is pre-metered. In a specific embodiment, the single-use or multi-use device is pre-filled. In certain embodiments, the multi- or repeat-use device is refillable. In certain embodiments, the device is refilled by inserting a pooled human IgG composition into a chamber of the device. In other embodiments, a chamber of the multi- or repeat-use device designed to hold the pooled human IgG composition is replaced with a new, pre-filled chamber.

Non-limiting examples of commercial intranasal delivery devices include the Equadel® nasal spray pump (Aptar Pharma), the Solovent dry powder device (BD Technologies), the Unidose nasal drug delivery device (Consort Medical PLC), the NasoNeb® Nasal Nebulizer (Medlnvent, LLC), the VeriDoser® nasal delivery device (Mystic Pharmaceuticals), the VRx2™ nasal delivery device (Mystic Pharmaceuticals), the DirectHaler™ Nasal device (Direct-Haler A/S), the TriViar™ single-use unit-dose dry powder inhaler (Trimel Pharmaceuticals), the SinuStar™ Aerosol Delivery System (Pari USA), the Aero Pump (Aero Pump GmbH), the Fit-Lizer™ nasal delivery device (Shin Nippon Biomedical Laboratories), the LMA MAD Nasal™ device (LMA North America, Inc.), the Compleo intranasal bioadhesive gel delivery system (Trimel Pharmaceuticals), Impel's Pressurized Olfactory Delivery (POD) device (Impel Neuropharma), the ViaNase™ electronic atomizer (Kurve Technology, Inc.), the OptiNose powder delivery device (OptiNose US Inc.), and the Optinose liquid delivery device (OptiNose US Inc.)

### Examples

### Example 1 - Presence of anti-ApoE4 Antibodies in Pooled Human IgG

The apolipoprotein E (*apoE*) gene has been genetically linked to the onset of Alzheimer's disease (Ertekin-Taner N., Neurol Clin., 25:611-667 (2007)). Moreover, polymorph ApoE4 (a major isoform of the apoE gene, characterized by residues R112 and R158) has been indicated in the etiology of Alzheimer's disease, where it may play a role in differentially modulating amyloid-β (Aβ) levels through the formation of an ApoE4-Aβ complex. Several investigators, noting these correlations, have explored the use of anti-ApoE4 monoclonal antibodies for the treatment of Alzheimer's disease (Tai et al., J Biol Chem. 2013 Feb 22;288(8):5914-26; Kim et al., J Exp Med. 2012 Nov 19;209(12):2149-56).

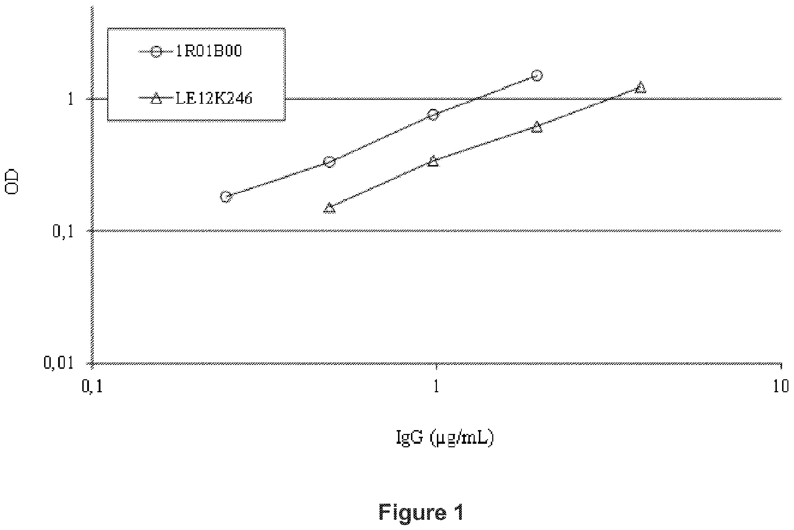

Anti-ApoE ELISAs were performed to determine if anti-ApoE4 antibodies are present in commercially available plasma-derived immunoglobulin G preparations. Briefly, the content of anti-ApoE4 antibodies in pooled human plasma (1R01B00) and a commercial 10% IVIG liquid commercial product (LE12K246) prepared from pooled human plasma was determined. As shown in Figure 1, anti-ApoE4 antibodies were detected in both pooled human plasma (circles) and IVIG product (triangles), with several-fold enrichment in the final IVIG preparation.

### Example 2 - Administration of Pooled Human Immunoglobulin G for Treatment of Alzheimer's Disease

A randomized, double-blind, placebo-controlled, two-arm, parallel study of the safety and effectiveness of intravenous immune globulin G (IVIG) administration for the treatment of mile-to-moderate Alzheimer's disease was performed. The primary objective of the study was To determine whether IVIG, 10% treatment either at a dose of 400 mg/kg body weight (BW)/2 weeks or 200 mg/ kg BW/2 weeks for 18 months slows the rate or prevents the progression of dementia symptoms in subjects with mild-to-moderate Alzheimer's Disease (AD) as compared to placebo, as measured by the cognitive subscale of the Alzheimer's Disease Assessment Scale (ADAS-Cog) and the Alzheimer's Disease Cooperative Study (ADCS)-Activities of Daily Living (ADL).

Other objectives of the study included: to whether IVIG, 10% treatment either at a dose of 400 mg/kg BW/2 weeks or 200 mg/kg BW/2 weeks for 9 months results in a significantly slower rate of progression of dementia symptoms in subjects with mild-to-moderate AD as compared to placebo, based on ADAS-Cog and ADCS-ADL; to compare the effect of 400 mg/kg BW/2 weeks to 200 mg/kg BW/2 weeks on the rate of progression of dementia symptoms as determined by ADAS-Cog and ADCS-ADL at 9 and 18 months; to evaluate the effect of IVIG, 10% treatment for 9 and 18 months on additional measures including the ADCS-Clinical Global Impression of Change (ADCS-CGIC), Modified Mini-Mental State (3MS) Examination and adjunct neuropsychological tests (cognition), Neuropsychiatric Inventory (NPI) (behavior), and Logsdon Quality of Life in Alzheimer's Disease (QOL-AD) (quality of life); to assess the short-term pharmacoeconomic impact of IVIG, 10% administration for 9 and 18 months as add-on pharmacotherapy in mild-to-moderate AD using ADCS-Resource Use Inventory (ADCS-RUI); to assess the impact of IVIG, 10% treatment in mild-to-moderate AD subjects for 9 and 18 months on the quality of life of their caregivers using the Caregiver Burden Questionnaire; to assess the safety and tolerability of two doses of IVIG, 10% administered biweekly for 9 and 18 months in subjects with mild-to-moderate AD; and to evaluate a panel of plasma, cerebrospinal fluid (CSF), and imaging biomarkers as a means of determining whether IVIG, 10% has anti-amyloid effects and whether changes in biomarkers from baseline to 9 months predict subsequent stabilization or improvement in cognitive, behavioral, and functional outcome measures at 18 months.

Briefly, 390 probable AD subjects with dementia of mild-to-moderate severity were enrolled and randomized at 44 centers within the ADCS consortium in the US and Canada. At screening, each subject underwent a mini-mental state examination (MMSE), as well as physical, neurological, and laboratory assessments. After eligibility has been determined, baseline cognitive and clinical assessments, as well as safety and biomarker/imaging assessments, were conducted prior to randomization.

Key inclusion criteria for the study included that the subjects (males and females) were of age 50 to 89 years at the time of screening, had been diagnosed with probably Alzheimer's disease, had mild (defined as MMES 21-26) to moderate (defined as MMSE 16-20) dementia at the time of screening, had received stable doses of AD medication (acetylcholinesterase inhibitor and/or memantine) for at least 12 weeks prior to screening, and who had an able caregiver (study partner) who could help facilitate the subject's participation.

Key exclusion criteria for the study included that the subjects had non-Alzheimer's dementia, currently resided in a skilled nursing facility, had clinically significant cardiovascular problems (*e*.*g*. congestive heart failure, clotting disorder, uncontrolled hypertension, recent unstable angina, or myocardial infarction), had recent central or peripheral thrombosis and/or thromboembolic disease, had specific findings on a brain MRI *(e.g.,* 2 or more microhemorrhages, major stroke, or multiple lacunae), had recent head trauma with loss of consciousness, contusion (brain), or open head injury, had an uncontrolled seizure disorder (e.g., ≥ 2 breakthrough seizures per year despite adequate antiepileptic drug treatment), had a modified Hachinski score > 4 at time of screening, had a malignancy, with the exception of the following: adequately treated basal cell or squamous cell carcinoma of the skin, carcinoma in situ of the cervix, and stable prostate cancer not requiring treatment, has an active autoimmune or neuro-immunologic disorder, had an untreated major depression, psychosis, or other major psychiatric disorder(s), had poorly controlled diabetes (HbA1c > 7.0%), had an active renal disease, had another clinically significant lab abnormalities (including abnormally high plasma viscosity levels; positive serology for HBV, HCV, or HIV), has a severe IgA deficiency (<7 mg/dL), had received IVIG treatment within the 5 years prior to screening, had received treatment with any investigational biologic(s) (e.g. active immunization or passive immunotherapies with monoclonal or polyclonal antibodies) for AD at any time, or any investigational drug(s) for AD within 3 months prior to screening, or was currently or recently participating in any other investigational drug or device studies.

Subjects meeting eligibility criteria and successfully completing baseline assessments were randomly assigned in a 1:1:1 ratio to receive intravenous (IV) infusions of either of two doses of IVIG, 10% or placebo (0.25% human albumin) every two weeks for 70 weeks (a total of 36 infusions) as an add-on to conventional Food and Drug Administration (FDA)-approved AD pharmacotherapy. The treatment groups were assigned as follows: Group 1: IVIG, 10% 400 mg/kg BW/2 weeks; Group 2: IVIG, 10% 200 mg/kg BW/2 weeks; and Group 3: Placebo (0.25% human albumin) at a dose of: 4 mL/kg BW/2 weeks, or 2 mL/kg BW/2 weeks.

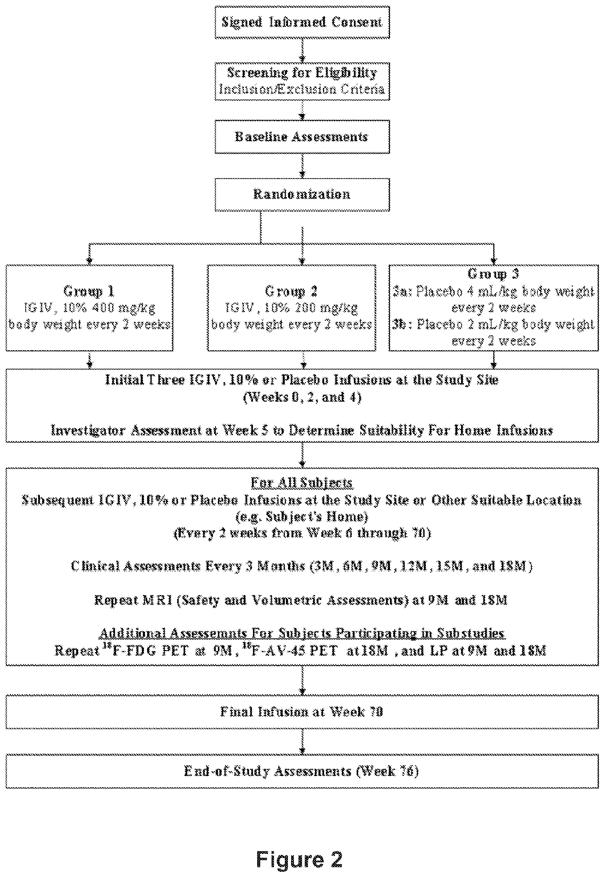

IV infusions of IVIG, 10% or placebo was administered every two weeks for 70 weeks (a total of 36 infusions), followed by a 6-week follow-up period without IVIG, 10%/placebo treatment. Two dose levels of IVIG, 10% (400 mg/kg BW/2 weeks and 200 mg/kg BW/2 weeks) were be studied. To maintain blind, half of the placebo subjects received a high infusion volume (4 mL/kg BW/2 weeks) and the other half a low infusion volume (2 mL/kg BW/2 weeks) to match the 400 mg/kg and 200 mg/kg IVIG, 10% doses, respectively. A schematic diagram depicting the study flow is provided in Figure 2.

The study was powered to compare the mean changes from baseline in ADAS-Cog and ADCS-ADL at 18 months between the 0.4 g/kg BW IVIG, 10% group and the placebo group using an analysis of covariance (ANCOVA) model accounting for the treatment effect and baseline value as a covariate. The following assumptions were made for the sample size calculation: the standard deviation (SD) of the change score at 18 months is 8 for ADAS-Cog and 11 for ADCSADL, the correlation of change score with baseline is 0.75 for ADAS-Cog and 0.79 for ADCS-ADL.

86 evaluable subjects per arm provide 80% power to detect a mean difference of 3.24 points in ADAS-Cog and a mean difference of 4.52 points in ADCS-ADL between the 0.4 g/kg BW IVIG, 10% group and the placebo groups, at a 5% significance level. Considering a 33% attrition rate, approximately 385 subjects will be randomized to one of the three groups (two treatment groups and one placebo group) with 1:1:1 randomization ratio.

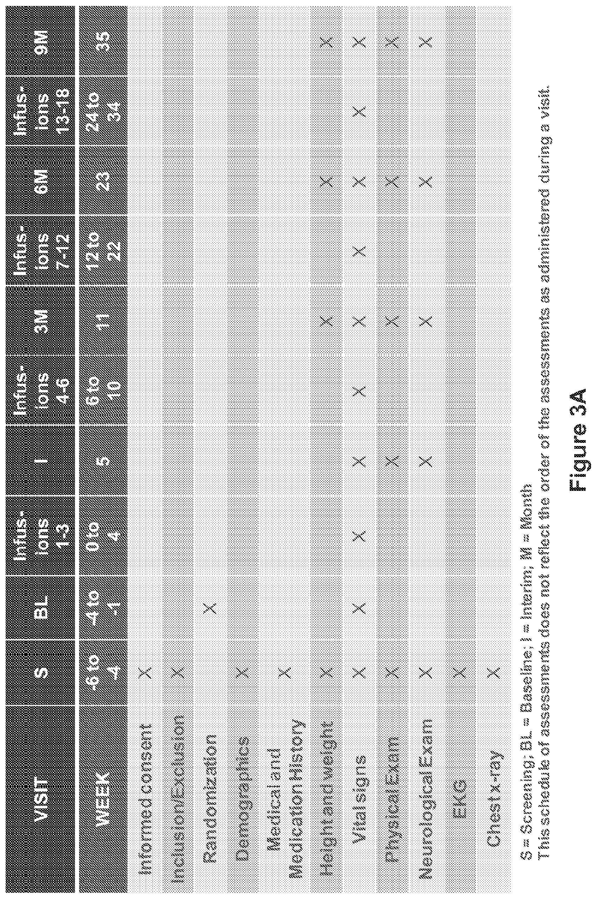

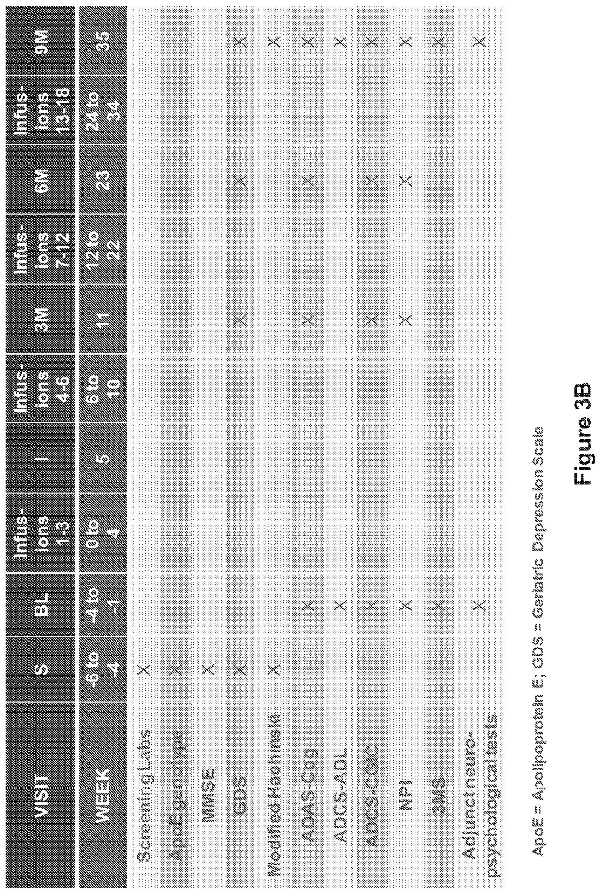

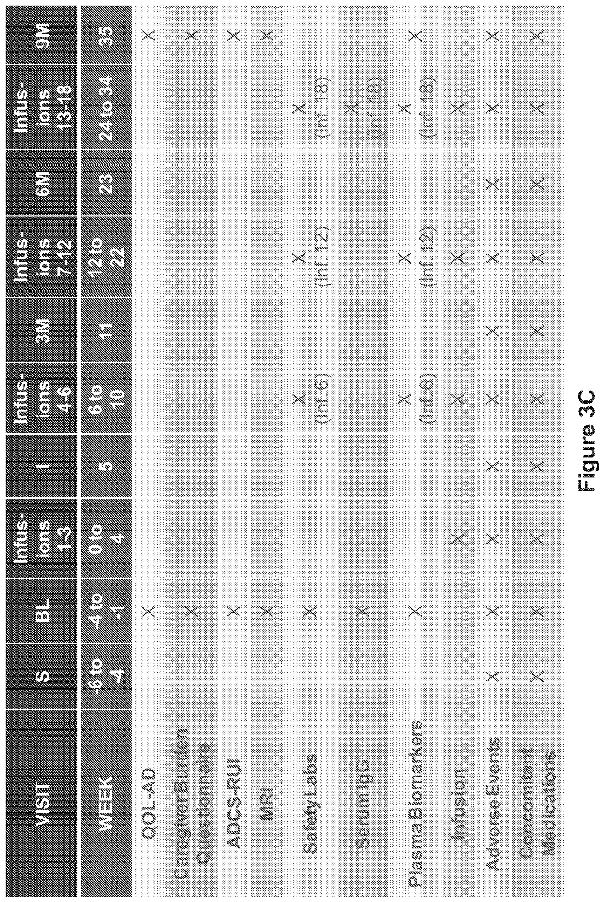

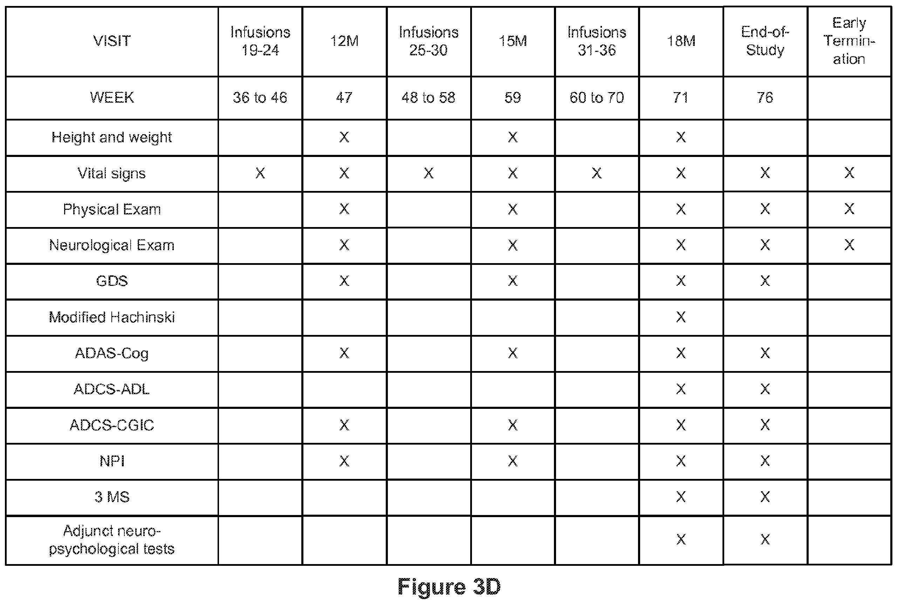

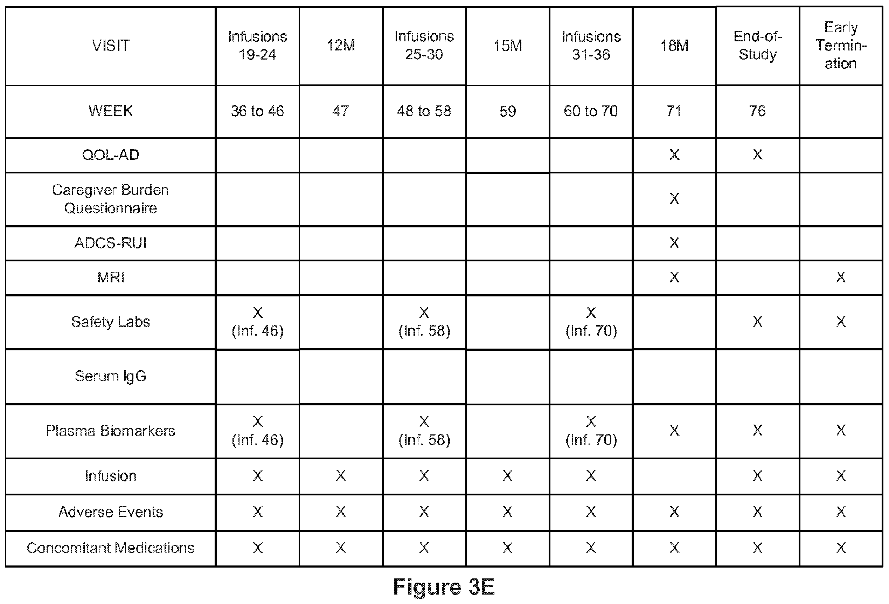

Subjects were monitored during the course of the trial by periodic assessment of various cognitive assessments, clinical, behavioral, and functional assessments, quality of life assessments, and a healthcare resource utilization assessment. A schedule of the assessments is provided as Figure 3A-3E.

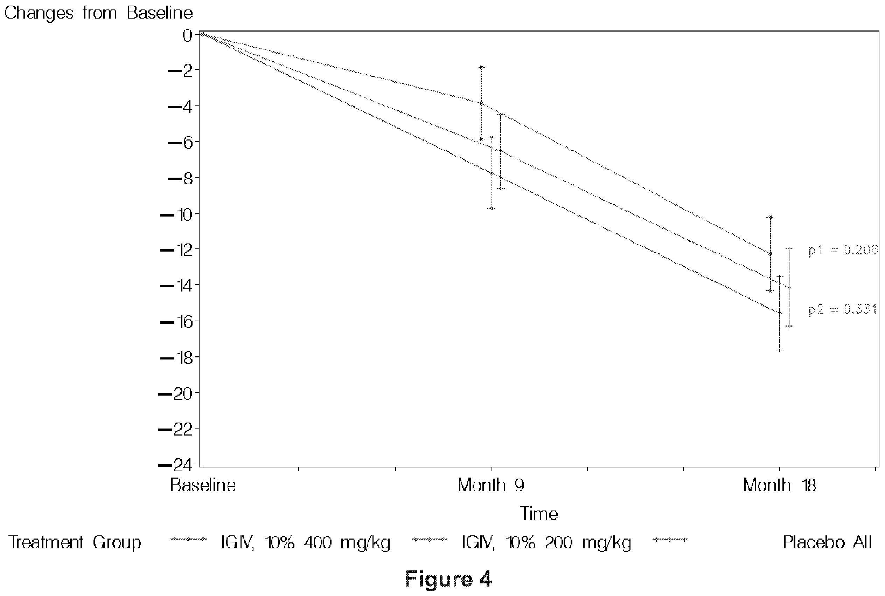

Overall, treatment of Alzheimer's patients by administration of 400 mg/kg/2 week IVIG resulted in reduced progression of dementia, measured by modified mini-mental state examination (3MS) analysis, as compared to administration of placebo (p1 = 0.206) and administration of 200 mg/kg/2 week IVIG (Figure 4).

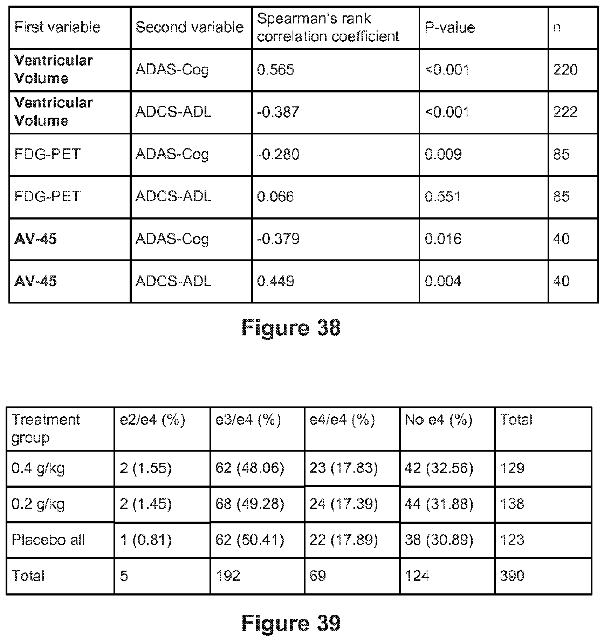

Patients were also classified into sub-populations of individuals having mild Alzheimer's disease (defined as MMSE 21-26), individuals having moderate Alzheimer's disease (defined as MMSE 16-20), individuals who were Apo4E carriers (e.g., having at least one Apo4E allele), and individuals who were Apo4E negative (e.g., having no ApoE alleles), for further sub-population analysis. ApoE4 genotype and allele distribution for this study is provided in Figure 39.

Even though the study was not powered to compare the mean changes from baseline in ADAS-Cog, ADCS-ADL, and 3MS at 18 months between subpopulations of the 0.4 g/kg BW IVIG, 10% group and subpopulations of the placebo group using an analysis of covariance (ANCOVA) model accounting for the treatment effect and baseline value as a covariate, several significant results were observed when analyzing the disease-state and ApoE4 status sub-population data.

Surprisingly, administration of 400 mg/kg/2 week IVIG to ApoE4 carriers (e.g., subjects having at least one Apo4E allele) resulted in a much greater reduction in the progression of dementia, as compared to subjects receiving either placebo (p = 0.012) or 200 mg/kg/2 week IVIG, than the entire Alzheimer's cohort (e.g., without separating by sub-population). This is illustrated in Figure 5, which shows the average modified mini-mental state examination (3MS) scores at months 9 and 18 of the trial. Treatment with 200 mg/kg/2 week IVIG did not reduce the progression of dementia in ApoE4 carriers.

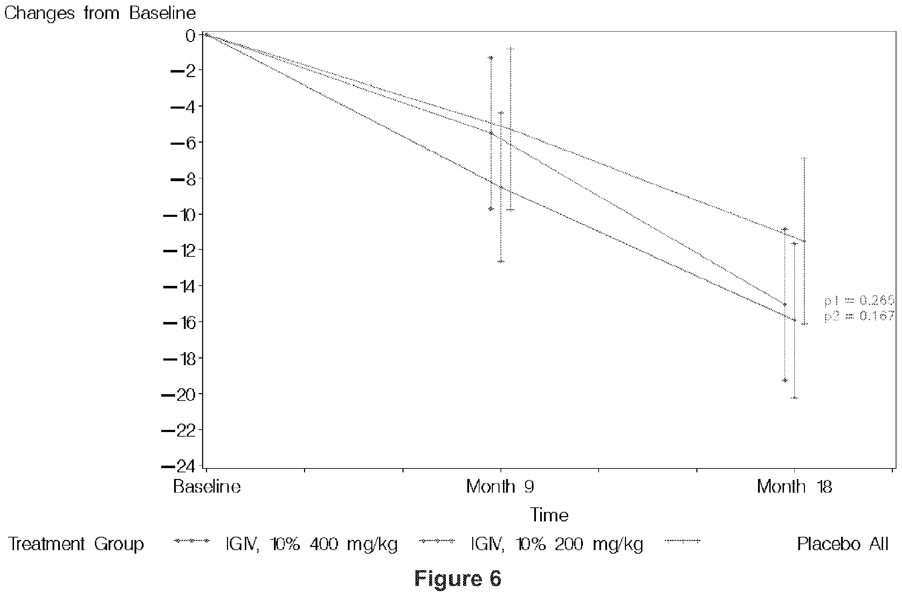

Conversely, administration of 200 mg/kg/2 week IVIG and 400 mg/kg/2 week IVIG to ApoE4 negative subjects (e.g., subjects not having an Apo4E allele) did not slow down the progression of dementia, measured by modified mini-mental state examination (3MS) analysis, as compared to administration of placebo (Figure 6).

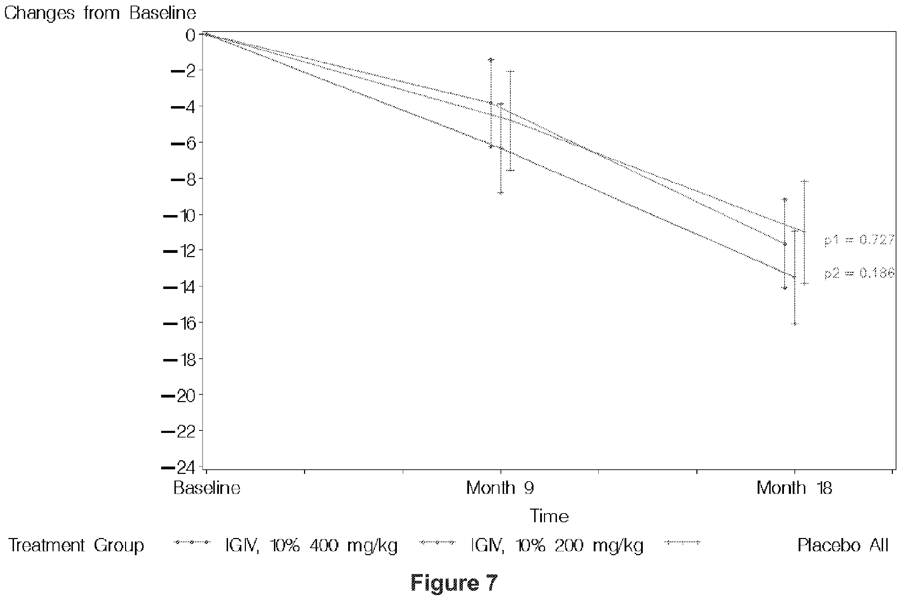

In addition, administration of 200 mg/kg/2 week IVIG and 400 mg/kg/2 week IVIG to subjects diagnosed with mild disease (e.g., subjects with an MME score of 21-26) did not slow down the progression of dementia, measured by modified mini-mental state examination (3MS) analysis, as compared to administration of placebo (Figure 7).

However, administration of 400 mg/kg/2 week IVIG to subjects diagnosed with moderate disease (e.g., subjects with an MME score of 16-20) did result in a greater reduction in the progression of dementia, as compared to subjects receiving either placebo (p = 0.067) or 200 mg/kg/2 week IVIG, than the entire Alzheimer's cohort (e.g., without separating by sub-population). This is illustrated in Figure 8, which shows the average modified mini-mental state examination (3MS) scores at months 9 and 18 of the trial (Figure 8A) and ADAS-Cog scores every three months during the trial (Figure 8C). Treatment with 200 mg/kg/2 week IVIG did not reduce the progression of dementia in subjects with moderate disease, as assessed by either 3MS or ADAS-Cog. Figure 8B shows that the positive effect of high dose IVIG treatment is also statistically significant among Apo4E carriers with moderate Alzheimer's disease (p = 0.011).

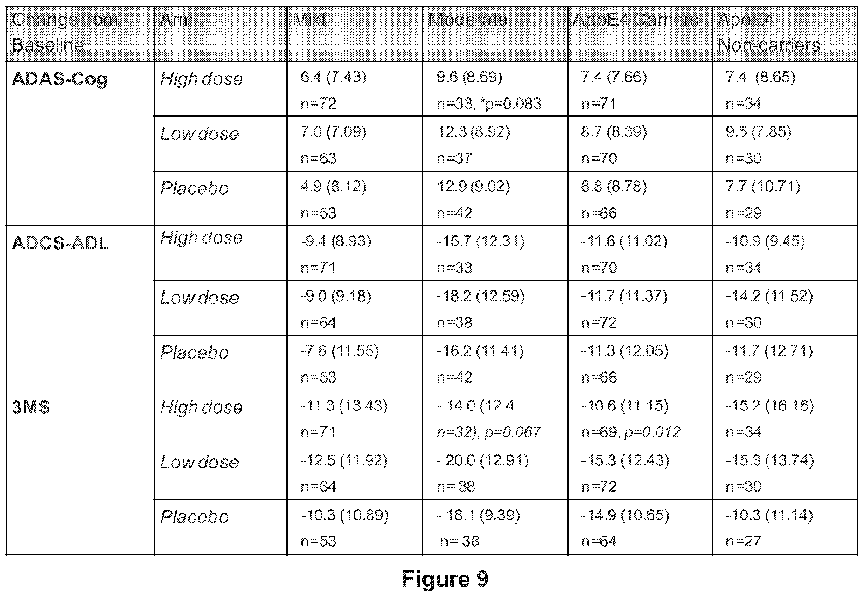

As shown in Figure 9, high dose IVIG treatment (e.g., 400 mg/kg/2 week IVIG) also slowed the progression of dementia in patients with moderate disease (e.g., subjects with an MME score of 16-20), as assessed by the Alzheimer's Disease Assessment Scale-Cognitive Subscale (ADAS-Cog).

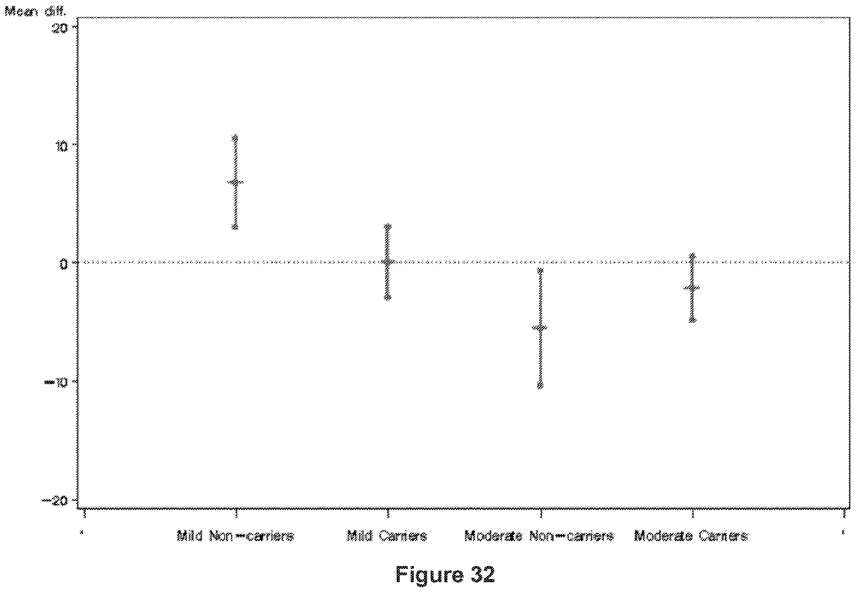

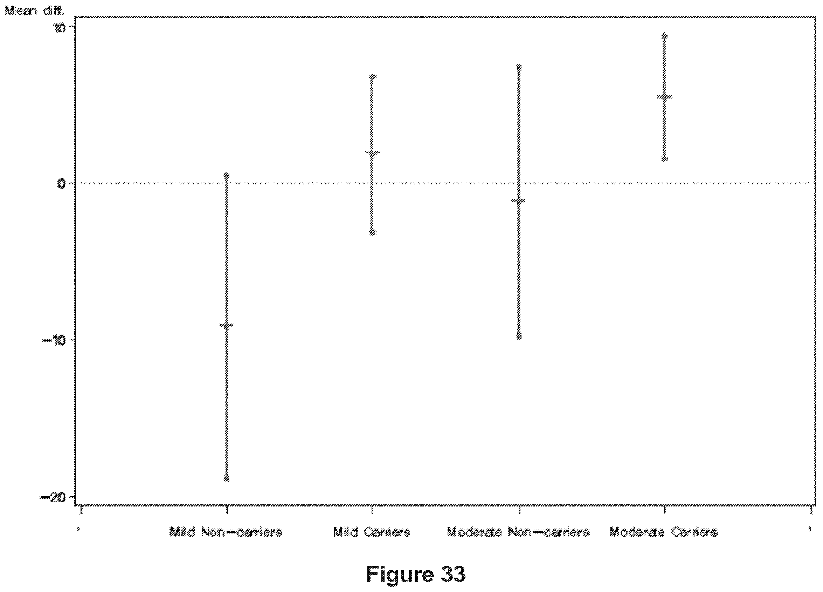

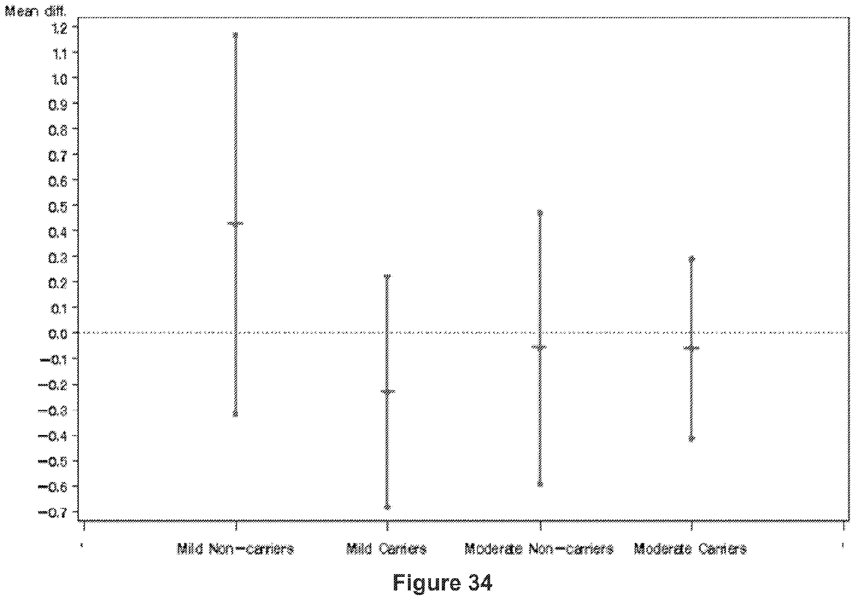

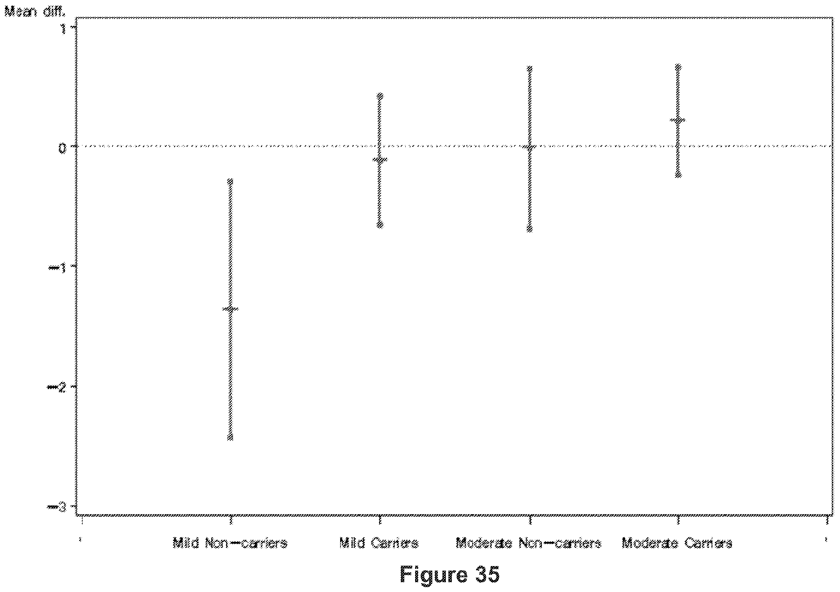

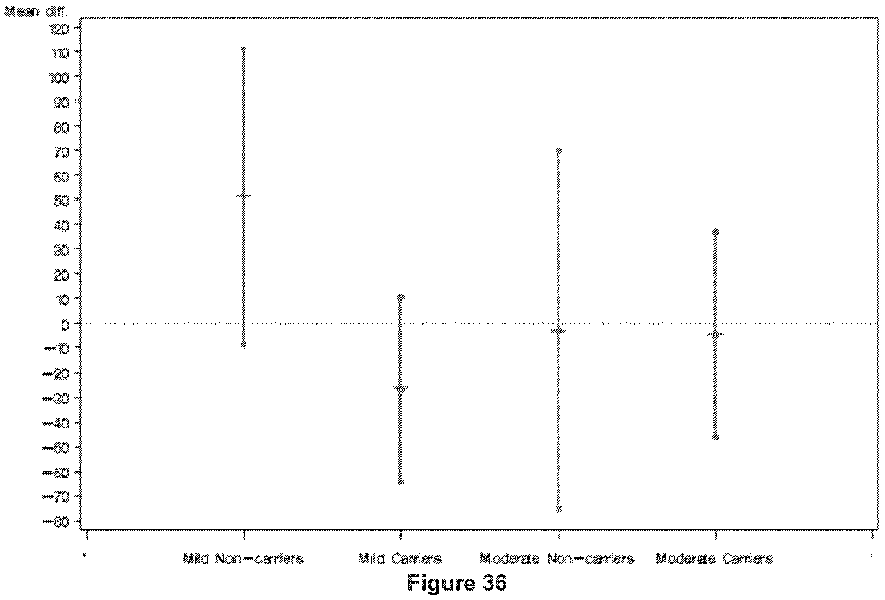

Graphical representations of the mean and 95% confidence intervals for differences between changes from baseline for subjects receiving high dose IVIG and placebo at 18 months for ADAS-Cog, 3MS, CGIC, clock drawing, and Trail B examinations are provided as Figures 32 to 36, respectively.

The Mini-Mental State Examination (MMSE) is a cognitive screening instrument that is validated and widely used in clinical practice and often employed as a measure of symptom severity in AD drug studies. The MMSE provides a 30-point composite rating for spatial and temporal orientation, verbal recall, simple attention, working memory, naming, repetition, comprehension, writing and constructional abilities. Scores range from 0 to 30 with lower values indicating more impairment. Subjects with MMSE scores of 16-26 inclusive were eligible for this study. The MMSE was performed at screening to confirm eligibility. The post-screening MMSE scores were derived from the 3MS examination performed at baseline, during the 9 M and 18 M visits, and at the end-of-study visit. The MMSE provides a metric familiar to many practicing physicians and was included as a safety measure. For review, *see,* Folstein MF, Folstein SE, McHugh PR. "Mini-mental state" A practical method for grading the cognitive state of patients for the clinician. J.Psychiatr.Res., 12:189-198 (1975).

The Modified Mini-Mental State Examination (3MS) test is a comprehensive validated cognitive examination tool that retains the brevity, the ease of administration, and the objective scoring of the MMSE, but provides a broader range and more refined scoring. Scores range from 0 to 100 with lower values indicating more impairment. The 3MS was performed at baseline, during the 9 M and 18 M visits, and at the end-of-study visit. The 3MS provides a metric familiar to many practicing physicians and will be included in secondary analyses. For review, *see,* Teng EL, Chui HC. The Modified Mini-Mental State (3MS) examination, J. Clin. Psychiatry, 48:314-318 (1987).

Cognitive Subscale of the Alzheimer's Disease Assessment Scale (ADAS-Cog) is validated and widely used as a primary cognitive outcome measure in AD pharmacotherapy studies. This is a psychometric instrument that evaluates memory (word recall, word recognition), attention, reasoning (following commands), language (naming, comprehension), orientation, ideational praxis (placing letter in envelope) and constructional praxis (copying geometric designs). Scoring is in the range of 0 to 70 with a higher score indicating greater impairment. This test was administered by experienced raters at each site at baseline, every 3 months during the 3 M, 6 M, 9 M, 12 M, 15 M, and 18 M visits, and at the end-of-study visit, or early termination visit. The ADASCog was the primary cognitive outcome measure for this study. For review, *see,* Rosen WG, Mohs RC, Davis KL. A new rating scale for Alzheimer's disease. Am. J. Psychiatry 141:1356-1364 (1984).

ADCS-Activities of Daily Living (ADCS-ADL) is a validated tool for assessing instrumental and basic activities of daily living based on a 23-item structured interview of the caregiver or qualified study partner. The scale has a range of 0 to 78, with lower scores indicating greater impairment. The ADCS-ADL was the primary measure of the subjects' functional status in this study and was assessed at baseline, during the 9 M and 18 M visits, and at the end-of-study visit. For review, *see,* Galasko D, Bennett D, Sano M et al. An inventory to assess activities of daily living for clinical trials in Alzheimer's disease, The Alzheimer's Disease Cooperative Study, Alzheimer Dis. Assoc. Disord., ;11 Suppl. 2:S33-S39 (1997).

### Example 3 - Analysis of IVIG Administration in Subjects with Moderate Alzheimer's Disease

The results of the IVIG treatment study presented in Example 2 were reevaluated using modified criteria for defining mild and moderate Alzheimer's disease. It was found that by increasing the power of the study (e.g., the number of individuals in the moderate disease cohort) by including additional patients with advanced Alzheimer's disease that were originally classified as having moderate disease, that high dose IVIG treatment of subject with moderate disease has a statistically significant effect.

The study presented in Example 2 defined subjects with moderate Alzheimer's disease as having an MMSE score of 20 or less (e.g., effectively MMSE = 16-20, inclusive because no individuals having an MMSE score below 16 were admitted to the study). Initial cognitive assessments of subjects having moderate disease, using the ADAS-Cog and 3MS cognitive examinations, suggested a positive trend in slowing the progression of the disease with administration of high dose IVIG (p = 0.083 and p = 0.067 for ADAS-Cog and 3MS examinations, respectively). However, as shown in Table 1, analysis of the data using redefined moderate disease cohorts, including subjects with MMSE scores of 21 and 22, shows that subjects with moderate Alzheimer's disease (e.g., MMSE of 14 to 22, inclusive) significantly benefit from high dose IVIG treatment. These data indicate that all individuals with moderate Alzheimer's disease, regardless of ApoE4 status, may benefit from high dose IVIG treatment.

**Table 1. Difference in the change in ADAS-Cog and 3MS examination score from baseline in subjects with moderate Alzheimer's disease treated with high dose IVIG (0.4 g/kg/2 week) as compared to placebo.**

| | **ADAS-Cog** | **3MS** |
|---|---|---|
| **MMSE ≤ 20** | -2.69 | 4.28 |
| | p = 0.083 (n = 75) | p = 0.067 (n = 70) |
| **MMSE ≤ 21** | -2.75 | 3.44 |
| | p = 0.046 (n = 97) | p = 0.09 (n = 92) |
| **MMSE ≤ 22** | -3.40 | 4.20 |
| | p = 0.006 (n= 112) | p = 0.029 (n = 116) |

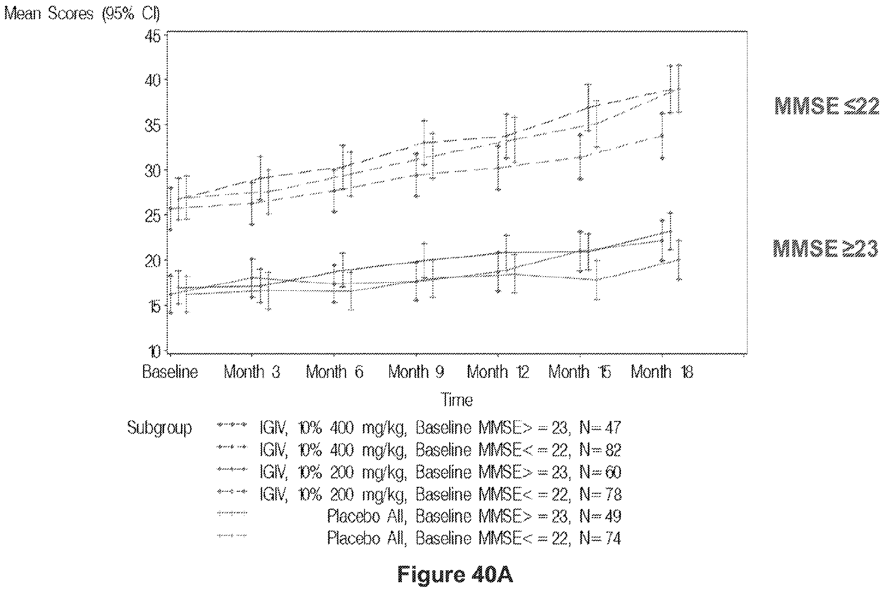

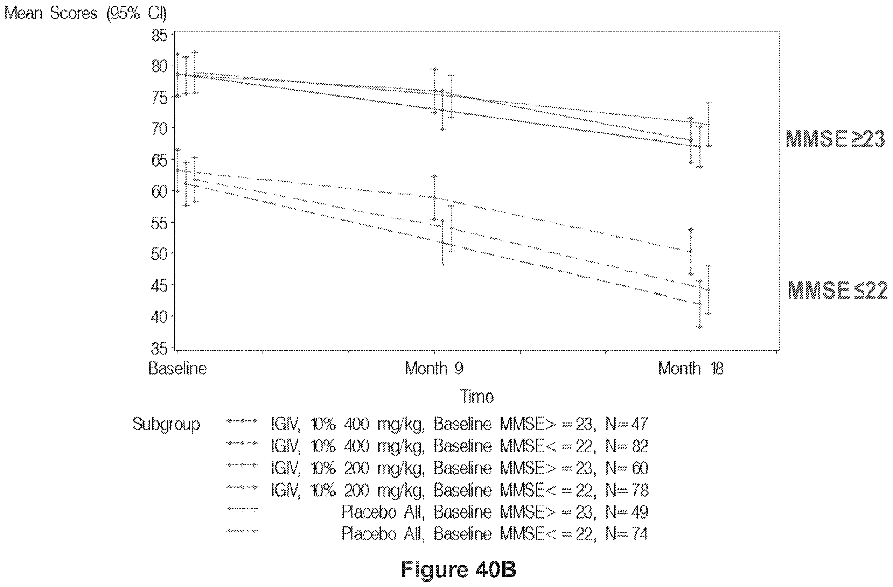

This positive result is illustrated in Figure 40, which reports the average ADAS-Cog (Figure 40A) and 3MS (Figure 40B) scores taken every three months during the trial for patients diagnosed with moderate (MMSE ≤ 22) and mild Alzheimer's disease (MMSE ≥ 23). As shown in Figure 40, treatment of moderate disease patients with 400 mg/kg/2 week IVIG reduced the progression of dementia in subjects with moderate, but not mild, Alzheimer's disease. Treatment with 200 mg/kg/2 week IVIG did not reduce the progression of dementia in subjects with mild or moderate disease, as assessed by either ADAS-Cog or 3MS.

As reported in **Table 2** and **Table 3,** further analysis of the data collected for the study reported in Example 1 show that treatment of subjects initially diagnosed with moderate Alzheimer's disease and/or carrying an ApoE4 allele with high dose (400 mg/kg/2 week), but not low dose (200 mg/kg/2 weeks), IVIG reduced the progression of dementia, as assessed by ADAS-Cog (p = 0.026 vs. placebo) or 3MS (p = 0.032 vs. placebo), respectively. Similarly, treatment of the same patient cohort with high dose (400 mg/kg/2 week), but not low dose (200 mg/kg/2 weeks), IVIG for 18 months slowed the reduction in FAS verbal fluency score from baseline (p = 0.031 vs. placebo; **Table 4**) and trail making test part B score from baseline (p = 0.079; **Table 5**).

**Table 2. Difference in the change in ADAS-Cog examination score from baseline, excluding subjects with MMSE > 22 who are ApoE4 negative, treated with high dose IVIG (0.4 g/kg/2 week), low dose IVIG (0.2 g/kg/2 week), and placebo.**

| **Visit** | **0.4 g/kg** | | | **0.2 g/kg** | | | **Placebo** | | |
|---|---|---|---|---|---|---|---|---|---|
| | **N** | **Mean** | **S.D.** | **N** | **Mean** | **S.D.** | **N** | **Mean** | **S.D.** |
| **Month 18** | **94** | **7.3** | **8.08** | **87** | **9.2** | **8.44** | **83** | **9.5** | **9.30** |
| | | **p=0.026 vs. placebo** | | | | | | | |

**Table 3. Difference in the change in 3MS examination score from baseline, excluding subjects with MMSE > 22 who are ApoE4 negative, treated with high dose IVIG (0.4 g/kg/2 week), low dose IVIG (0.2 g/kg/2 week), and placebo.**

| | **0.4 g/kg** | | | **0.2 g/kg** | | | **Placebo** | | |
|---|---|---|---|---|---|---|---|---|---|
| **Visit** | **N** | **Mean** | **S.D.** | **N** | **Mean** | **S.D.** | **N** | **Mean** | **S.D.** |
| **Month 18** | **92** | **-11.6** | **12.49** | **89** | **-15.5** | **12.87** | **79** | **-14.8** | **10.56** |
| | | **p=0.032 vs. placebo** | | | | | | | |

**Table 4. Difference in the change in FAS verbal fluency score from baseline, excluding subjects with MMSE > 22 who are ApoE4 negative, treated with high dose IVIG (0.4 g/kg/2 week), low dose IVIG (0.2 g/kg/2 week), and placebo.**

| **Visit** | **0.4 g/kg** | | | **0.2 g/kg** | | | **Placebo** | | |
|---|---|---|---|---|---|---|---|---|---|
| | **N** | **Mean** | **S.D.** | **N** | **Mean** | **S.D.** | **N** | **Mean** | **S.D.** |
| **Month 18** | **91** | **-4.3** | **8.57** | **84** | **-7.8** | **9.05** | **77** | **-6.5** | **8.38** |
| | | **p=0.031 vs. placebo** | | | | | | | |

**Table 5. Difference in the change in trail making test part B (Trail B) score from baseline, excluding subjects with MMSE > 22 who are ApoE4 negative, treated with high dose IVIG (0.4 g/kg/2 week), low dose IVIG (0.2 g/kg/2 week), and placebo.**

| **Visit** | **0.4 g/kg** | | | **0.2 g/kg** | | | **Placebo** | | |
|---|---|---|---|---|---|---|---|---|---|
| | **N** | **Mean** | **S.D.** | **N** | **Mean** | **S.D.** | **N** | **Mean** | **S.D.** |
| **Month 18** | **57** | **9.5** | **59.51** | **47** | **31.8** | **62.10** | **42** | **35.1** | **68.95** |
| | | **p=0.079 vs. placebo** | | | | | | | |

Overall, this study shows that subjects having moderately severe Alzheimer's disease and subject that are ApoE4 carriers can benefit from IVIG therapy. ApoE4 carriers diagnosed with moderately sever Alzheimer's disease appear to benefit the most from IVIG therapy, as measured by 3MS and ADAS-Cog examinations. This is surprising given that previous studies have suggested that the presence of an ApoE4 allele limits therapeutic efficacy and safety. For example, the ApoE4 allele has been strongly associated with the incidence of vasogenic edema, which was not observed in this study. These results suggest that IVIG therapy relies on a different mechanism of action than does monoclonal antibody therapy.

### Example 4 - Analysis of Biomarkers in Alzheimer's Subjects Administered IVIG or Placebo

To further evaluate the efficacy of intravenous immunoglobulin G (IVIG) administration for the treatment and/or management of Alzheimer's disease, biomarker levels from subjects participating in the study described in Example 2 were investigated. The results of these analyses further strengthen the conclusion that administration of high doses of IVIG (e.g., 0.3-0.8 g/kg/2 weeks) is beneficial for subjects with moderate disease, and especially for carriers of the ApoE4 gene.

Biomarkers that were investigated in the study included: Aβ40 and Aβ42 levels in plasma and cerebrospinal fluid (CSF) at 9 and 18 months; anti-Aβ40 and anti-Aβ42 antibody titers in plasma and cerebrospinal fluid (CSF) at 9 and 18 months (including anti-monomer, anti-oligomer, and anti-fibril antibodies); total and phosphorylated tau protein levels in CSF at 9 and 18 months; volumetric MRI, including ventricular enlargement, total ventricular volume, as well as whole brain and hippocampal atrophy at 9 and 18 months; Cerebral glucose metabolism using [¹⁸F]-2-fluorodeoxyglucose (18F-FDG) positron emission tomography (PET) imaging at 9 months; and cerebral amyloid deposition using [18F]-florpiramine (18F-AV-45) PET imaging at 18 months. The CSF, FDG-PET, and AV-45 PET outcomes were measured only in subgroup of subjects (target of 40 subjects in each treatment group for the CSF and the FDG-PET sub-studies, and target of 33 subjects in each treatment group for the AV-45 PET sub-study).

### Cerebral Glucose Metabolism ¹⁸F-FDG Positron Emission Tomography

At 6 months, it was found that Alzheimer's subjects treated with high dose IVIG (0.4 kg/2 week) showed improvement in cerebral glucose metabolism in both hemispheres of the brain. Typically, cerebral glucose metabolism declines by 10% to 20% annually in untreated Alzheimer's patients.

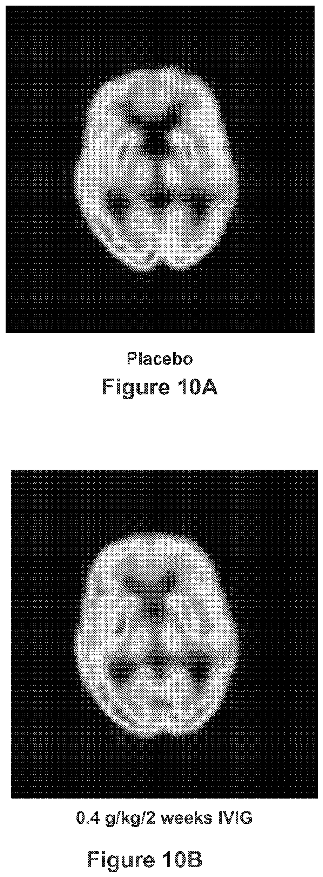

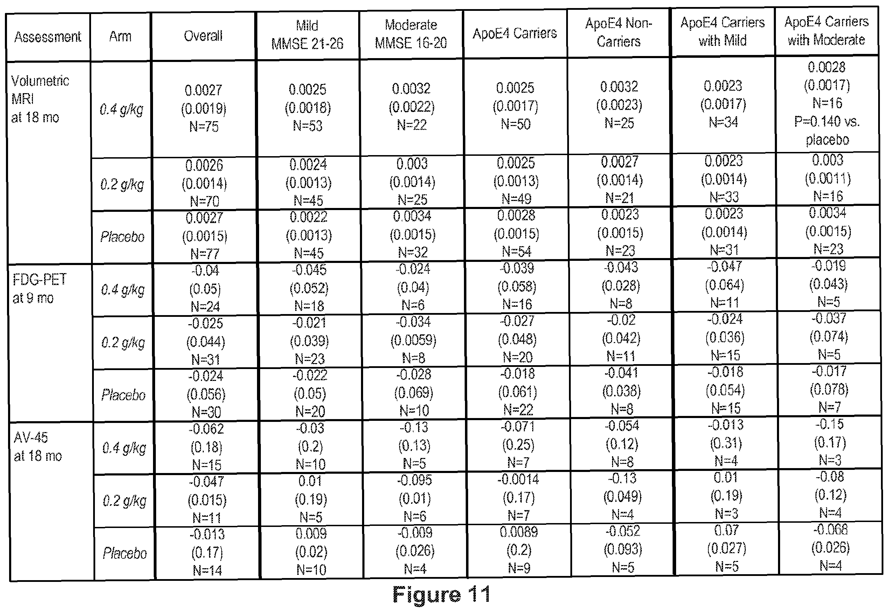

A typical temporoparietal and prefrontal pattern of glucose hypometabolism, imaged according to standard [18F]-2-fluorodeoxyglucose (18F-FDG) positron emission tomography (PET) imaging, for subjects treated with placebo is shown in Figure 10A. In contrast, subjects administered high dose IVIG (0.4 g/kg/2 weeks) show improvement in both hemispheres. A typical temporoparietal and prefrontal pattern of glucose hypometabolism, imaged as above, for subjects administered high dose IVIG is shown in Figure 10B. A summary of glucose metabolism in all cohorts and patient sub-populations is shown in Figure 11.

### Volumetric MRI

Neuronal loss in normal aging causes brain atrophy. This is exasperated in Alzheimer's patients, where neuronal degeneration in Alzheimer's disease (AD) causes accelerated brain atrophy. Because the skull is a closed space, brain atrophy causes progressive enlargement of the fluid-filled cerebral ventricles. Thus, the rate of ventricular enlargement over time provides an objective measure of the rate of progression of Alzheimer's disease.

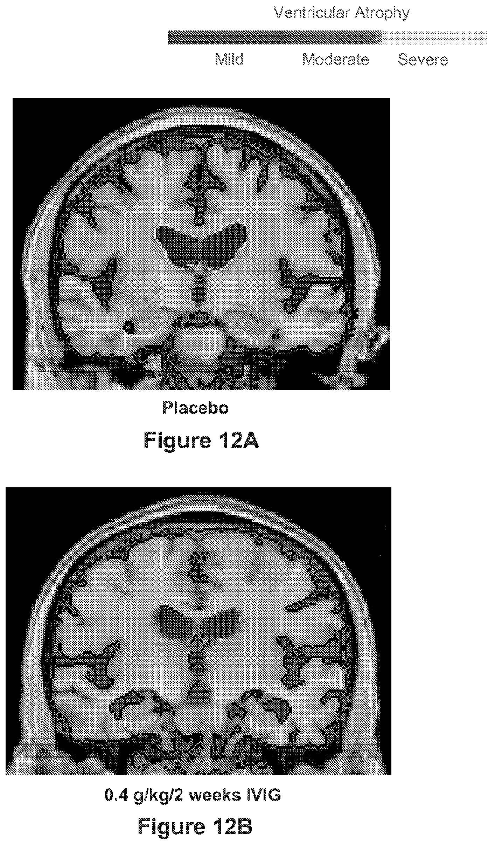

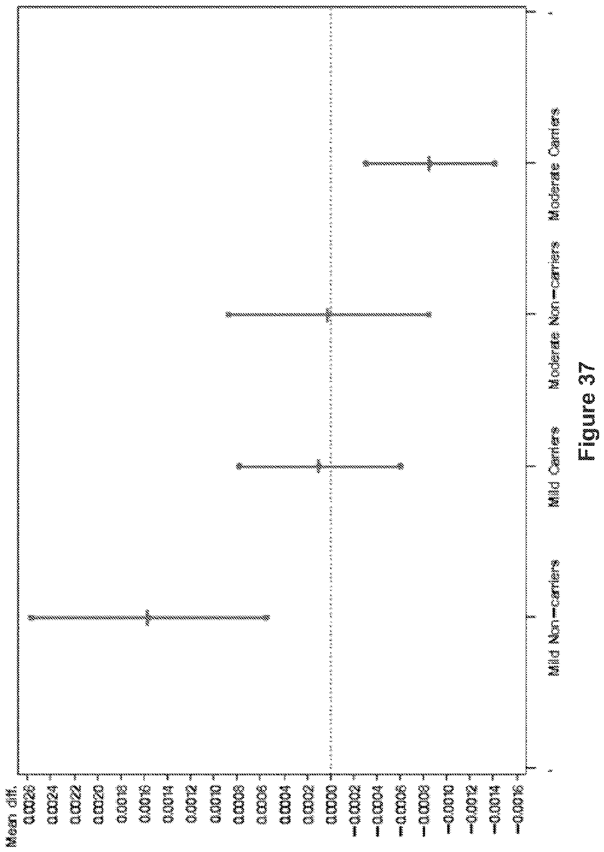

Ventricular volume was determined by volumetric MRI for a subset of subjects enrolled in the study described in Example 2. At 18 months, a positive trend was found for ApoE4 carrier subjects with moderate Alzheimer's disease receiving high dose IVIG (0.4 g/kg/2 weeks) (p = 0.140), as shown in Table 2, below. Images showing typical ventricular atrophy in the brains of Alzheimer's subjects from the placebo and high dose IVIG treatment groups are shown in Figures 12A and 12B, respectively. A summary of mean change from baseline of volumetric MRI (normalized by baseline intracranial volume) in all cohorts and patient sub-populations is shown in Figure 11. A graphical representation of the mean and 95% confidence intervals for differences between changes from baseline for subjects receiving high dose IVIG and placebo at 18 months, as measured by volumetric MRI, is provided in Figure 37.

**Table 6. Changes in ventricular volume from baseline at 18 months in ApoE4 carrier subjects with moderate Alzheimer's disease.**

| **Treatment Group** | **Estimate** | **S.E.** | **P-value** |
|---|---|---|---|
| **0.4 g/kg/2 weeks IVIG** | -0.00068 | 0.00045 | 0.140 |
| **0.2 g/kg/2 weeks IVIG** | -0.00027 | 0.00046 | 0.563 |

### Cerebral Amyloid Deposition Using [¹⁸F]-Florpiramine (¹⁸F-AV-45) PET Imaging

Florbetapir is a PET scanning radiopharmaceutical compound containing the radionuclide fluorine-18, recently FDA approved as a diagnostic tool for Alzheimer's disease, which binds to amyloid aggregates in the brain. Florbetapir binding was analyzed in a subset of subjects enrolled in the study described in Example 2. As shown in Table 3 below, PET scans showed decreases in florbetapir binding in all subjects receiving high dose IVIG therapy (0.4 g/kg/2 weeks), which were even more pronounced in ApoE4 carriers receiving high dose IVIG. These decreases are indicative of decreases in amyloid burden in the brains of these subjects. A summary of mean change from baseline of florbetapir binding in all cohorts and patient sub-populations receiving 0.4 g/kg/2 weeks IVIG is shown in Figure 11.

**Table 7. Changes in florpiramine imaging from baseline at 18 months in ApoE4 carrier subjects with moderate Alzheimer's disease.**

| **Treatment Group** | **N** | **Mean** | **95% CI** |
|---|---|---|---|
| **0.4 g/kg/2 weeks IVIG** | 15 | -0.062 | -0.163 to 0.039 |
| **0.4 g/kg/2 weeks IVIG ApoE4 Carriers** | 7 | -0.071 | -0.160 to 0.157 |
| **0.2 g/kg/2 weeks IVIG** | 11 | -0.047 | -0.148 to 0.053 |
| **Placebo** | 14 | -0.013 | -0.110 to 0.085 |
| **Placebo ApoE4 Carriers** | 9 | 0.009 | -0.146 to 0.164 |

### Aβ40 Protein and anti-Aβ40 Antibody levels in Plasma

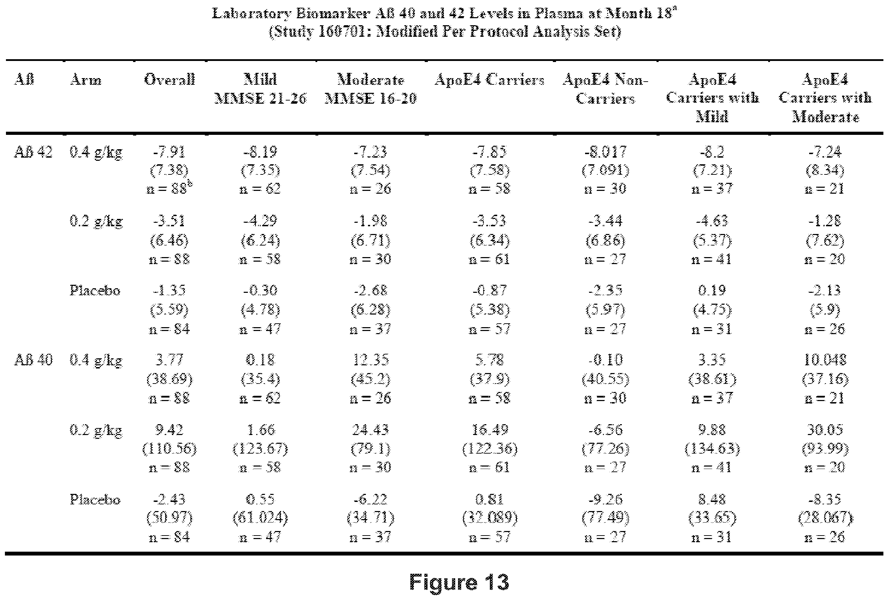

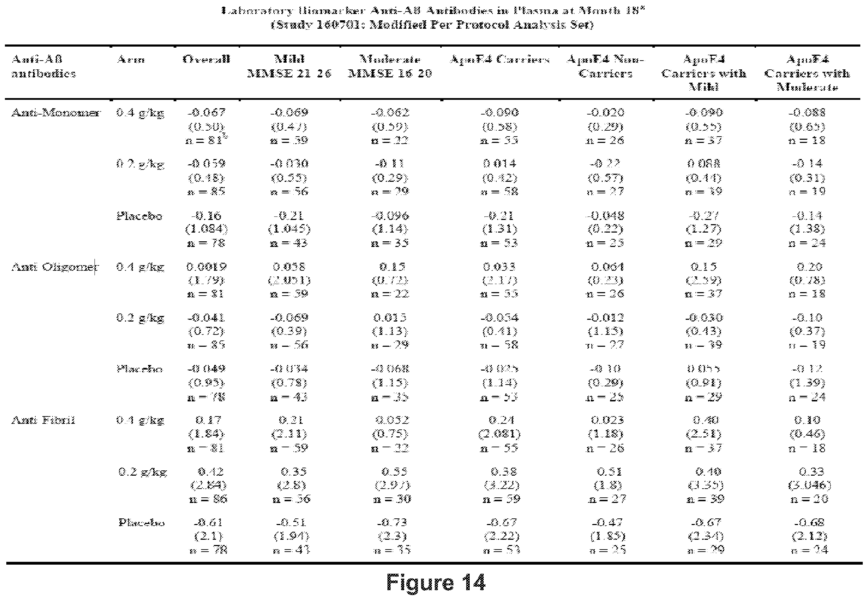

Aβ40 peptide and anti-Aβ40 antibody plasma levels were determined for subjects enrolled in the study described in Example 2. Overall, there was no significant change in the Aβ40 peptide or anti-Aβ40 antibody plasma levels in any of the treatment cohorts. Summaries of mean change from baseline of Aβ40 peptide and anti-Aβ40 antibody plasma levels for all cohorts and patient sub-populations are found in Figures 13 and 14, respectively.

### Aβ42 Protein and anti-Aβ42 Antibody levels in Plasma

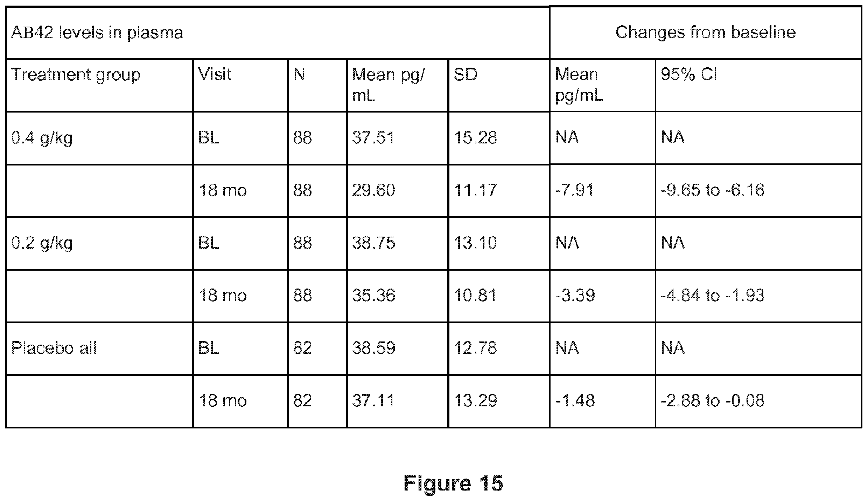

Aβ42 peptide and anti-Aβ42 antibody plasma levels were determined for subjects enrolled in the study described in Example 2. Overall, there was no significant change in the anti-Aβ42 antibody plasma levels in any of the treatment cohorts. However, as shown in Figure 15, plasma levels of Aβ42 peptide decreased in patient cohorts receiving 0.2 g/kg/2 weeks IVIG (mean 9% decrease) and 0.4 g/kg/2 weeks IVIG (mean 21% decrease). Summaries of mean change from baseline of Aβ40 peptide and anti-Aβ40 antibody plasma levels for all cohorts and patient sub-populations are found in Figures 13 and 14, respectively.

Taken together, the above data demonstrates that IVIG treatment significantly reduces plasma levels of Aβ42 peptide in a dose-dependent manner. The opposite effect is seen for plasma levels of Aβ40 peptides, which are increased in patient cohorts treated with IVIG.

### Aβ40 Peptide levels in Cerebrospinal fluid (CSF)

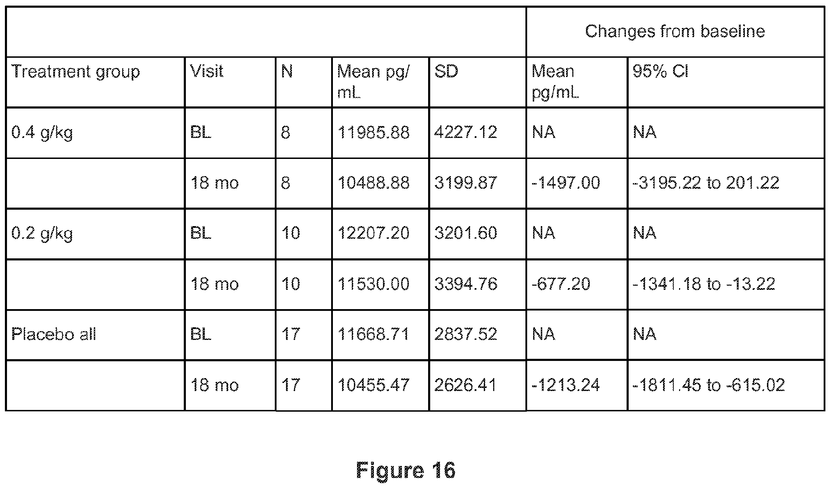

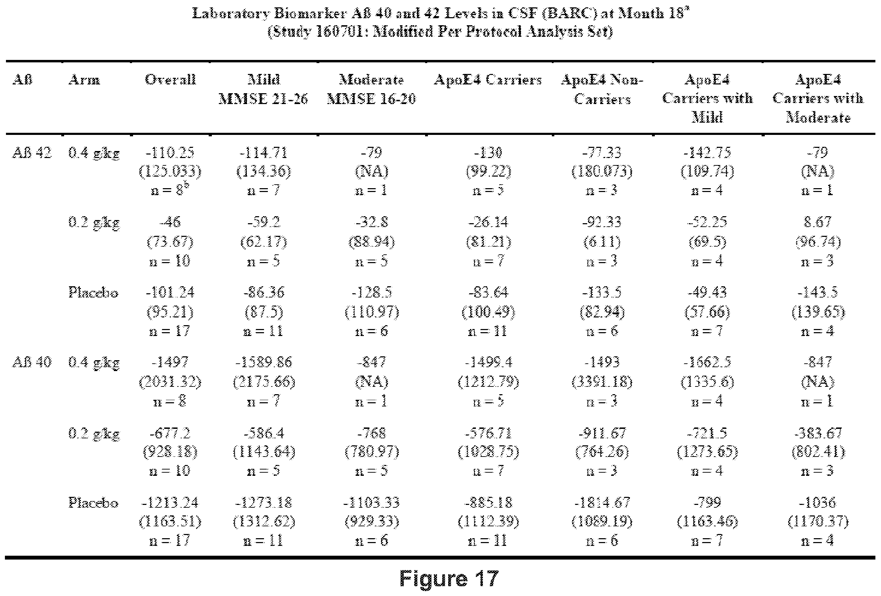

Aβ40 peptide levels in the CSF of subjects enrolled in the study described in Example 2 were determined. Overall, a small decrease in CSF Aβ40 peptide levels was seen for all cohorts, as seen in the data presented in Figure 16. A summary of mean change from baseline of Aβ40 peptide CSF levels for all cohorts and patient sub-populations is found in Figure 17.

### Aβ42 Peptide levels in Cerebrospinal fluid (CSF)

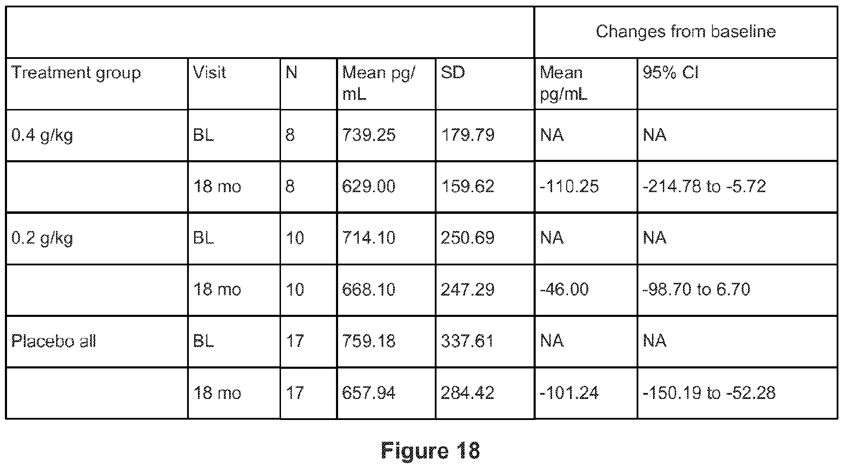

Aβ42 peptide levels in the CSF of subjects enrolled in the study described in Example 2 were determined. Overall, a modest decrease in CSF Aβ42 peptide levels was seen for all cohorts, as seen in the data presented in Figure 18. A summary of mean change from baseline of Aβ42 peptide CSF levels for all cohorts and patient sub-populations is found in Figures 17.

### IgG levels in Cerebrospinal fluid (CSF)

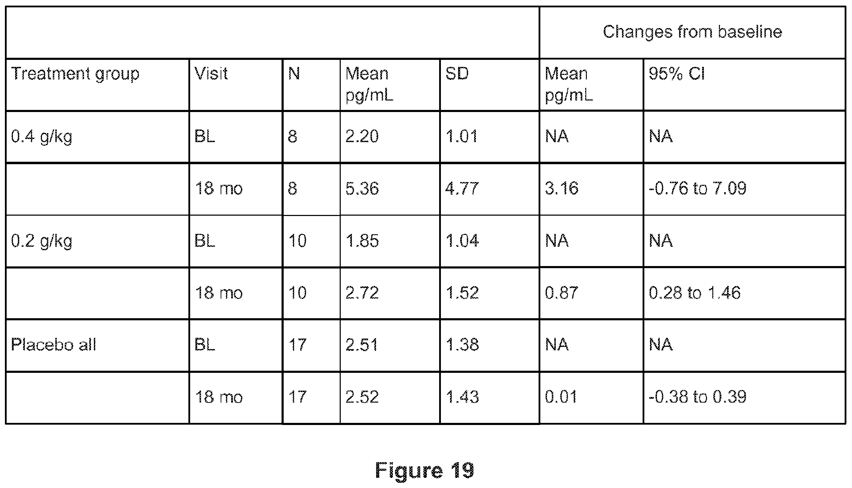

IgG levels in the CSF of subjects enrolled in the study described in Example 2 were determined. Overall, a modest increase in CSF IgG levels in subjects receiving low dose IVIG and a larger increase in CSF IgG levels in subjects receiving high dose IVIG was observed, as seen in Figure 19. Little to no increase in CSF IgG was observed in subjects receiving placebo. These data are consistent with the passage of IgG through the blood brain barrier. Interestingly, prior to the initiation of IVIG treatment, baseline CSF IgG levels were significantly lower (p = 0.038 (t-test); **Table 8**) in ApoE4 carriers (2.2 mg/mL) than in ApoE4 negative subjects (2.7 mg/mL).

**Table 8. Baseline levels of IgG in the cerebrospinal fluid (CSF) of ApoE4 positive and ApoE4 negative Alzheimer's patients.**

| | **N** | **Mean (mg/mL)** | **95% CI** |
|---|---|---|---|
| **ApoE4 Carriers** | 50 | 2.2 | 1.9 to 2.5 |
| **ApoE4 Non-Carriers** | 27 | 2.7 | 2.3 to 3.2 |

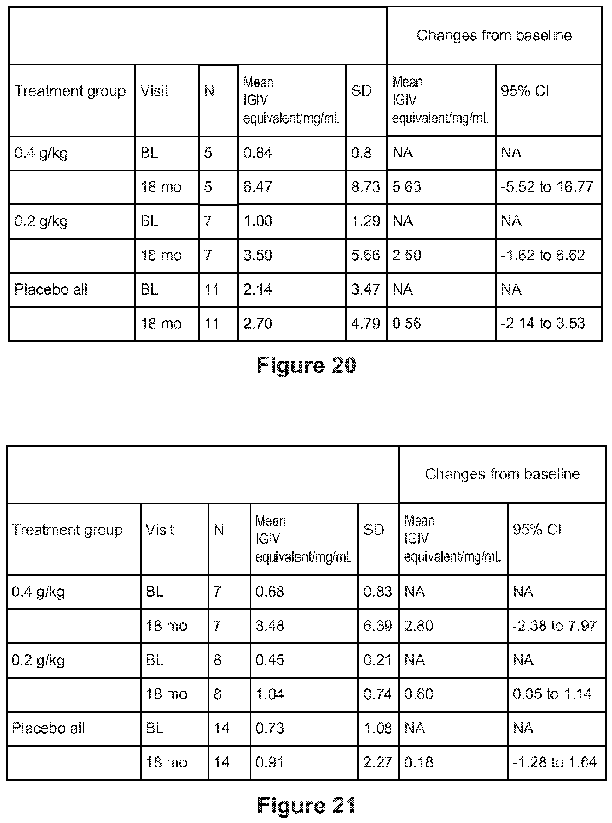

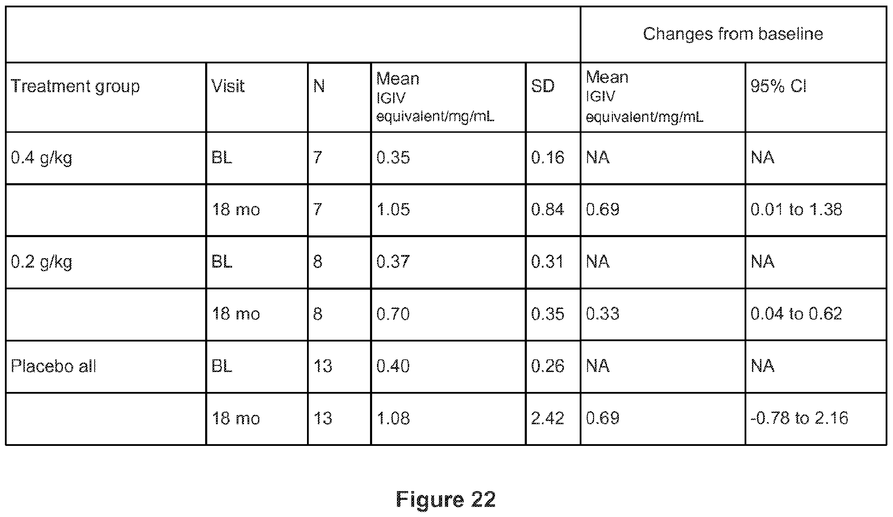

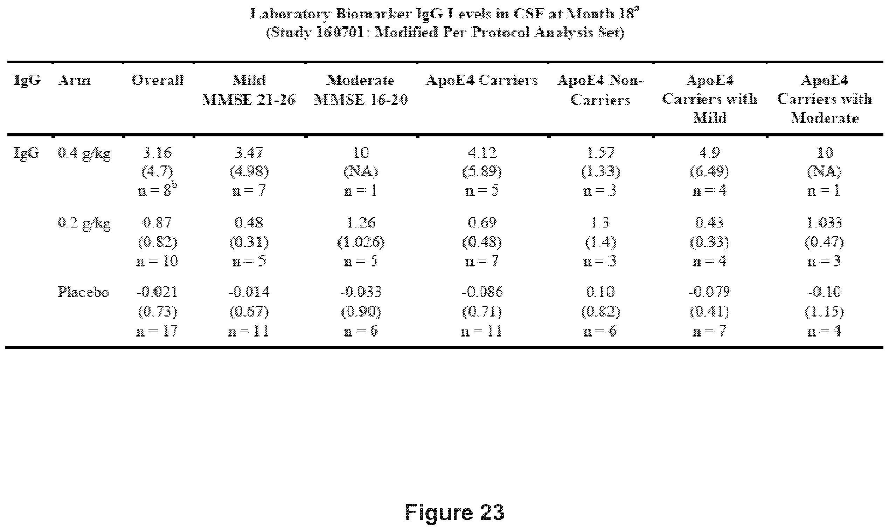

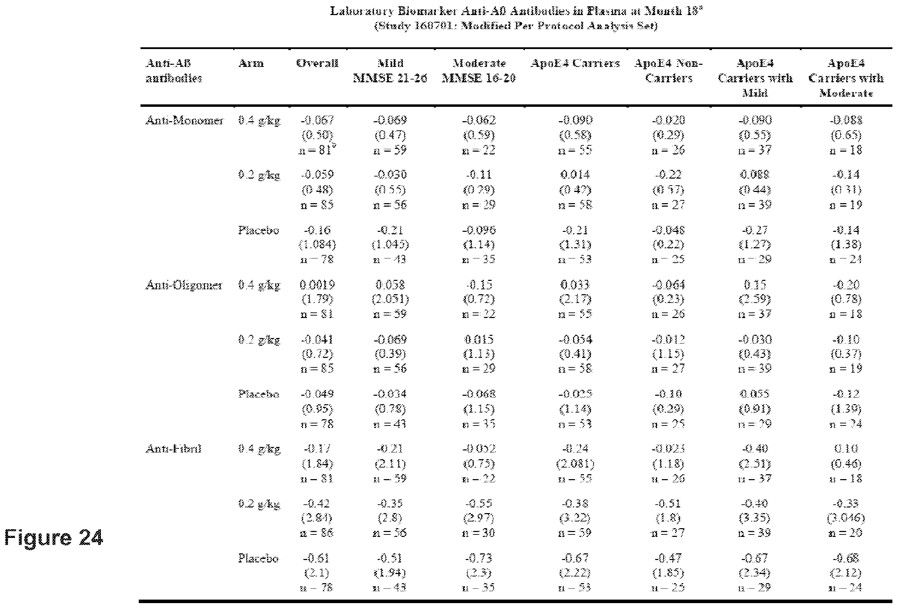

Likewise, moderate and large increases in anti-Aβ fibril and anti-Aβ oligomer antibodies were seen in the CSF of subjects receiving low and high dose IVIG, as shown in Figures 20 and 21, respectively. Similar increases in anti-Aβ monomer CSF levels were seen in IVIG treatment groups, however, an increase in anti-Aβ monomer CSF levels were also seen in subjects receiving placebo, as shown in Figure 22. Summaries of mean change from baseline of total IgG and anti-Aβ antibody subtypes for all cohorts and patient sub-populations are found Figures 23 and 24, respectfully.

Taken together, these data show that IgG passes through the blood-brain barrier after intravenous administration. Dose-dependent increases in total IgG, anti-Aβ oligomer antibodies, and anti-Aβ fibril antibodies were observed in the CSF of patients in the IVIG treatment cohorts.

### Tau protein levels in Cerebrospinal fluid (CSF)

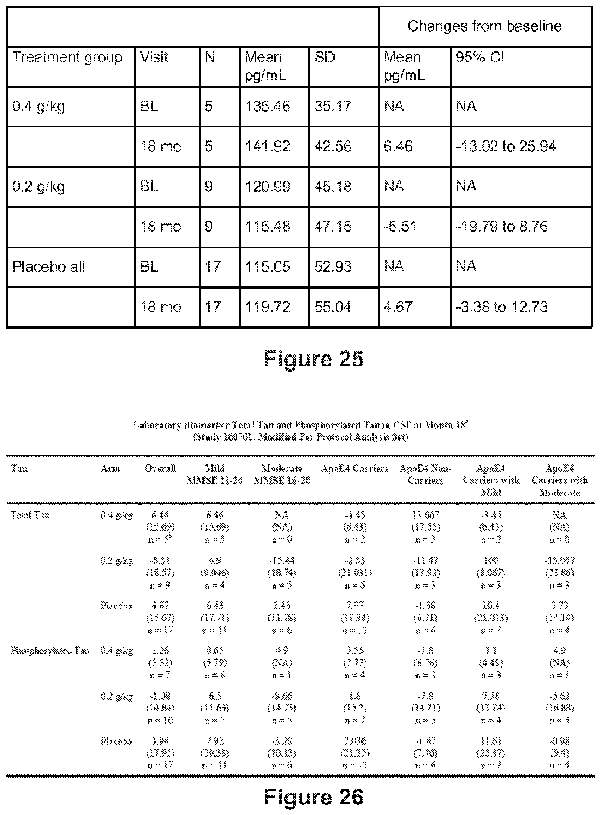

Tau is an axon protein that promotes assembly and stability of microtubules and vesicle transport. In the CSF, tau is considered a downstream biomarker used for monitoring effects on downstream pathogenic processes, such as neuronal degeneration or intra-neuronal tangle formation downstream of the anticipated primary effect of anti-Aβ intervention. Tau levels in the CSF of subjects enrolled in the study described in Example 2 were determined. Overall, there was no significant change in the level of CSF tau in any of the treatment cohorts, as shown in Figure 25. A summary of mean change from baseline for tau CSF levels for all cohorts and patient sub-populations is found in Figure 26.

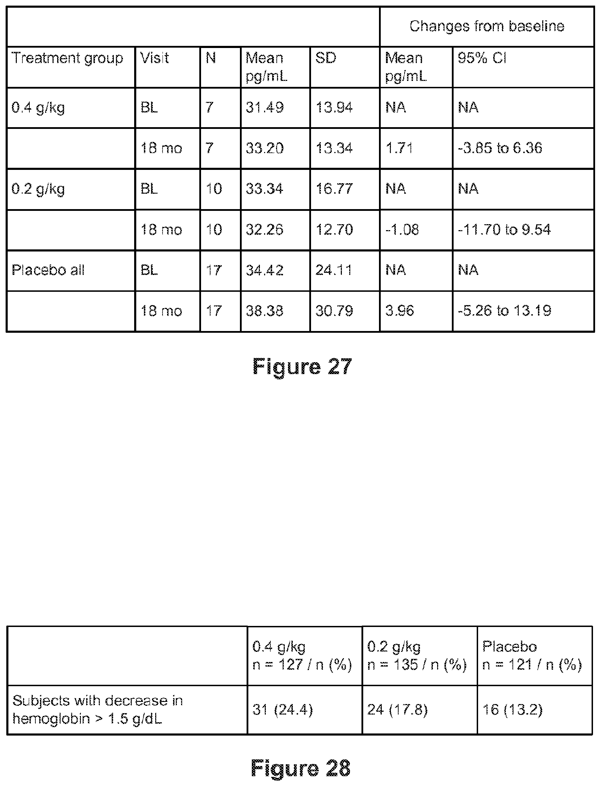

Phosphorylated tau is insoluble, lacks affinity for microtubules, and self-associates into paired helical filament structures. Increased levels of phosphorylated tau in the CSF correlate with AD cognitive impairment. Phosphorylated tau levels in the CSF of subjects enrolled in the study described in Example 2 were determined. Overall, there was no significant change in the level of CSF tau in any of the treatment cohorts, as shown in Figure 27. A summary of mean change from baseline for phosphorylated tau CSF levels for all cohorts and patient sub-populations is found in Figure 27.

### IgG levels in Serum

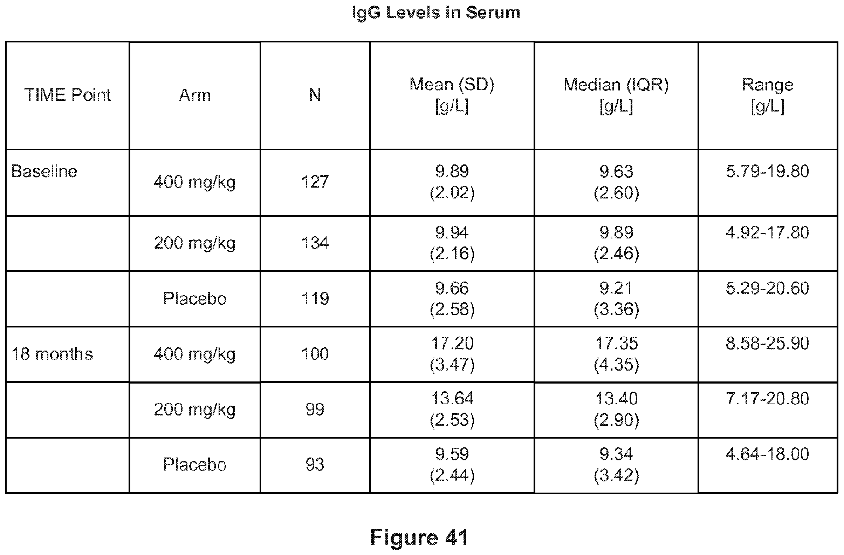

IgG levels in the blood serum of subjects enrolled in the study described in Example 2 were determined. Overall, a modest increase in serum IgG levels in subjects receiving low dose IVIG and a larger increase in serum IgG levels in subjects receiving high dose IVIG was observed, as seen in Figure 41. No increase in CSF IgG was observed in subjects receiving placebo.

### Correlations of Imaging Biomarkers with Primary Endpoints

Significant correlations were found between IVIG treatment outcomes measured using imaging biomarkers and primary cognitive assessments. As shown in Figure 38, the strongest correlations were seen between ventricular volume assessment by MRI imaging and ADCS-Cog or ADCS-ADL. Strong correlations were also seen between AV-45 PET imaging and ADCS-ADL assessment.

### Example 5 - Safety Profile of IVIG Treatment for Alzheimer's Disease

Overall, the IVIG treatment study for Alzheimer's disease described in Example 2 had an excellent safety profile.

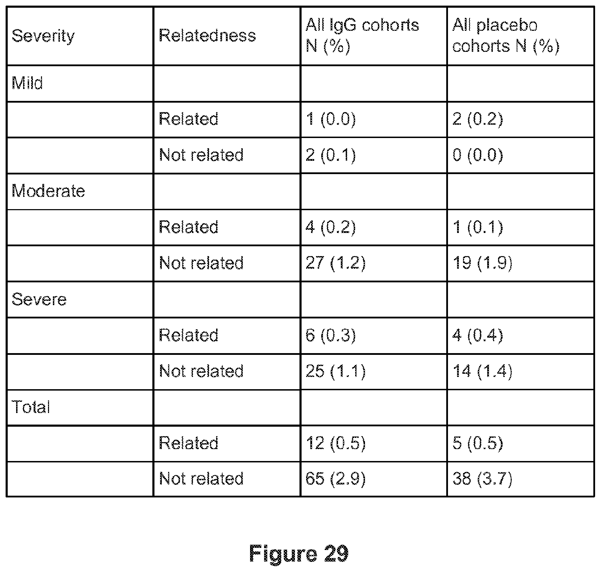

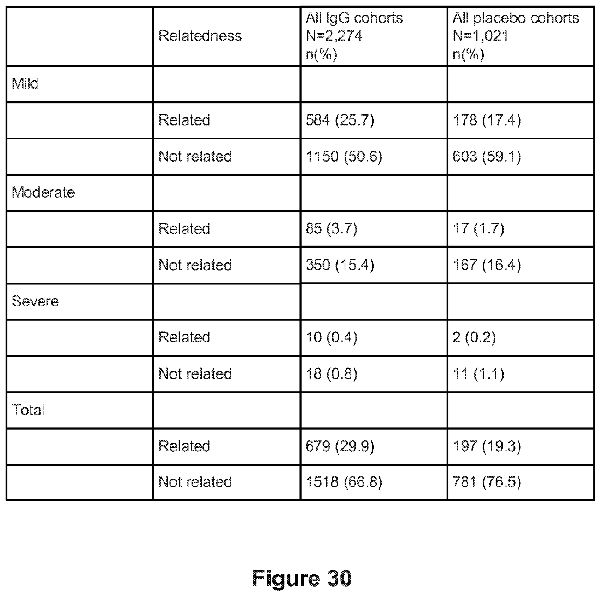

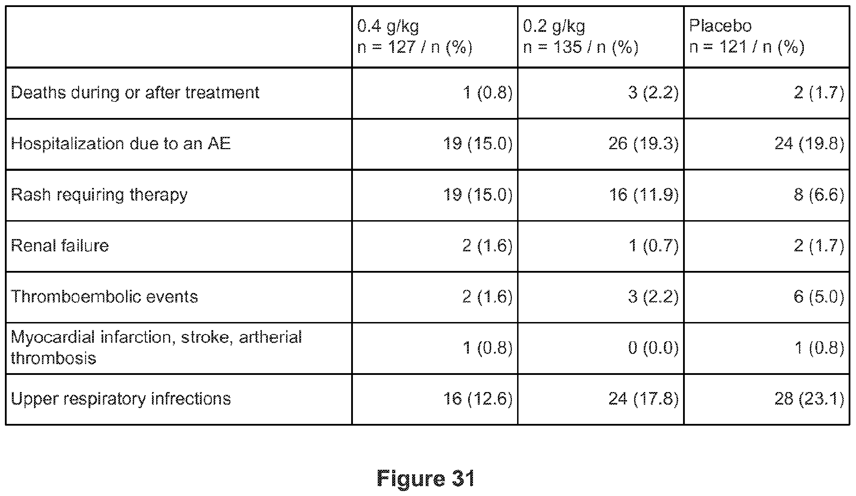

Decreases in hemoglobin levels and clinical signs of hemolysis are labeled adverse events for all commercially sold IVIG medicaments. However, as shown in Figure 28, there was a small increase in the occurrence of decreased hemoglobin levels in subject administered low and high dose IVIG. There was no evidence of hemolysis in any of the subject. Additionally, each subject's LDH levels was within in a normal range. Furthermore, there was no overall increase in serious side effects (Figure 29) and only a small increase in non-serious adverse events (Figure 30). There was a small increase in the occurrence of a rash requiring therapy, as shown in Figure 31.

### Example 6 - Analysis of IVIG Administration in Subjects Classified with Florbetapir Scores of ≥ 1.2"

To further identify Alzheimer's patient sub-populations that will benefit from treatment with pooled immunoglobulin G, the results of the IVIG treatment study presented in Example 2 were reevaluated with respect to the patients' florbetapir score. Advantageously, it was found that high dose pooled IgG treatment (0.4 g/kg/2 weeks) provided a therapeutic benefit to patients having a florbetapir score ≥ 1.2. These results are contrasted to treatment with low dose pooled IgG treatment (0.2 g/kg/2 weeks) and placebo, which demonstrated little to no benefit.

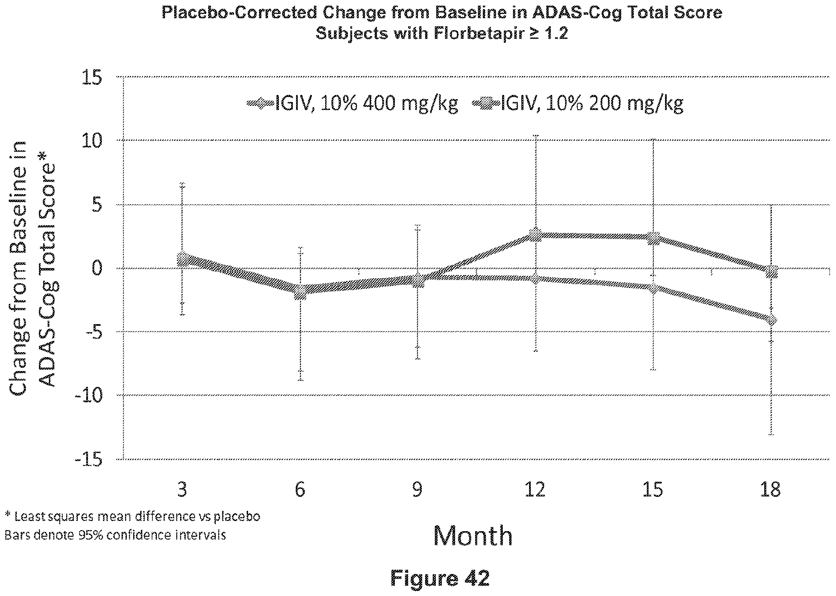

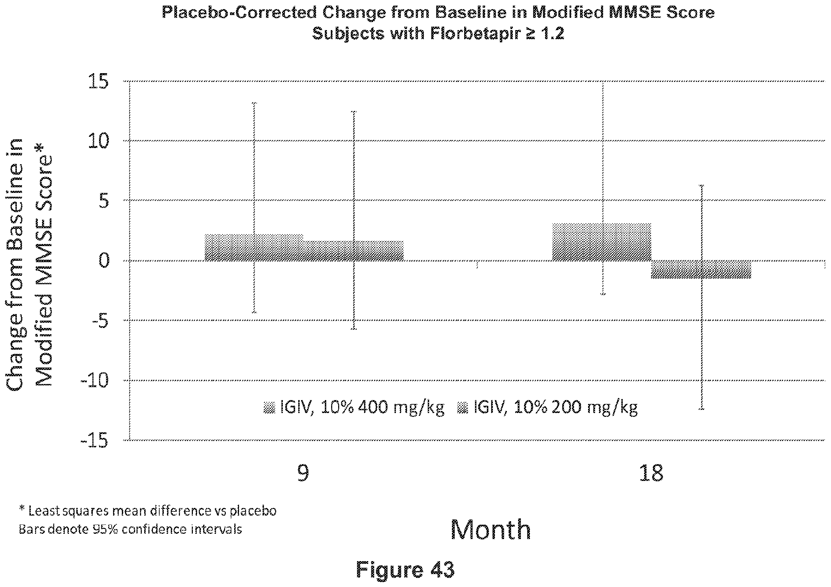

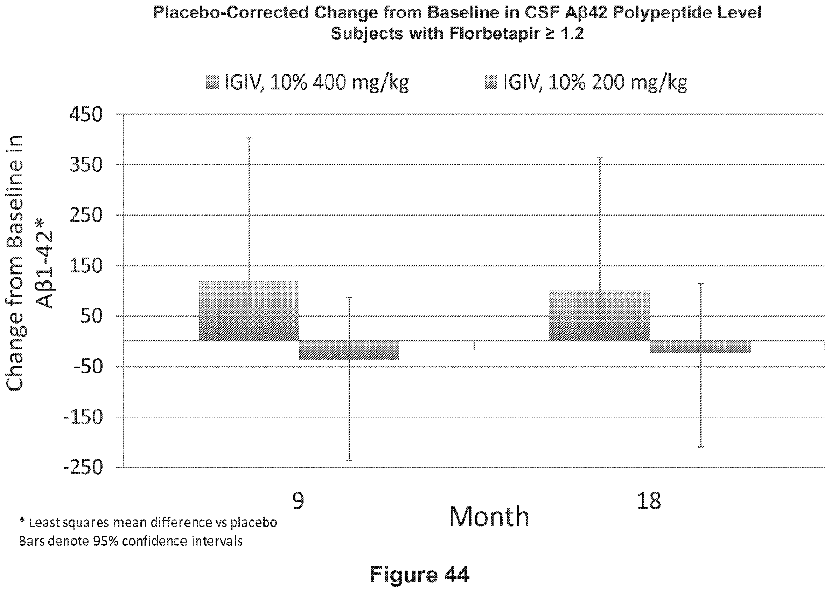

Figures 42 and 43 evidence that dementia progressed more slowly in patients with florbetapir scores ≥ 1.2 receiving high dose IVIG therapy than in patients with florbetapir scores < 1.2 receiving low dose IVIG therapy. Specifically, the progression of dementia in patients receiving high and low dose IVIG was tracked over a period of 18 months using the ADAS-Cog (Figure 42) and modified MMSE (Figure 43) examinations. Slowed progression of dementia is demonstrated by the lower ADAS-Cog scores (Figure 42) and higher modified MMSE scores (Figure 43) for the high dose IVIG cohort, normalized to the placebo cohort, as compared to the low dose IVIG cohort. These results show that low dose IgG treatment provided little to no benefit, as compared to placebo, while high dose IgG reduced the progression of dementia in patients with florbetapir scores ≥ 1.2. Figure 44 shows that administration of high dose IVIG, but not low dose IVIG, reduced CSF Aβ42 peptide levels in patients with florbetapir scores ≥ 1.2.

Taken together, these results show that Alzheimer's patients with a florbetapir score ≥ 1.2 can benefit from high dose IVIG therapy, *e*.*g*., 300 mg IgG/kg to 800 mg IgG/kg body weight of the subject per two week period.

### Example 7 - Analysis of IVIG Administration in Subjects with and without Amyloid Plaques

To further identify Alzheimer's patient sub-populations that will benefit from treatment with pooled immunoglobulin G, the results of the IVIG treatment study presented in Example 2 were reevaluated with respect to whether or not the patients displayed amyloid plaques. Advantageously, it was found that high dose pooled IgG treatment (0.4 g/kg/2 weeks) provided a therapeutic benefit to patients without amyloid plaques.

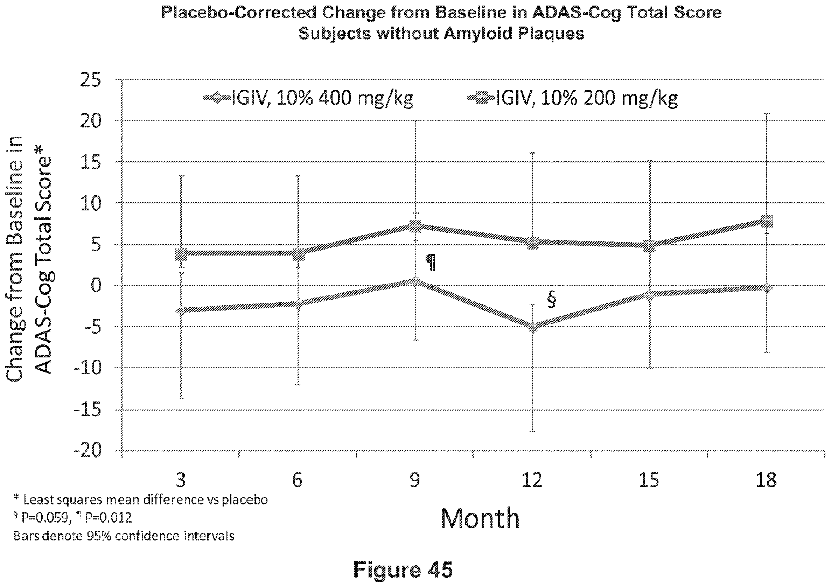

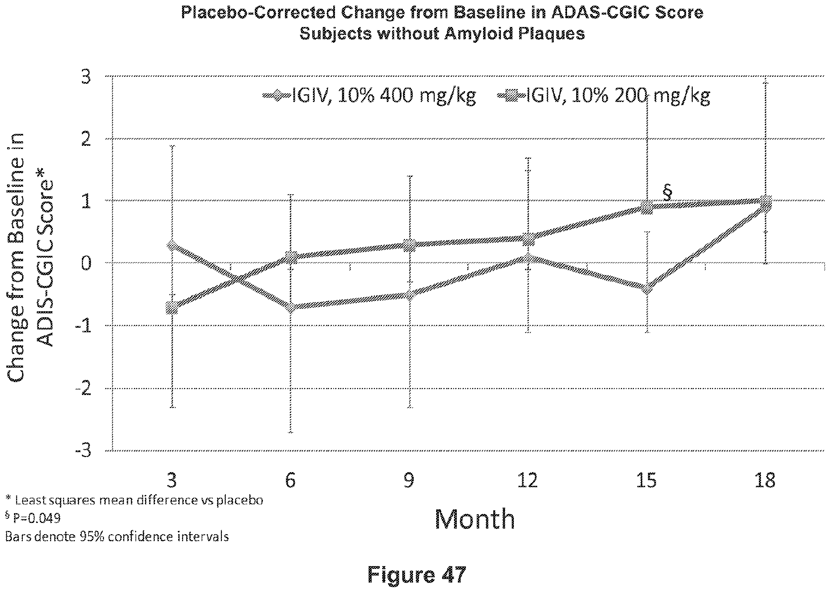

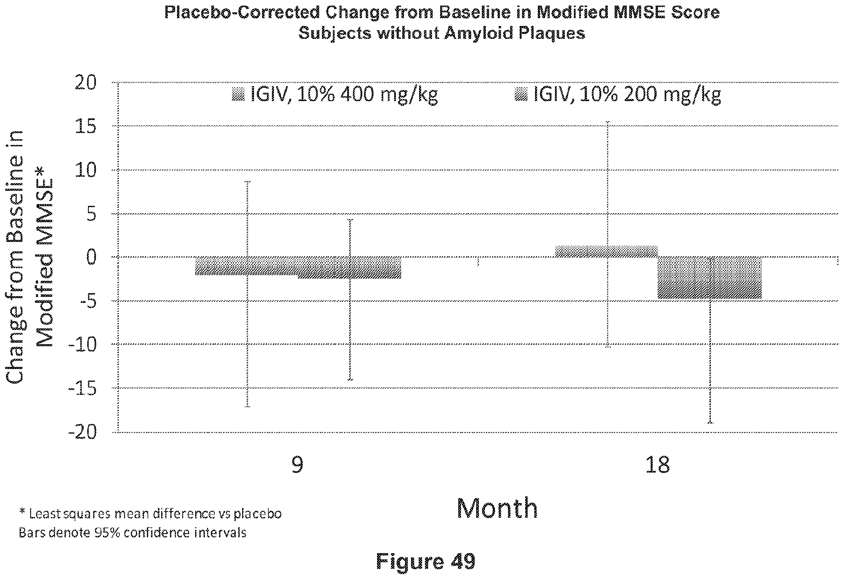

For example, Figures 45, 47, and 49 evidence that dementia progressed more slowly in subjects without amyloid plaques who receiving high dose IVIG therapy than in subjects without amyloid plaques receiving low dose IVIG therapy. Specifically, the progression of dementia in patients receiving high and low dose IVIG was tracked over a period of 18 months using the ADAS-Cog (Figure 45), ADAS-CGIC (Figure 47) and modified MMSE (Figure 49) examinations. Slowed progression of dementia is demonstrated by the lower ADAS-Cog and ADAS-CGIC scores (Figures 45 and 47, respectively) and higher modified MMSE scores (Figure 49) for the high dose IVIG cohort, normalized to the placebo cohort, as compared to the low dose IVIG cohort. These results show that low dose IgG treatment provided little to no benefit, as compared to placebo, while high dose IgG reduced the progression of dementia in subjects without amyloid plaques.

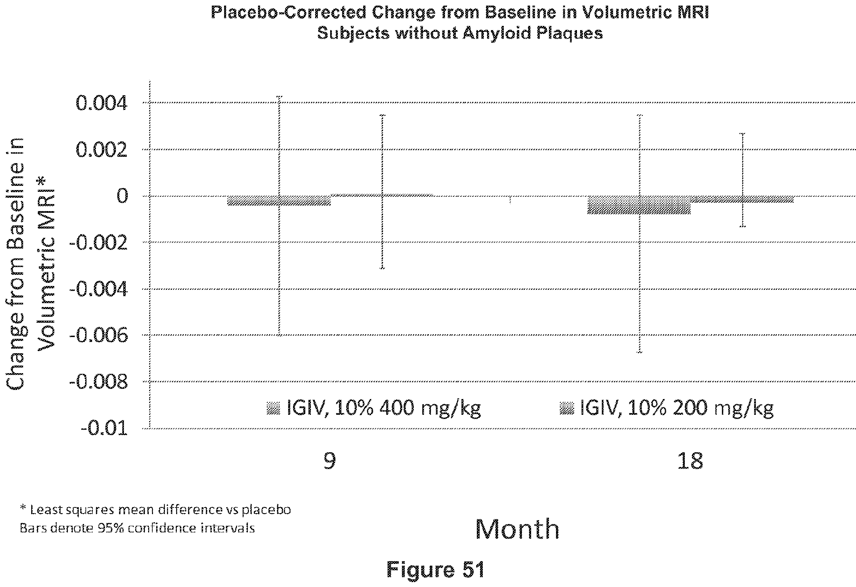

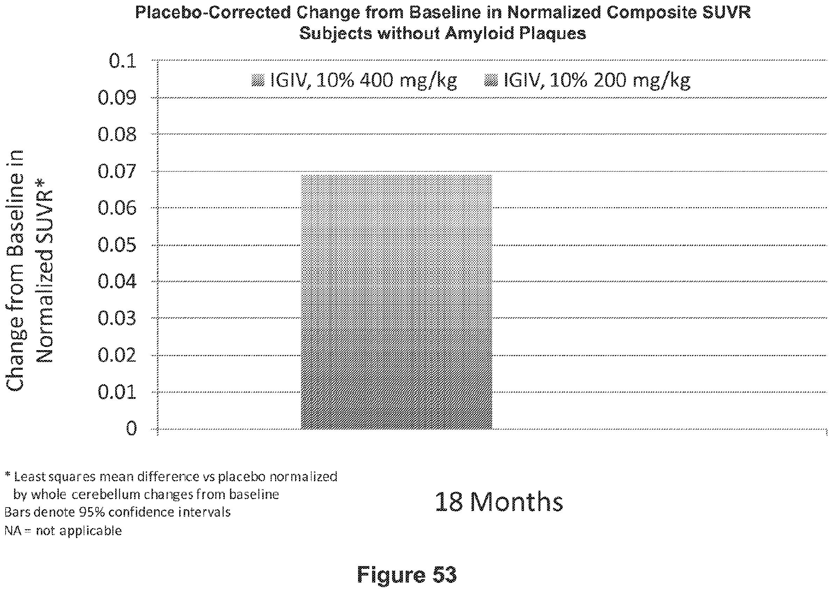

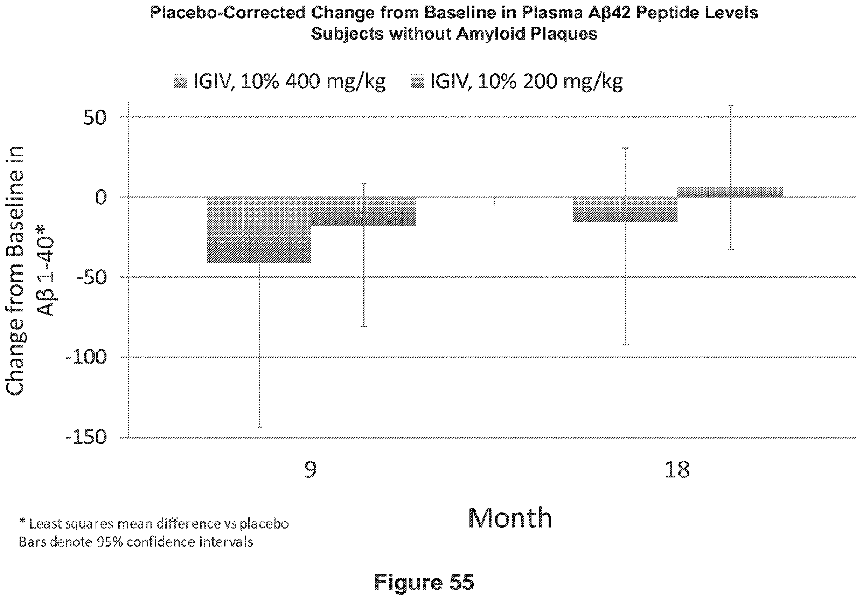

Further evidence of the beneficial results are shown by reduced ventricular volume (Figure 51), determined by volumetric MRI as described above, and increased composite standardized uptake value ratio (SUVR; Figure 53) in Alzheimer's patients without amyloid plaques who received high dose IgG (0.4 g/kg/2 weeks) therapy. Consistent with these findings, administration of high dose IgG reduced the change in baseline for plasma Aβ42 peptide levels in Alzheimer's patients without amyloid plaques (Figure 55).

Taken together, these results show that Alzheimer's patients without amyloid plaques can benefit from high dose IVIG therapy, *e*.*g*., 300 mg IgG/kg to 800 mg IgG/kg body weight of the subject per two week period.

## Claims

1. A composition comprising a therapeutically effective amount of pooled human immunoglobulin G (IgG) for use in treating Alzheimer's disease in a subject with moderately severe Alzheimer's disease who is carrier of at least one APOE4 allele,
wherein the amount of pooled human IgG is from 300 mg/kg to 800 mg/kg body weight of the subject per two week period,
wherein the amount is administered in one or more doses during the two week period after initiation of a therapeutic regimen, and
wherein the subject has a Mini-Mental Status Examination (MMSE) score of from 14 to 22.

2. The composition for use according to claim 1, wherein the subject is homozygous for the APOE4 allele.

3. The composition for use according to claim 1, wherein the subject is heterozygous for the APOE4 allele.

4. The composition for use according to claim 3, wherein the subject has an APOE4/APOE3 genotype.

5. The composition for use according to claim 3, wherein the subject has an APOE4/APOE2 genotype.

6. The composition for use according to any of claims 1 to 5, wherein the amount of pooled human IgG is administered in a single dose over the two week period.

7. The composition for use according to any of claims 1 to 5, wherein the amount of pooled human IgG is administered in multiple doses over the two week period.

8. The composition for use according to claim 7, wherein the dose is administered every week.

9. The composition for use according to any of claims 1 to 8, wherein the amount of pooled human IgG is 400 mg/kg body weight of the subject per two week period.

10. The composition for use according to any of claims 1 to 8, wherein the amount of pooled human IgG is 600 mg/kg body weight of the subject per two week period.

11. The composition for use according to any of claims 1 to 8, wherein the amount of pooled human IgG is 800 mg/kg body weight of the subject per two week period.

12. The composition for use according to any of claims 1 to 11, wherein the amount of pooled human IgG is administered by intravenous administration, subcutaneous administration, intramuscular administration, or intraperitoneal administration.

13. The composition for use according to any of claims 1 to 11, wherein the amount of pooled human IgG is administered by intranasal administration, intrathecal administration, intracerebral administration, intracerebroventricular administration, epidural administration, or spinal administration.

14. The composition for use according to any of claims 1 to 13, wherein the subject is also administered a therapeutically effective amount of a second composition for treatment of Alzheimer's disease.

15. The composition for use according to any of claims 1-14, wherein the subject has a Mini-Mental Status Examination (MMSE) score of from 14 to 20.

## Patentansprüche

1. Zusammensetzung, umfassend eine therapeutische wirksame Menge von gepooltem humanem Immunoglobulin G (IgG) zur Verwendung bei der Behandlung von Morbus Alzheimer in einem Subjekt mit mittelschwerer Morbus Alzheimer, welches ein Träger mindestens eines APOE4-Allels ist, wobei die Menge an gepooltem humanem IgG von 300 mg/kg bis 800 mg/kg Körpergewicht des Subjekts pro zwei-Wochen-Periode beträgt,
wobei die Menge in einer oder mehreren Dosis/Dosen, während der zwei-Wochen-Periode, nach Beginn des therapeutischen Regimes, verabreicht wird, und
wobei das Subjekt einen Mini-Mental-Status-Untersuchung (Mini-Mental Status Examination, MMSE)-Score von 14 bis 22 hat.

2. Zusammensetzung zur Verwendung gemäß Anspruch 1, wobei das Subjekt homozygot für das APOE4-Allel ist.

3. Zusammensetzung zur Verwendung gemäß Anspruch 1, wobei das Subjekt heterozygot für das APOE4-Allel ist.

4. Zusammensetzung zur Verwendung gemäß Anspruch 3, wobei das Subjekt einen APOE4/APOE3-Genotyp hat.

5. Zusammensetzung zur Verwendung gemäß Anspruch 3, wobei das Subjekt einen APOE4/APOE2-Genotyp hat.

6. Zusammensetzung zur Verwendung gemäß irgendeinem der Ansprüche 1 bis 5, wobei die Menge an gepooltem humanem IgG in einer einzelnen Dosis innerhalb der zwei-Wochen-Periode verabreicht wird.

7. Zusammensetzung zur Verwendung gemäß irgendeinem der Ansprüche 1 bis 5, wobei die Menge an gepooltem humanem IgG in mehreren Dosen innerhalb der zwei-Wochen-Periode verabreicht wird.

8. Zusammensetzung zur Verwendung gemäß Anspruch 7, wobei die Dosis jede Woche verabreicht wird.

9. Zusammensetzung zur Verwendung gemäß irgendeinem der Ansprüche 1 bis 8, wobei die Menge an gepooltem humanem IgG 400 mg/kg Körpergewicht pro zwei-Wochen-Periode beträgt.

10. Zusammensetzung zur Verwendung gemäß irgendeinem der Ansprüche 1 bis 8, wobei die Menge an gepooltem humanem IgG 600 mg/kg Körpergewicht pro zwei-Wochen-Periode beträgt.

11. Zusammensetzung zur Verwendung gemäß irgendeinem der Ansprüche 1 bis 8, wobei die Menge an gepooltem humanem IgG 800 mg/kg Körpergewicht pro zwei-Wochen-Periode beträgt.

12. Zusammensetzung zur Verwendung gemäß irgendeinem der Ansprüche 1 bis 11, wobei die Menge an gepooltem humanem IgG mittels intravenöser Verabreichung, subkutaner Verabreichung, intramuskulärer Verabreichung oder intraperitonealer Verabreichung verabreicht wird.

13. Zusammensetzung zur Verwendung gemäß irgendeinem der Ansprüche 1 bis 11, wobei die Menge an gepooltem humanem IgG mittels intranasaler Verabreichung, intrathekaler Verabreichung, intrazerebraler Verabreichung, intrazerebroventrikulärer Verabreichung, epiduraler Verabreichung oder spinaler Verabreichung verabreicht wird.

14. Zusammensetzung zur Verwendung gemäß irgendeinem der Ansprüche 1 bis 13, wobei auch eine therapeutisch wirksame Menge einer zweiten Zusammensetzung zur Behandlung von Morbus Alzheimer an das Subjekt verabreicht wird.

15. Zusammensetzung zur Verwendung gemäß irgendeinem der Ansprüche 1 bis 14, wobei das Subjekt einen Mini-Mental-Status-Untersuchung (Mini-Mental Status Examination, MMSE)-Score von 14 bis 20 hat.

## Revendications

1. Composition comprenant une quantité thérapeutiquement efficace d'immunoglobuline G (IgG) humaine groupée pour l'utilisation dans le traitement de la maladie d'Alzheimer dans un sujet avec une maladie d'Alzheimer modérément sévère qui est porteur d'au moins un allèle APOE4,
dans laquelle la quantité d'IgG humaine groupée est de 300 mg/kg à 800 mg/kg de poids corporel du sujet par période de deux semaines,
dans laquelle la quantité est administrée en une ou plusieurs doses pendant la période de deux semaines après l'initiation d'un régime thérapeutique, et
dans laquelle le sujet a un score dans un mini-examen de l'état mental (MMSE) de 14 à 22.

2. La composition pour l'utilisation selon la revendication 1, dans laquelle le sujet est homozygote de l'allèle APOE4.

3. La composition pour l'utilisation selon la revendication 1, dans laquelle le sujet est hétérozygote de l'allèle APOE4.

4. La composition pour l'utilisation selon la revendication 3, dans laquelle le sujet a un génotype APOE4/APOE3.

5. La composition pour l'utilisation selon la revendication 3, dans laquelle le sujet a un génotype APOE4/APOE2.

6. La composition pour l'utilisation selon l'une quelconque des revendications 1 à 5, dans laquelle la quantité d'IgG humaine groupée est administrée en une seule dose sur la période de deux semaines.

7. La composition pour l'utilisation selon l'une quelconque des revendications 1 à 5, dans laquelle la quantité d'IgG humaine groupée est administrée en doses multiples sur la période de deux semaines.

8. La composition pour l'utilisation selon la revendication 7, dans laquelle la dose est administrée chaque semaine.

9. La composition pour l'utilisation selon l'une quelconque des revendications 1 à 8, dans laquelle la quantité d'IgG humain groupée est de 400 mg/kg de poids corporel du sujet par période de deux semaines.

10. La composition pour l'utilisation selon l'une quelconque des revendications 1 à 8, dans laquelle la quantité d'IgG humain groupée est de 600 mg/kg de poids corporel du sujet par période de deux semaines.

11. La composition pour l'utilisation selon l'une quelconque des revendications 1 à 8, dans laquelle la quantité d'IgG humain groupée est de 800 mg/kg de poids corporel du sujet par période de deux semaines.

12. La composition pour l'utilisation selon l'une quelconque des revendications 1 à 11, dans laquelle la quantité d'IgG humain groupée est administrée par voie intraveineuse, par voie sous-cutanée, par voie intramusculaire, ou par voie intrapéritonéale.

13. La composition pour l'utilisation selon l'une quelconque des revendications 1 à 11, dans laquelle la quantité d'IgG humain groupée est administrée par voie intranasale, par voie intrathécale, par voie intracérébrale, par voie intracérébroventriculaire, par voie épidurale, ou par voie spinale.

14. La composition pour l'utilisation selon l'une quelconque des revendications 1 à 13, dans laquelle une quantité thérapeutiquement efficace d'une deuxième composition pour le traitement de la maladie d'Alzheimer est aussi administrée au sujet.

15. La composition pour l'utilisation selon l'une quelconque des revendications 1 à 14, dans laquelle le sujet a un score dans un mini-examen de l'état mental (MMSE) de 14 à 20.
